(19) Europäisches Patentamt
European Patent Office
Office européen des brevets

(11) EP 3 925 671 A1

(12) **EUROPEAN PATENT APPLICATION**
published in accordance with Art. 153(4) EPC

(43) Date of publication:
**22.12.2021 Bulletin 2021/51**

(21) Application number: **20755453.6**

(22) Date of filing: **13.02.2020**

(51) Int Cl.:
*A61Q 19/00* (2006.01) *A61Q 5/00* (2006.01)
*A61Q 5/02* (2006.01) *A61Q 5/12* (2006.01)
*A61K 8/20* (2006.01) *A61K 8/24* (2006.01)
*A61K 8/36* (2006.01) *A61K 8/40* (2006.01)
*A61K 8/41* (2006.01) *A61K 8/46* (2006.01)
*A61K 8/67* (2006.01)

(86) International application number:
**PCT/JP2020/005669**

(87) International publication number:
**WO 2020/166678 (20.08.2020 Gazette 2020/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.02.2019 JP 2019023739**
**21.06.2019 JP 2019115882**

(71) Applicant: **Miyoshi Oil & Fat Co., Ltd.**
**Tokyo 124-8510 (JP)**

(72) Inventors:
- **KANEKO, Kotaro**
  **Tokyo 124-8510 (JP)**
- **YASHITA, Akira**
  **Tokyo 124-8510 (JP)**
- **KAWAI, Koji**
  **Tokyo 124-8510 (JP)**
- **KAWAKAMI, Hayato**
  **Iwakura-shi, Aichi 482-8511 (JP)**
- **IBA, Nobuyo**
  **Iwakura-shi, Aichi 482-8511 (JP)**

(74) Representative: **Angerhausen, Christoph**
**Boehmert & Boehmert**
**Anwaltspartnerschaft mbB**
**Pettenkoferstrasse 22**
**80336 München (DE)**

# (54) COSMETIC INGREDIENT, COSMETIC, AND PRODUCTION METHOD FOR COSMETIC

(57) Provided are a novel cosmetic compounding agent capable of imparting superior properties such as a water and moisture retention property to a cosmetic for use on the hair and skin; a cosmetic using the cosmetic compounding agent; and a method for producing the cosmetic. The cosmetic compounding agent of the present invention contains an organic ammonium salt having a hydrogen-bonding functional group(s) in a cation and/or an anion. In a preferable embodiment, the cation is an ammonium cations represented by the following formula (I):

$$N^+[R^1]_n[R^2]_{4-n} \qquad (I)$$

wherein each $R^1$ independently represents, for example, a hydroxyalkyl group in which there is at least one hydroxy group, an alkyl moiety is a linear or branched moiety having 1 to 10 carbon atoms, and the alkyl moiety may contain an oxygen atom(s), and
wherein each $R^2$ independently represents a hydrogen atom or a linear or branched alkyl group having 1 to 18 carbon atoms, and n represents an integer of 0 to 4.

EP 3 925 671 A1

## Description

TECHNICAL FIELD

The present invention relates to a cosmetic compounding agent, a cosmetic and a production method thereof.

BACKGROUND ART

Conventionally, it is known that ingredients for imparting a water and moisture retention effect are added to a cosmetic for hair treatment and skin care, etc. However, these ingredients may cause a poor feeling of use such as a stickiness; desired are ingredients satisfying a water and moisture retention property and a feeling(s) of use.

Hair dries upon losing the moisture content therein due to, for example, a dry air, a shampoo with a high detergency, the heat of a dryer, natural drying after bathing, and damages from getting one's hair permed or colored. When the moisture content in the hair has reached an inadequate level due the drying of the hair, cuticle peeling will occur more easily; the cuticles are dominant tissues for imparting a gloss to the hair and keeping the hair healthy by covering the surface of the hair so as to prevent water from evaporating. As a result of cuticle peeling, the hair will dry and, for example, due to a loss in smoothness and a dryness of the hair, not only a poor flexibility and finger-combing capability of the hair will be observed, but the hair will be more likely to spread, and split ends and breakages will occur more easily. Therefore, water and moisture retention of the hair is essential in terms of avoiding problems owing to the drying of the hair and keeping the hair beautiful and healthy.

Conventionally, it is suggested that a multivalent alcohol(s) as a moisture-retaining ingredient be added to a hair treatment composition such as a shampoo (e.g. Patent documents 1 and 2). However, in the case of a conventional hair treatment composition, for example, there may not be achieved a satisfactory water and moisture retention property under a low-humidity environment such as an environment involving dryness after washing one's hair and an environment involving dryness during winter season, and the water and moisture retention property may not be able to be maintained for a long period of time due to a volatility of the composition; a novel hair treatment composition capable of compensating for these imperfections is desired.

In Patent document 3, there are disclosed organic ammonium salts such as imidazolium salt, pyrrolidinium salt, piperidinium salt, pyridinium salt and phosphonium salt. While these types of organic ammonium salts have a property of retaining moisture, they have, for example, safety problems as well as a problem of not being able to exert a satisfactory water and moisture retention effect when used on the hair.

Although the applicant of the present invention has proposed an organic salt (ionic liquid) having a hydrogen-bonding functional group(s) in a cation or an anion (Patent documents 4 and 5), they have not specifically considered the water and moisture retention effect thereof as well as the application thereof to the hair and for skin care purpose.

It is known that skin roughness is mainly caused by reduction in moisture of the skin. For example, skin dries due to, for example, a dryness in the air during the winter season, skin cleansing, aging and a reduction in skin secretion. If neglecting a dry condition of the skin, the level of tension and glow of the skin will decrease, which will easily lead to a so-called rough condition of the skin. In order to avoid skin roughness, it is essential to maintain a normal skin function by preventing the reduction in moisture contained in the horny layer. In order to retain the moisture content in the horny layer, there are conventionally known various skin care agents for moisture retention purpose that are capable of imparting a proper amount of moisture to the skin.

Conventionally, it is suggested that a multivalent alcohol(s) as a moisture-retaining ingredient be added to a skin care agent (e.g. Patent document 1). However, in the case of a conventional skin care agent, for example, there may not be achieved a satisfactory water and moisture retention property under a low-humidity environment such as an environment involving dryness during winter season, and the water and moisture retention property may not be able to be maintained for a long period of time due to a volatility of the agent; a novel skin care agent capable of compensating for these imperfections is desired.

In Patent document 3, there are disclosed organic ammonium salts such as imidazolium salt, pyrrolidinium salt, piperidinium salt, pyridinium salt and phosphonium salt. While these types of organic ammonium salts have a property of retaining moisture, they have, for example, safety problems as well as a problem of not being able to exert a satisfactory water and moisture retention effect when used for skin care purpose.

The applicant of the present invention has proposed an organic salt (ionic liquid) having a hydrogen-bonding functional group(s) in cations or anions; and a water and moisture retention agent (Patent documents 4 to 6).

PRIOR ART DOCUMENTS

Patent documents

Patent document 1: JP-A-2014-136678
Patent document 2: JP-A-2013-40153
Patent document 3: JP-A-2014-151015
Patent document 4: JP-A-2014-131974
Patent document 5: JP-A-2014-131975
Patent document 6: JP-A-2019-023185

SUMMARY OF THE INVENTION

Problems to be solved by the invention

However, while there has been desired a cosmetic compounding agent having a high short- or long-term water and moisture retention property due to its non-volatility and retaining nature, a high affinity to the hair and skin etc. and a high safety, and providing a superior feeling(s) of use, there has never been a cosmetic compounding agent satisfying all of these properties. Further, it is required that the effects of active ingredients be efficiently expressed when dissolved i.e. it has been desired that a solubility and permeability of such active ingredients be improved.
If the cosmetic compounding agent is a hair treatment agent, desired is a cosmetic compounding agent providing a favorable feeling(s) of use, and having a superior adherability to the proteins contained in the hair and a superior thermal stability; if the cosmetic compounding agent is a skin care agent, desired is a cosmetic compounding agent providing a favorable feeling(s) of use, and capable of improving an affinity and permeability to the skin as well as a whitening effect.
Conventionally, it is known that a multivalent alcohol(s) be used in a cosmetic compounding agent for the purpose of imparting a water and moisture retention capability; desired are a cosmetic compounding agent having a higher water and moisture retention capability and the abovementioned effects as well as a composition using such cosmetic compounding agent.
The present invention was made in view of the aforementioned circumstances. A main object of the present invention is to provide a novel cosmetic compounding agent capable of imparting superior properties such as a water and moisture retention property to a cosmetic in consideration of use on the hair and skin; a cosmetic using such cosmetic compounding agent; and a method for producing such cosmetic.
Particularly, if the cosmetic compounding agent is a hair treatment agent, the object is to provide a hair treatment agent and a hair treatment composition using such hair treatment agent, the hair treatment agent being such a type of agent that has a high adherability to the hair, and a superior water retention property and affinity; brings about favorable feelings of use such as a finger-combing capability, flexibility, texture, elasticity, resilience, volume increase, cohesiveness, moist feeling, gloss, smoothness and non-stickiness; has a superior adherability to the proteins contained in the hair, a superior stability and a superior thermal stability; and exhibits a superior solubility of an active ingredient(s).
Particularly, if the cosmetic compounding agent is a skin care agent, the object is to provide a skin care agent and a skin care composition using such skin care agent, the skin care agent being such a type of agent that has a superior short- or long-term water and moisture retention property, and a superior moisture barrier property of the skin; brings about favorable feelings of use such as a moisture retention feeling, non-stickiness and skin compatibility; has a superior affinity and permeability to the skin, and a high safety to the skin; exhibits a high solubility of an active ingredient(s); and has a whitening effect as well as a superior antistatic property.

Means to solve the problems

In order to solve the aforementioned problems, the cosmetic compounding agent of the present invention is characterized by containing an organic ammonium salt having a hydrogen-bonding functional group(s) in a cation and/or an anion.
In a preferable embodiment, the cation is an ammonium cation.
In a preferable embodiment, the ammonium cation is represented by the following formula (I):
[Chemical formula 1]

$$N^+[R^1]_n[R^2]_{4-n} \qquad (I)$$

wherein each $R^1$ independently represents a hydroxyalkyl group in which there is at least one hydroxy group, an alkyl moiety is a linear or branched moiety having 1 to 10 carbon atoms, and the alkyl moiety may contain an oxygen atom(s); a carboxyalkyl group in which there is at least one carboxy group, an alkyl moiety is a linear or branched moiety having 1 to 10 carbon atoms, and the alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and the alkyl moiety may contain an oxygen atom(s), and
wherein each $R^2$ independently represents a hydrogen atom or a linear or branched alkyl group having 1 to 18 carbon atoms, and n represents an integer of 0 to 4.

In a preferable embodiment, the cosmetic compounding agent is a hair treatment agent.
In a preferable embodiment, the cosmetic compounding agent is a skin care agent.
The cosmetic of the present invention contains the above cosmetic compounding agent.
In a preferable embodiment, the cosmetic is a hair treatment composition.
In a preferable embodiment, the cosmetic is a skin care composition.
A method for producing the cosmetic of the present invention comprises a step of adding the above cosmetic compounding agent.
The method for producing the cosmetic of the present invention comprises a step of adding an ammonium cation-forming base and an anion-forming acid that form an organic ammonium salt composed of an anion and an ammonium cation represented by the following formula (I):
[Chemical formula 1]

$$N^+[R^1]_n[R^2]_{4-n} \qquad (I)$$

wherein each $R^1$ independently represents a hydroxyalkyl group in which there is at least one hydroxy group, an alkyl moiety is a linear or branched moiety having 1 to 10 carbon atoms, and the alkyl moiety may contain an oxygen atom(s); a carboxyalkyl group in which there is at least one carboxy group, an alkyl moiety is a linear or branched moiety having 1 to 10 carbon atoms, and the alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and the alkyl moiety may contain an oxygen atom(s), and
wherein each $R^2$ independently represents a hydrogen atom or a linear or branched alkyl group having 1 to 18 carbon atoms, at least one $R^2$ represents a hydrogen atom, and n represents an integer of 0 to 4.

Effects of the invention

According to the cosmetic compounding agent of the present invention, the cosmetic using such cosmetic compounding agent, and the method for producing such cosmetic, the cosmetic compounding agent has a high short- or long-term water and moisture retention property due to its non-volatility and retaining nature, a high affinity to the hair and skin etc. and a high safety; provides a superior feeling(s) of use; and is capable of improving a solubility and permeability of an active ingredient(s) such that the effects of the active ingredient(s) can be efficiently expressed.
Particularly, if the cosmetic compounding agent is a hair treatment agent, there can be provided a hair treatment agent and a hair treatment composition using such hair treatment agent, the hair treatment agent being such a type of agent that has a high adherability to the hair, and a superior water retention property, affinity and antistatic property; brings about favorable feelings of use such as a finger-combing capability, flexibility, texture, elasticity, resilience, volume increase, cohesiveness, moist feeling, gloss, smoothness and non-stickiness; has a superior adherability to the proteins contained in the hair, a superior stability and a superior thermal stability; and exhibits a superior solubility of an active ingredient(s).
If the cosmetic compounding agent is a skin care agent, there can be provided a skin care agent and a skin care composition using such skin care agent, the skin care agent being such a type of agent that has a superior short- or long-term water and moisture retention property, and a superior moisture barrier property of the skin; brings about favorable feelings of use such as a moisture retention feeling, non-stickiness and skin compatibility; has a superior affinity and permeability to the skin, and a high safety to the skin; exhibits a high solubility of an active ingredient(s); and has a whitening effect as well as a superior antistatic property.

BRIEF DESCRIPTION OF THE DRAWINGS

FIG.1A is a series of photographs showing appearances of hairs after air drying and SEM images of hair surfaces in evaluations of Table 14.
FIG.1B is a series of photographs showing appearances of hairs after air drying and SEM images of hair surfaces in evaluations of Table 14.
FIG.2 is a series of photographs showing appearances of hairs after air drying in evaluations of Table 16.
FIG.3 is a series of photographs showing appearances of hairs after air drying in evaluations of Table 17.

MODE FOR CARRYING OUT THE INVENTION

The present invention is described in detail hereunder.

1. Cosmetic compounding agent

The cosmetic compounding agent of the present invention is mainly directed to one added as a raw material to a cosmetic as a product.

Although not particularly limited, examples of a cosmetic compounding agent include a hair treatment agent added as a raw material to a hair treatment composition as a product suitable for use in the hair or the like; and a skin care agent added as a raw material to a skin care composition as a product suitable for use in skin or the like. Although not particularly limited, examples of the aforementioned hair include hairs of human body, such as scalp hairs, mustaches/beards, eyebrows, eyelashes, nasal hairs, ear hairs, underarm hairs and body hairs; the cosmetic compounding agent of the present invention is applicable to a cosmetic associated with these hairs. Although not particularly limited, examples of a target of the aforementioned skin care agent include dermis, skin, cuticle, nails, and mucous membranes in oral and nasal cavities; the cosmetic compounding agent of the present invention is applicable to a cosmetic associated with these targets.

The cosmetic compounding agent of the present invention contains an organic ammonium salt having a hydrogen-bonding functional group(s) in a cation and/or an anion.

In the present invention, the organic ammonium salt contains an organic cation or $NH_4^+$ with a nitrogen atom being an ion center, and an organic anion.

1-1. Cation

Although not particularly limited, as a cation(s) of the organic ammonium salt, there may be listed a cation with a nitrogen atom being an ion center, such as an ammonium cation (organic ammonium cation $NR_4^+$ substituted by an organic group(s) (at least one R is an organic group, while the rest of them are hydrogen atoms) and $NH_4^+$); and a cation other than the ammonium cation, such as a cation(s) of the organic ammonium salt with the nitrogen atom therein being substituted by an organic group, examples of which include an imidazolium cation, pyridinium cation, pyrrolidinium cation, piperidinium cation, pyrrolinium cation, pyrazinium cation, triazolium cation, isoquinolinium cation, oxazolinium cation, thiazolinium cation, morpholinium cation, guanidium cation, pyrimidinium cation, piperazinium cation, triadinium cation, quinolinium cation, indolinium cation, quinoxalinium cation, isooxazolium cation and cationic amino acid. Among them, preferred are an ammonium cation, imidazolium cation, pyridinium cation, pyrrolidinium cation, piperidinium cation and morpholinium cation; more preferred is an ammonium cation. Here, the cations exemplified above are listed as collective terms for cations including cations having a substituent group(s) such as a hydrogen-bonding functional group other than the cations having the basic structures described above.

It is preferred that the organic ammonium salt used in the present invention have a hydrogen-bonding functional group(s) in a cation.

Although not particularly limited, examples of the hydrogen-bonding functional group include an oxygen-containing group, a nitrogen-containing group, a sulfur-containing group, a phosphorus-containing group, and a hydrogen atom directly bonded to nitrogen.

Although not particularly limited, examples of the oxygen-containing group include a hydroxy group, a carbonyl group, an ether group, an ester group, an aldehyde group, a carboxy group, a carboxylate group, a urea group, a urethane group, an amide group, an oxazole group, a morpholin group, a carbamic acid group and a carbamate group.

Although not particularly limited, examples of the nitrogen-containing group include an amino group and a nitro group.

Although not particularly limited, examples of the sulfur-containing group include a sulfate group ($-O-S(=O)_2-O-$),

a sulfonyl group (-S(=O)$_2$O-), a sulfonic acid group (-S(=O)$_2$-), a mercapto group (-SH), a thioether group (-S-), a thiocarbonyl group (-C(=S)-), a thiourea group (-N-C(=S)-N-), a thiocarboxy group (-C(=S)OH), a thiocarboxylate group (-C(=S)O-), a dithiocarboxy group (-C(=S)SH) and a dithiocarboxylate group (-C(=S)S-).

Although not particularly limited, examples of the phosphorus-containing group include a phosphate group (-O-P(=O)(-O-)-O-), a phosphonic acid group (-P(=O)(-O-)-O-), a phosphinic acid group (-P(=O)-O-), a phosphite group (-O-P(-O-)-O-), a phosphonous acid group (-P(-O-)-O-), a phosphinous acid group (-P-O-) and a pyrophosphate group [(-O-P(=O)(-O-))$_2$-O-].

Among them, as a hydrogen-bonding functional group(s) present in a cation, preferred are a hydroxy group, a carboxy group, a carboxylate group, an ester group, a carbonyl group, an ether group, and a hydrogen atom directly bonded to nitrogen. Among them, more preferred are a hydroxy group, a carboxy group, a carboxylate group, an ether group, and a hydrogen atom directly bonded to nitrogen; even more preferred are a hydroxy group, a carboxy group, a carboxylate group, and a hydrogen atom directly bonded to nitrogen; particularly preferred are a hydroxy group and a hydrogen atom directly bonded to nitrogen. For example, as a hydrogen-bonding functional group-containing substituent group, there may be listed a hydroxyalkyl group, a carboxyalkyl group, a hydroxycarboxyalkyl group, an alkyl ester group and an alkyl ether group.

The aforementioned hydroxyalkyl group has at least one hydroxy group, and the alkyl moiety thereof is a linear or branched, preferably linear moiety preferably having 1 to 10, more preferably 1 to 6, even more preferably 1 to 4 carbon atoms; such alkyl moiety may also contain an oxygen atom(s).

The aforementioned carboxyalkyl group has at least one carboxy group, and the alkyl moiety thereof is a linear or branched, preferably linear moiety preferably having 1 to 10, more preferably 1 to 6, even more preferably 1 to 5 carbon atoms; such alkyl moiety may also contain an oxygen atom(s).

The aforementioned hydroxycarboxyalkyl group has at least one hydroxy group and at least one carboxy group, and the alkyl moiety thereof is a linear or branched, preferably linear moiety preferably having 1 to 10, more preferably 1 to 6, even more preferably 1 to 5 carbon atoms; such alkyl moiety may also contain an oxygen atom(s).

Here, when the alkyl moiety contains an oxygen atom(s), such oxygen atom(s), for example, either form or contain, in the alkyl moiety, an ether bond, a carbonyl group, a hydroxy group, a carboxylate group, an ester bond, an amide bond, a urea bond or a urethane bond. Therefore, in this invention, the expression "the alkyl moiety contains an oxygen atom(s)" encompasses a case where the alkyl moiety is interrupted by, or the hydrogen atom(s) are substituted by a group serving as an oxygen atom-containing atom group that even contains a hetero atom(s) such as a nitrogen atom.

Examples of the above hydroxyalkyl group include a monohydroxyalkyl group and a polyhydroxyalkyl group; each alkyl group may contain an oxygen atom(s). Although not particularly limited, specific examples thereof include a hydroxyalkyl group, a hydroxyalkoxyalkyl group, an alkoxyhydroxyalkyl group and a hydroxypolyalkyleneoxyalkyl group.

Although not particularly limited, examples of the monohydroxyalkyl group include a hydroxymethyl group, 1-hydroxyethyl group, 2-hydroxyethyl group, 1-hydroxypropane-1-yl group, 2-hydroxypropane-1-yl group, 3-hydroxypropane-1-yl group, 1-hydroxypropane -2-yl group, 2-hydroxypropane-2-yl group, 1-hydroxybutane-1-yl group, 2-hydroxybutane-1-yl group, 3-hydroxybutane-1-yl group, 4-hydroxybutane -1-yl group, 1-hydroxy-2-methylpropane-1-yl group, 2-hydroxy-2-methylpropane-1-yl group, 3-hydroxy-2-methylpropane-1-yl group, 1-hydroxybutane-2-yl group, 2-hydroxybutane-2-yl group, 3-hydroxybutane-2-yl group, 4-hydroxybutane-2-yl group, 1-hydroxy-2-methylpropane-2-yl group, 1,1-dimethyl-2-hydroxyethyl group, 5-hydroxypentane-1-yl group, 6-hydroxyhexane-1-yl group, 7-hydroxyheptane-1-yl group, 8-hydroxyoctane-1-yl group, 9-hydroxynonane-1-yl group and 10-hydroxydecane-1-yl group. It is preferred that the monohydroxyalkyl group be such that the alkyl moiety thereof has 1 to 10 carbon atoms, more preferably 1 to 6 carbon atoms, even more preferably 1 to 4 carbon atoms, particularly preferably 1 to 3 carbon atoms.

Although not particularly limited, examples of the polyhydroxyalkyl group include a di-, tri-, tetra-, penta-, hexa-, hepta- or octahydroxyalkyl group. Although not particularly limited, specific examples thereof include dihydroxyethyl groups such as 1,2-dihydroxyethyl group; dihydroxypropane-1-yl groups such as 1,2-dihydroxypropane-1-yl group and 2,3-dihydroxypropane-1-yl group; dihydroxypropane-2-yl groups such as 1,2-dihydroxypropane-2-yl group and 1,3-dihydroxypropane-2-yl group; trihydroxypropane-1-yl group; trihydroxypropane-2-yl group; dihydroxybutane-1-yl groups such as 1,2-dihydroxybutane-1-yl group, 1,3-dihydroxybutane-1-yl group, 1,4-dihydroxybutane-1-yl group, 2,3-dihydroxybutane-1-yl group, 2,4-dihydroxybutane-1-yl group and 3,4-dihydroxybutane-1-yl group; trihydroxybutane-1-yl groups such as 1,2,3 trihydroxybutane-1-yl group, 1,2,4 trihydroxybutane-1-yl group, 1,3,4 trihydroxybutane-1-yl group and 2,3,4 trihydroxybutane-1-yl group; tetrahydroxybutane-1-yl group; dihydroxy-2-methylpropane-1-yl groups such as 1,2-dihydroxy-2-methylpropane-1-yl group, 1,3-dihydroxy-2-methylpropane-1-yl group and 2,3-dihydroxy-2-methylpropane-1-yl group; trihydroxy-2-methylpropane-1-yl group; tetrahydroxy-2-methylpropane-1-yl group; dihydroxybutane-2-yl groups such as 1,2-dihydroxybutane-2-yl group, 1,3-dihydroxybutane-2-yl group, 1,4-dihydroxybutane-2-yl group, 2,3-dihydroxybutane-2-yl group, 2,4-dihydroxybutane-2-yl group and 3,4-dihydroxybutane-2-yl group; trihydroxybutane-2-yl groups such as 1,2,3 trihydroxybutane-2-yl group, 1,2,4 trihydroxybutane- 2-yl group, 1,3,4 trihydroxybutane-2-yl group and 2,3,4 trihydroxybutane-2-yl group; tetrahydroxybutane-2-yl group; 1,3-dihydroxy-2-methylpropane-2-yl group, 1,3-dihydroxy-2-ethylpropane-2-yl group, 1,3-dihydroxy-2-hydroxymethylpropane-2-yl group; di-, tri-, tetra- or pentahydroxypentane-1-yl group; di-, tri-, tetra-, penta- or hexahydroxyhexane-1-yl group; di-, tri-, tetra-, penta-, hexa- or heptahydroxyheptane-1-yl group; and di-, tri-, tetra-, penta-, hexa-, hepta- or octahydroxyoctane-1-yl group. It is preferred that the polyhydroxyalkyl group have 2 to 8, preferably 2 to 4, even more preferably 2 to 3 hydroxy groups. It is preferred that the alkyl moiety of such polyhydroxyalkyl group have 1 to 6, more preferably 1 to 4 carbon atoms. n is preferably an integer of 1 to 2.

Further, one preferable example is a branched polyhydroxyalkyl group represented by the following formula.

[Chemical formula 2]

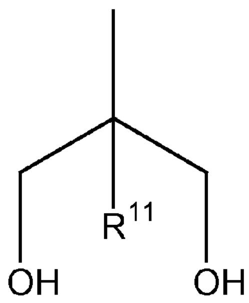

(In this formula, $R^{11}$ represents a hydrogen atom, a linear alkyl group having 1 to 4 carbon atoms, or a linear monohydroxyalkyl group having 1 to 4 carbon atoms.)

Among the above polyhydroxyalkyl groups, preferred are 2,3-dihydroxypropane-1-yl group, 1,3-dihydroxypropane-2-yl group, 1,3-dihydroxy-2-methylpropane-2-yl group, 1,3-dihydroxy-2-ethylpropane-2-yl group, 1,3-dihydroxy-2-hydroxymethylpropane-2-yl group and pentahydroxyhexane-1-yl group.

Examples of the aforementioned carboxyalkyl group include a monocarboxyalkyl group and a polycarboxyalkyl group. Specific examples thereof include groups having an equivalent structure with those obtained by substituting the hydroxy groups in any of the mono-, di-, tri-, tetra-, penta-, hexa-, hepta- or octahydroxyalkyl groups exemplified above with a carboxy group(s) (alkyl moieties of such groups may contain oxygen atoms).

Although not particularly limited, examples of the monocarboxyalkyl group include a carboxymethyl group, 1-carboxyethyl group, 2-carboxyethyl group, 1-carboxypropane-1-yl group, 2-carboxypropane-1-yl group, 3-carboxypropane-1-yl group, 1-carboxypropane-2-yl group, 2-carboxypropane-2-yl group, 1-carboxybutane-l-yl group, 2-carboxybutane-1-yl group, 3-carboxybutane-1-yl group, 4-carboxybutane-1-yl group, 1-carboxy-2-methylpropane-1-yl group, 2-carboxy-2-methylpropane-1-yl group, 3-carboxy-2-methylpropane-1-yl group, 1-carboxybutane-2-yl group, 2-carboxybutane-2-yl group, 3-carboxybutane-2-yl group, 4-carboxybutane-2-yl group, 1-carboxy-2-methylpropane-2-yl group, 5-carboxypentane-1-yl group, 6-carboxyhexane-1-yl group, 7-carboxyheptane-1-yl group, 8-carboxyoctane-1-yl group, 9-carboxynonane-1-yl group, 10-carboxydecane-1-yl group. It is preferred that the carboxyalkyl group be such that the alkyl moiety thereof has 1 to 10, more preferably 1 to 6, even more preferably 1 to 5 carbon atoms.

Although not particularly limited, examples of the abovementioned hydroxycarboxyalkyl group include groups having an equivalent structure with those obtained by substituting part of the hydroxy groups in any of the di-, tri-, tetra-, penta-, hexa-, hepta- or octahydroxyalkyl groups exemplified above with a carboxy group(s) (alkyl moieties of such groups may contain oxygen atoms). Examples of a monohydroxycarboxyalkyl group having one hydroxy group and one carboxy group, include 2-hydroxy-3-carboxybutane-1-yl group (carnitine), 1-hydroxyethyl-2-carboxyethyl group (serine) and 2-hydroxyethyl-2-carboxyethyl group (threonine). As a hydroxycarboxyalkyl group, 2-hydroxy-3-carboxybutane-1-yl group (carnitine) is preferred. It is preferred that the hydroxycarboxyalkyl group be such that the alkyl moiety thereof has 1 to 10, more preferably 1 to 6, even more preferably 1 to 5 carbon atoms.

Although not particularly limited, examples of the aforementioned alkyl ester group include groups having an equivalent structure with those obtained by esterifying the carboxy groups in the carboxyalkyl groups exemplified above. Examples of a monoalkyl ester group having one ester group include 1-acetoxyethane-2-yl group (acetylcholine) and 1-ethoxyethane-2-yl group. As an alkyl ester group, 1-acetoxyethane-2-yl group (acetylcholine) is preferred.

As a functional group other than the hydrogen-bonding functional group(s) present in a cation, there may be employed, for example, an alkyl group. It is preferred that such alkyl group be a linear or branched group having 1 to 18 carbon atoms, more preferably a linear or branched group having 1 to 12 carbon atoms, even more preferably a linear or branched group having 1 to 8 carbon atoms, particularly preferably a linear or branched group having 1 to 4 carbon atoms. Although not particularly limited, examples of the alkyl group include a methyl group, ethyl group, propane-1-yl group, propane-2-yl group, butane-1-yl group, 2-methylpropane-1-yl group, butane-2-yl group, 2-methylpropane-1-yl group, pentane-1-yl group, 1-methylbutane-1-yl group, 2-methylbutane-1-yl group, 3-methylbutane-1-yl group, 1-ethylbutane-1-yl group, 1,1-dimethylpropane-1-yl group, 1,2-dimethylpropane-1-yl group, 2,2-dimethylpropane-1-yl group,

hexane-1-yl group, heptane-1-yl group, octane-1-yl group, octane-1-yl group, nonane-1-yl group, decane-1-yl group, dodecane-1-yl group, tetradecane-1-yl group, hexadecane-1-yl group and octadecane-1-yl group.

While it is also a preferable embodiment to have the cation in the organic ammonium salt used in the present invention all substituted by alkyl groups, in terms of the effects of the present invention i.e. achieving a water and moisture retention property, it is preferred that at least one hydrogen-bonding functional group-containing alkyl group and/or hydrogen atom directly bonded to nitrogen be present in the cation of the organic ammonium salt used in the present invention. In this case, it is preferred that at least one moiety in the cation into which a functional group can be introduced (atoms contained in chemical structures as basic skeletons, such as a nitrogen moiety, and a carbon moiety composing a ring together with nitrogen) be substituted by a hydrogen-bonding functional group-containing alkyl group(s), and the rest of those moieties be substituted by an alkyl group(s). Further, it is more preferred that they are only composed of hydrogen-bonding functional group-containing alkyl groups and/or hydrogen atoms directly bonded to nitrogen; it is even more preferred that there is contained at least one hydrogen atom directly bonded to nitrogen. A particularly preferable cation structure is a cation composed of hydrogen-bonding functional group-containing alkyl groups and hydrogen atoms directly bonded to nitrogen.

The aforementioned hydrogen-bonding functional group is preferably a hydroxy group.

As the cation of the organic ammonium salt, preferred are an ammonium cation, imidazolium cation, pyridinium cation, pyrrolidinium cation, piperidinium cation and morpholinium cation, among which an ammonium cation is more preferred.

As for the cosmetic compounding agent of the present invention, it is preferred that the cation of the organic ammonium salt be an ammonium cation represented by the following formula (I).

[Chemical formula 1]

$$N^+[R^1]_n[R^2]_{4-n} \qquad (I)$$

(In this formula, each $R^1$ independently represents a hydroxyalkyl group in which there is at least one hydroxy group, an alkyl moiety is a linear or branched moiety having 1 to 10 carbon atoms, and such alkyl moiety may contain an oxygen atom(s); a carboxyalkyl group in which there is at least one carboxy group, an alkyl moiety is a linear or branched moiety having 1 to 10 carbon atoms, and such alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and such alkyl moiety may contain an oxygen atom(s). Each $R^2$ independently represents a hydrogen atom or a linear or branched alkyl group having 1 to 18 carbon atoms. n represents an integer of 0 to 4.)

[1] In the formula (I), it is preferred that n be an integer of 1 to 4.
[2] In [1], it is preferred that $R^2$ be a hydrogen atom.
[3] In [1], it is preferred that $R^1$ be a monohydroxyalkyl group or monocarboxyalkyl group whose alkyl moiety is a linear or branched moiety having 1 to 10 carbon atoms. As for the number of the carbon atoms in the alkyl moiety, it is preferred that there are 1 to 6, more preferably 1 to 4 carbon atoms in the case of a monohydroxyalkyl group; and it is preferred that there are 1 to 6, more preferably 1 to 5 carbon atoms in the case of a monocarboxyalkyl group. Among them, a monohydroxyalkyl group is preferred.
[4] In [3], it is preferred that $R^2$ be a hydrogen atom.
[5] In [1], it is preferred that $R^2$ be a linear or branched alkyl group having 1 to 18 carbon atoms. it is preferred that the alkyl group have 1 to 18, more preferably 1 to 12, even more preferably 1 to 8, particularly preferably 1 to 4 carbon atoms.
[6] In [1], it is preferred that at least one $R^1$ be a polyhydroxyalkyl group in which there are at least two hydroxy groups, and an alkyl moiety is a linear or branched moiety having 1 to 10 carbon atoms. The number of the hydroxy groups is preferably 2 to 8, more preferably 2 to 4, even more preferably 2 to 3. The number of the carbon atoms in the alkyl moiety is preferably 1 to 6, more preferably 1 to 4. n is preferably an integer of 1 to 2.
[7] In [6], it is preferred that $R^2$ be a hydrogen atom.

1-2. Anion

Although not particularly limited, examples of anions of the organic ammonium salt used in the present invention include a halogen-based anion, a sulfur-based anion, a phosphorus-based anion, a cyan-based anion, a boron-based anion, a fluorine-based anion, a nitrogen oxide-based anion and a carboxylic acid-based anion, among which preferred are a halogen-based anion, a sulfur-based anion, a boron-based anion and a carboxylic acid-based anion, more preferred are a halogen-based anion, a sulfur-based anion and a carboxylic acid-based anion, and even more preferred is a carboxylic acid-based anion.

Although not particularly limited, examples of the halogen-based anion include a chloride ion, bromide ion and

iodine ion.

Although not particularly limited, examples of the sulfur-based anion include a sulfonate anion, a hydrogen sulfonate anion, an alkyl sulfonate anion (e.g. methane sulfonate, ethyl sulfonate, butyl sulfonate, benzene sulfonate, p-toluene sulfonate, 2,4,6-trimethylbenzene sulfonate, styrene sulfonate, 3-sulfopropyl methacrylate anion and 3-sulfopropyl acrylate), a sulfate anion, a hydrogen sulfate anion and an alkyl sulfate anion (e.g. methyl sulfate anion, ethyl sulfate anion, butyl sulfate anion, octyl sulfate anion, 2-(2-methoxyethoxy)ethyl sulfate anion).

Although not particularly limited, examples of the phosphorus-based anion include a phosphate anion, a hydrogen phosphate anion, a dihydrogen phosphate anion, a phosphonate anion, a hydrogen phosphonate anion, a dihydrogen phosphonate anion, a phosphinate anion, a hydrogen phosphinate anion, an alkyl phosphate anion (e.g. dimethylphosphate, diethylphosphate, dipropylphosphate anion and dibutylphosphate anion), an alkyl phosphonate anion (e.g. methylphosphonate anion, ethylphosphonate anion, propylphosphonate anion, butylphosphonate anion and methylmethylphosphonate anion), an alkylphosphinate anion and a hexaalkylphosphate anion.

Although not particularly limited, examples of the cyan-based anion include a tetracyanoborate anion, a dicyanamide anion, a thiocyanate anion and an isothiocyanate anion.

Although not particularly limited, examples of the boron-based anion include a tetraalkylborate anions such as a tetrafluoroborate anion, a bisoxalateborate anion and a tetraphenylborate anion.

Although not particularly limited, examples of the fluorine-based anion include a bis(fluorosulfonyl)imide anion, a bis(perfluoroalkylsulfonyl)imide anion (e.g. bis(trifluoromethylsulfonyl)imide anion, bis(pentafluoroethylsulfonyl)imide, bis(heptafluoropropanesulfonyl)imide anion and bis(nonafluorobutylsulfonyl)imide), a perfluoroalkyl sulfonate anion (e.g. trifluoromethane sulfonate anion, pentafluoroethane sulfonate anion, heptafluoropropane sulfonate anion, nonaflate anion and perfluoro octane sulfonate anion), a fluorophosphate anion (e.g. hexafluorophosphate anion and tri(pentafluoroethyl)trifluorophosphate anion), a tris(perfluoroalkylsulfonyl)methide anion (e.g. tris(trifluoromethanesulfonyl)methide anion, tris(pentafluoroethanesulfonyl)methide anion, tris(heptafluoropropanesulfonyl)methide anion and tris(nonafluorobutanesulfonyl)methide anion) and a fluorohydrogenate anion.

In terms of the effects of the present invention i.e. achieving a water and moisture retention property, the anion may employ a halogen atom-containing boron-based anion or a halogen-based anion; if used as a liquid at 25°C, it is more preferred that the anion be a halogen atom-containing boron-based anion.

In terms of the effects of the present invention i.e. achieving a water and moisture retention property, if the anion is a halogen atom-containing boron-based anion or a halogen-based anion, a bromide ion is preferred more than a chloride ion, and a halogen atom-containing boron-based anion is even more preferred.

Although not particularly limited, examples of the nitrogen oxide-based anion include a nitrate anion and a nitrite anion.

The aforementioned carboxylic acid-based anion is an organic acid anion that has, in one molecule, at least one carboxylic acid anion ($-COO^-$), and may contain an oxygen-containing group, a nitrogen-containing group, a sulfur-containing group and/or a phosphorus-containing group, among which an oxygen-containing group is preferred. Even as such oxygen-containing group, a hydroxy group and a carboxy group are more preferred, where a hydroxy group is even more preferred. Although not particularly limited, examples of the carboxylic acid-based anion include a saturated aliphatic carboxylic acid anion, an unsaturated aliphatic carboxylic acid anion, an alicyclic carboxylic acid anion, an aromatic carboxylic acid anion, a saturated aliphatic hydroxycarboxylic acid anion, an unsaturated aliphatic hydroxycarboxylic acid anion, an alicyclic hydroxycarboxylic acid anion, an aromatic hydroxycarboxylic acid anion, a carbonyl carboxylic acid anion, an alkyl ether carboxylic acid anion, a halogen carboxylic acid anion and an amino acid anion. (The number of the carbon atoms in the carboxylic acid anions listed below include the number of the carbon atoms in carboxy groups.)

The aforementioned saturated aliphatic carboxylic acid anion is comprised of a linear or branched aliphatic saturated hydrocarbon group and at least one carboxylic acid anion, may contain a carboxy group and a carboxylate group, and preferably has 1 to 22 carbon atoms. Although not particularly limited, specific examples thereof include anions obtained by dissociating protons from, for example, formic acid, acetic acid, propionic acid, butyric acid, valeric acid, caproic acid, enanthic acid, caprylic acid, pelargonic acid, capric acid, lauric acid, myristic acid, pentadecylic acid, palmitic acid, margaric acid, stearic acid, arachidic acid, heneicosylic acid, behenic acid, isobutyric acid, 2-methylbutyric acid, isovaleric acid, 2-ethylhexanoic acid, isononanoic acid, isopalmitic acid, isostearic acid, oxalic acid, malonic acid, succinic acid, glutaric acid, adipic acid, pimelic acid, suberic acid, azelaic acid and sebacic acid.

The aforementioned unsaturated aliphatic carboxylic acid anion is comprised of a linear or branched aliphatic unsaturated hydrocarbon group and at least one carboxylic acid anion, may contain a carboxy group and a carboxylate group, and preferably has 3 to 22 carbon atoms. Although not particularly limited, specific examples thereof include anions obtained by dissociating protons from, for example, acrylic acid, methacrylic acid, crotonic acid, palmitoleic acid, oleic acid, vaccenic acid, linoleic acid, linolenic acid, eleostearic acid, arachidonic acid, maleic acid and fumaric acid.

The aforementioned alicyclic carboxylic acid anion is comprised of a non-aromatic saturated or unsaturated carbon ring and at least one carboxylic acid anion, and preferably has 6 to 20 carbon atoms. Particularly, an alicyclic

carboxylic acid anion having a cyclohexane ring skeleton is preferred; although not particularly limited, specific examples thereof include anions obtained by dissociating protons from, for example, cyclohexanecarboxylic acid and cyclohexanedicarboxylic acid.

The aforementioned aromatic carboxylic acid anion is comprised of one or multiple aromatic rings and at least one carboxylic acid anion, and preferably has 6 to 20 carbon atoms. Particularly, an aromatic carboxylic acid anion having a benzene ring skeleton is preferred; although not particularly limited, specific examples thereof include anions obtained by dissociating protons from, for example, benzoic acid, cinnamic acid, phthalic acid, isophthalic acid and terephthalic acid.

The aforementioned saturated aliphatic hydroxycarboxylic acid anion is comprised of a linear or branched aliphatic saturated hydrocarbon group, at least one carboxylic acid anion and at least one hydroxy group, may contain a carboxy group and a carboxylate group, and preferably has 2 to 24 carbon atoms. Particularly, a saturated aliphatic hydroxycarboxylic acid anion having 1 to 4 hydroxy groups and 2 to 7 carbon atoms is preferred. Although not particularly limited, specific examples thereof include anions obtained by dissociating protons from, for example, glycolic acid, lactic acid, tartronic acid, glyceric acid, hydroxyacetic acid, hydroxybutyric acid, 2-hydroxydecanoic acid, 3-hydroxydecanoic acid, 12-hydroxystearic acid, dihydroxystearic acid, cerebronic acid, malic acid, tartaric acid, citramalic acid, citric acid, isocitric acid, leucine acid, mevalonic acid and pantoic acid.

The aforementioned unsaturated aliphatic hydroxycarboxylic acid anion is comprised of a linear or branched aliphatic unsaturated hydrocarbon group, at least one carboxylic acid anion and at least one hydroxy group, and preferably has 3 to 22 carbon atoms. Although not particularly limited, specific examples thereof include anions obtained by dissociating protons from, for example, ricinolic acid, ricinoleic acid, and ricineraidic acid.

The aforementioned alicyclic hydroxycarboxylic acid anion is comprised of a non-aromatic saturated or unsaturated carbon ring, at least one carboxylic acid anion and at least one hydroxy group, and preferably has 4 to 20 carbon atoms. Particularly, an alicyclic hydroxycarboxylic acid anion having a six-membered ring skeleton and 1 to 4 hydroxy groups is preferred; although not particularly limited, specific examples thereof include anions obtained by dissociating protons from, for example, hydroxycyclohexanecarboxylic acid, dihydroxycyclohexanecarboxylic acid, quinic acid (1,3,4,5-tetrahydroxycyclohexanecarboxylic acid) and shikimic acid. Further, anions obtained by dissociating protons from hydroxy group-containing cyclic lactones may also be preferably used; although not particularly limited, specific examples thereof include anions obtained by dissociating protons from, for example, ascorbic acid and erythorbic acid.

The aforementioned aromatic hydroxycarboxylic acid anion is comprised of one or multiple aromatic rings and at least one carboxylic acid anion, and preferably has 6 to 20 carbon atoms. Particularly, an aromatic carboxylic acid anion having a benzene ring skeleton and 1 to 3 hydroxy groups is preferred; although not particularly limited, specific examples thereof include anions obtained by dissociating protons from, for example, salicylic acid, hydroxybenzoic acid, dihydroxybenzoic acid, trihydroxybenzoic acid, hydroxymethylbenzoic acid, vanillic acid, syringic acid, protocatechuic acid, gentisic acid, orsellinic acid, mandelic acid, benzylic acid, atrolactic acid, phloretic acid, coumaric acid, umbellic acid, caffeic acid, ferulic acid and sinapic acid.

The aforementioned carbonyl carboxylic acid anion is a carboxylic acid anion having a carbonyl group(s) in a molecule and having 3 to 22 carbon atoms; preferred is a carbonyl carboxylic acid anion having 1 to 2 carbonyl groups and 3 to 7 carbon atoms. Particularly, preferred is a carbonyl carboxylic acid anion represented by $CH_3((CH_2)_pCO(CH_2)_q)COO^-$ (p and q each represent an integer of 0 to 2). Although not particularly limited, specific examples thereof include anions obtained by dissociating protons from, for example, levulinic acid and pyruvic acid.

The aforementioned alkyl ether carboxylic acid anion is a carboxylic acid anion having an ether group(s) in a molecule and having 2 to 22 carbon atoms, including a polyoxyalkylene alkyl ether carboxylic acid anion(s); preferred is an alkyl carboxylic acid anion having 1 to 2 ether groups and 2 to 12 carbon atoms. Particularly, preferred is an alkyl ether carboxylic acid anion or polyoxyethylene alkyl ether carboxylic acid anion represented by $CH_3(CH_2)_rO(CH_2)_sCOO^-$ (r and s each represent an integer of 0 to 4). Although not particularly limited, specific examples thereof include anions obtained by dissociating protons from, for example, methoxyacetic acid, ethoxyacetic acid, methoxybutyric acid and ethoxybutyric acid.

As the aforementioned halogen carboxylic acid anion, a halogen carboxylic acid anion having 2 to 22 carbon atoms is preferred. Although not particularly limited, specific examples thereof include anions obtained by dissociating protons from, for example, fluorine-substituted halogen carboxylic acids such as trifluoroacetic acid, trichloroacetic acid, tribromoacetic acid, pentafluoropropionic acid, pentachloropropionic acid, pentabromopropionic acid, perfluorononanoic acid, perchlorononanoic acid and perbromononanoic acid.

Although not particularly limited, examples of the aforementioned amino acid anion include anions obtained by dissociating protons from, for example, glycine, alanine, glutamic acid, N-acetylglutamic acid, arginine, asparagine, aspartic acid, isoleucine, glutamine, histidine, cysteine, leucine, lysine, proline, phenylalanine, threonine, serine, tryptophan, tyrosine, methionine, valine, sarcosine, aminobutyric acid, methylleucine, aminocaprylic acid, aminohexanoic acid, norvaline, aminovaleric acid, aminoisobutyric acid, thyroxine, creatine, ornithine, opine, theanine, tricholomine, kainic acid, domoic acid, ibotenic acid, acromelic acid, cystine, hydroxyproline, phosphoserine and desmosine.

In terms of the effects of the present invention i.e. an adherability and affinity of the hair treatment agent to the hair; a water and moisture retention property as well as an antistatic property that are brought to the hair by the hair treatment agent; an adherability to and stabilization of the proteins of the hair; a thermal stability; and a solubility of active ingredients, in other words, in terms of senses such as a finger-combing capability, flexibility, texture, elasticity, resilience, volume increase, cohesiveness, moist feeling, gloss, smoothness and non-stickiness of the hair, it is preferred that the anion be any of the aforementioned halogen-based anion, sulfur-based anion, phosphorus-based anion, cyan-based anion, nitrogen oxide-based anion and carboxylic acid-based anion, among which the halogen-based anion, sulfur-based anion, phosphate-based anion, boron-based anion and carboxylic acid-based anion are preferred, the carboxylic acid-based anion and halogen-based anion are more preferred, and the carboxylic acid-based anion is even more preferred.

In terms of the effects of the present invention i.e. a short- or long-term water and moisture retention property, a moisture barrier property of skin, an antistatic property and a high solubility of a particular active ingredient(s) that are brought about by the skin care agent, in other words, in terms of, for example, senses such as a persistent moisture retention feeling, a non-stickiness and a skin compatibility; a high affinity and permeability to skin; a high safety as being low-irritating; and a whitening effect, it is preferred that the anion be any of the aforementioned sulfur-based anion, phosphorus-based anion, cyan-based anion, nitrogen oxide-based anion, carboxylic acid-based anion and halogen-based anion, among which the sulfur-based anion, phosphorus-based anion and carboxylic acid-based anion are more preferred, the phosphorus-based anion and carboxylic acid-based anion are even more preferred, and the carboxylic acid-based anion is particularly preferred.

The aforementioned halogen-based anion, sulfur-based anion, phosphorus-based anion, cyan-based anion, nitrogen oxide-based anion, boron-based anion, carboxylic acid-based anion and the like are preferred, among which the halogen-based anion, sulfur-based anion, phosphate-based anion, boron-based anion and carboxylic acid-based anion are preferred, the halogen-based anion, sulfur-based anion, phosphate-based anion and carboxylic acid-based anion are more preferred, and the carboxylic acid-based anion is even more preferred.

In terms of the water and moisture retention effect, it is preferred that the carboxylic acid anion be a hydrophilic carboxylic acid anion; as such carboxylic acid anion, it is preferred that the number of the carbon atoms therein be not larger than 8, more preferably not larger than 6. It is preferred that a hydrogen-bonding functional group(s) be present in anions; and it is preferred that as a functional group, there be contained a hydrogen-bondable group(s) such as an oxygen-containing group, a nitrogen-containing group, a sulfur-containing group and a phosphorus-containing group. Specifically, preferred are a hydroxy group, an amino group, a carbonyl group, a carboxy group, a carboxylate group, a sulfonyl group, a sulfate ester group, a phosphate group and a phosphate ester group, among which more preferred are a hydroxy group, a carboxy group, a carboxylate group, a sulfonyl group and a phosphate group, even more preferred are a hydroxy group, a carboxy group and a carboxylate group, and particularly preferred is a hydroxy group. Although not particularly limited, as a carboxylic acid anion having not more than 8 carbon atoms, preferred are, for example, a formic acid anion, acetic acid anion, propionic acid anion, butyric acid anion, valeric acid anion, caproic acid anion, enanthic acid anion, caprylic acid anion, oxalic acid anion, malonic acid anion, succinic acid anion, glutaric acid anion, adipic acid anion, pimelic acid anion, suberic acid anion, acrylic acid anion, methacrylic acid anion, crotonic acid anion, cyclohexanecarboxylic acid anion, cyclohexanedicarboxylic acid anion, benzoic acid anion, phthalic acid anion, isophthalic acid anion, terephthalic acid anion, glycolic acid anion, lactic acid anion, tartronic acid anion, glyceric acid anion, hydroxyacetic acid anion, hydroxybutyric acid anion, cerebronic acid anion, malic acid anion, tartaric acid anion, citramalic acid anion, citric acid anion, isocitric acid anion, leucine acid anion, mevalonic acid anion, pantoic acid anion, hydroxycyclohexanecarboxylic acid anion, dihydroxycyclohexanecarboxylic acid anion, quinic acid(1,3,4,5-tetrahydroxycyclohexanecarboxylic acid) anion, shikimic acid anion, salicylic acid anion, hydroxybenzoic acid anion, dihydroxybenzoic acid anion, trihydroxybenzoic acid anion, hydroxymethylbenzoic acid anion, vanillic acid anion, syringic acid anion, protocatechuic acid anion, gentisic acid anion, orsellinic acid anion, mandelic acid anion, levulinic acid anion, pyruvic acid anion, methoxyacetic acid anion, ethoxyacetic acid anion, methoxybutyric acid anion, ethoxybutyric acid anion, trifluoroacetic acid anion, trichloroacetic acid anion, tribromoacetic acid anion, pentafluoropropionic acid anion, pentachloropropionic acid anion, pentabromopropionic acid anion, glycine anion, alanine anion, glutamic acid anion, N-acetylglutamic acid anion, arginine anion, asparagine anion, aspartic acid anion, isoleucine anion, glutamine anion, histidine anion, cysteine anion, leucine anion, lysine anion, proline anion, threonine anion, serine anion, methionine anion, valine anion, sarcosine anion, aminobutyric acid anion, methylleucine anion, aminocaprylic acid anion, aminohexanoic acid anion, norvaline anion, aminovaleric acid anion, aminoisobutyric acid anion, creatine anion, ornithine anion, opine anion, theanine anion, tricholomine anion, ibotenic acid anion, cystine anion, hydroxyproline anion and phosphoserine anion; as a carboxylic acid anion having not more than 6 carbon atoms, more preferred are, for example, a formic acid anion, acetic acid anion, propionic acid anion, butyric acid anion, valeric acid anion, caproic acid anion, oxalic acid anion, malonic acid anion, succinic acid anion, glutaric acid anion, adipic acid anion, acrylic acid anion, methacrylic acid anion, crotonic acid anion, cyclohexanecarboxylic acid anion, glycolic acid anion, lactic acid anion, tartronic acid anion, glyceric acid anion, hydroxyacetic acid anion, hydroxybutyric acid anion, cerebronic acid anion,

malic acid anion, tartaric acid anion, citramalic acid anion, citric acid anion, isocitric acid anion, leucine acid anion, mevalonic acid anion, pantoic acid anion, hydroxycyclohexanecarboxylic acid anion, dihydroxycyclohexanecarboxylic acid anion, levulinic acid anion, pyruvic acid anion, methoxyacetic acid anion, ethoxyacetic acid anion, methoxybutyric acid anion, ethoxybutyric acid anion, trifluoroacetic acid anion, trichloroacetic acid anion, tribromoacetic acid anion, pentafluoropropionic acid anion, pentachloropropionic acid anion, pentabromopropionic acid anion, glycine anion, alanine anion, glutamic acid anion, N-acetylglutamic acid anion, arginine anion, asparagine anion, aspartic acid anion, isoleucine anion, glutamine anion, histidine anion, cysteine anion, leucine anion, lysine anion, proline anion, threonine anion, serine anion, methionine anion, valine anion, sarcosine anion, aminobutyric acid anion, methylleucine anion, aminohexanoic acid anion, norvaline anion, aminovaleric acid anion, aminoisobutyric acid anion, creatine anion, ornithine anion, opine anion, theanine anion, tricholomine anion, ibotenic acid anion, cystine anion, hydroxyproline anion and phosphoserine anion; as a hydroxy group-containing carboxylic acid anion, particularly preferred are, for example, a glycolic acid anion, lactic acid anion, tartronic acid anion, glyceric acid anion, hydroxyacetic acid anion, hydroxybutyric acid anion, cerebronic acid anion, malic acid anion, tartaric acid anion, citramalic acid anion, citric acid anion, isocitric acid anion, leucine acid anion, mevalonic acid anion, pantoic acid anion, hydroxycyclohexanecarboxylic acid anion, dihydroxycyclohexanecarboxylic acid anion, quinic acid(1,3,4,5-tetrahydroxycyclohexanecarboxylic acid) anion, shikimic acid anion, salicylic acid anion, hydroxybenzoic acid anion, dihydroxybenzoic acid anion, trihydroxybenzoic acid anion, hydroxymethylbenzoic acid anion, vanillic acid anion, syringic acid anion, protocatechuic acid anion, gentisic acid anion, orsellinic acid anion, mandelic acid anion, tricholomine anion, ibotenic acid anion, hydroxyproline anion, serine anion and threonine anion, among which more preferred are a glycolic acid anion, lactic acid anion, malic acid anion, tartaric acid anion, citric acid anion and quinic acid anion.

Further, in order to improve the effects of the present invention, it is particularly preferred that the organic ammonium salt used in the present invention be an organic salt having a hydrogen-bonding functional group(s) in both a cation and an anion.

Meanwhile, in terms of an emollient effect of the skin care agent, a barrier property brought about thereby, a solubility of an oil-soluble ingredient(s), an improvement in stability of an emulsification composition, an improvement in feeling of use, a cleansing effect and a retention of skin moisture, a lipophilic carboxylic acid anion is preferable; as such carboxylic acid anion, though depending on the number of the substituent groups such as a hydroxy group(s), for example, in a case of a saturated or unsaturated aliphatic carboxylic acid anion having one carboxylic acid anion, it is preferred that the number of the carbon atoms thereof be not smaller than 8, more preferably not smaller than 12, even more preferably not smaller than 18. Although not particularly limited, specific and preferable examples of such lipophilic carboxylic acid anion include a caprylic acid anion, pelargonic acid anion, capric acid anion, lauric acid anion, myristic acid anion, pentadecylic acid anion, palmitic acid anion, margaric acid anion, stearic acid anion, isostearic acid anion, arachidic acid anion, heneicosylic acid anion, behenic acid anion, palmitoleic acid anion, oleic acid anion, vaccenic acid anion, linoleic acid anion and linolenic acid anion, among which more preferred are an oleic acid anion, linoleic acid anion and isostearic acid anion.

1-3. Supplementary notes on cation and anion

In terms of safety, it is preferred that the organic ammonium salt used in the cosmetic compounding agent of the present invention employ a natural compound in a cation and/or an anion. Although not particularly limited, examples of such cation include a choline cation, acetylcholine cation, acetylthiocholine cation, carnitine cation, betaine, sulfobetaine, ethyl(2-methoxyethyl)dimethylammonium cation, benzylcinchonidinium and carbamyl-β-methylcholine cation; examples of such anion include a citric acid anion, lactic acid anion, malic acid anion, ascorbic acid anion, glycolic acid anion, tartaric acid anion, quinic acid anion, acetic acid anion, butyric acid anion, caproic acid anion, caprylic acid anion, capric acid anion, succinic acid anion, oleic acid anion, linoleic acid anion, alanine anion and glycine anion; examples of amino acids that can be used both as cations and anions include glycine, alanine, glutamic acid, N-acetylglutamic acid, arginine, asparagine, aspartic acid, isoleucine, glutamine, histidine, cysteine, leucine, lysine, proline, phenylalanine, threonine, serine, tryptophan, tyrosine, methionine, valine, sarcosine, aminobutyric acid, methylleucine, aminocaprylic acid, aminohexanoic acid, norvaline, aminovaleric acid, aminoisobutyric acid, thyroxine, creatine, ornithine, opine, theanine, tricholomine, kainic acid, domoic acid, ibotenic acid, acromelic acid, cystine, hydroxyproline, phosphoserine and desmosine. Although not particularly limited, specific examples of combinations of a cation and an anion include trisacetate, trisisostearate, trisoleate, trislinoleate, trislactate, trisglycolate, trissuccinate, trismalate, tristartrate, trisfumarate, trisascorbate, triscitrate, 2-amino-2-methyl-1,3-propanediol acetate, 2-amino-2-methyl-1,3-propanediol isostearate, 2-amino-2-methyl-1,3-propanediol oleate, 2-amino-2-methyl-1,3-propanediol linoleate, 2-amino-2-methyl-1,3-propanediol lactate, 2-amino-2-methyl-1,3-propanediol glycolate, 2-amino-2-methyl-1,3-propanediol succinate, 2-amino-2-methyl-1,3-propanediol malate, 2-amino-2-methyl-1,3-propanediol tartrate, 2-amino-2-methyl-1,3-propanediol fumarate, 2-amino-2-methyl-1-propanol lactate, γ-aminobutyric acid lactate, ε-aminocaproic acid lactate, monoethanolamine lactate, diethanolamine lactate, diethanolamine glycolate, diethanolamine succinate, 2- amino-1,3-propanediol acetate, 2-amino-1,3-propanediol glycolate, 2-amino-1,3-propanediol succinate, 2-amino-1,3-propanediol citrate, triethanolamine lactate, triethanolamine glycolate, triethanolamine citrate, 2-amino-1,3-propanediol lactate, 2-amino-2-ethyl-1,3-propanediol lactate, 1-amino-1-deoxy-D-glucitol lactate, ammonium lactate, tetraethanolamine lactate, tris(2,3-dihydroxypropyl)-1-hydroxy-2-(hydroxymethyl)-2-butanaminium lactate,
choline methanesulfonate, choline phosphate, choline hypophosphite and choline hydrochloride; it is particularly preferred that both the cations and anions be natural ones such as choline acetate, choline glycolate, choline lactate, choline succinate, choline tartrate and choline ascorbate.

In terms of safety, if the cations have a monohydroxyalkyl(s), preferred are a triethanolammonium cation and diethanolammonium cation, particularly preferred is a triethanolammonium cation.

Further, in terms of safety and usage, it is preferred that the organic ammonium salt used in the present invention employ, as a raw material(s) (e.g. acids, bases), compounds listed in Japanese standards of quasi-drug ingredients (JSQI), Japanese standards of quasi-drug additives, Japanese pharmacopoeia (JP), Japanese pharmaceutical codex (JPC), Japanese pharmaceutical excipients (JPE) and Japan's specifications and standards for food additives (JSFA). It is more preferred that there be used an organic ammonium salt whose raw material(s) composing the cations and/or anions are those described in JSQI, JP, JPC, JPE and JSFA; and an organic ammonium salt described in JSQI. Although not particularly limited, preferable examples of a cation include a monoethanolammonium cation, diethanolammonium cation, triethanolammonium cation, 2-amino-2-hydroxymethyl-1,3-propanediol ammonium cation, 2-amino-2-methyl-1-propanol ammonium cation and 2-amino-2-methyl-1,3-propanediol ammonium cation; in terms of low odor, more preferred are a 2-amino-2-hydroxymethyl-1,3-propanediol ammonium cation and 2-amino-2-methyl-1,3-propanediol ammonium cation. Further, examples of an anion include an acetic acid anion, caprylic acid anion, capric acid anion, lauric acid anion, myristic acid anion, palmitic acid anion, stearic acid anion, oleic acid anion, linoleic acid anion, lactic acid anion, glycolic acid anion, succinic acid anion, citric acid anion, chloride anion, fumaric acid anion, phosphoric acid anion and ascorbic acid anion. Moreover, examples of amino acids that can be used as a cation and an anion include glycine, alanine, arginine, aspartic acid, histidine, cysteine, proline, serine, tryptophan, tyrosine, methionine, aminobutyric acid, aminohexanoic acid, cystine, glutamic acid, isoleucine, phenylalanine, threonine, tryptophan, methionine, valine and theanine.

1-4. Organic ammonium salt and cosmetic compounding agent

As for the cosmetic compounding agent of the present invention, the organic ammonium salt may be in an anhydrous state (anhydride) or a hydrate that has absorbed the water in the air. A hydrate refers to a compound whose moisture rate has reached a saturated state after being left in the air at 25°C and absorbing the water therein. A compound that does not absorb water when left in the air at 25°C is not a hydrate, but an anhydride.

When the organic ammonium salt used in the present invention is a hydrate, the water content in the hydrate will be suppressed from evaporating such that the water and moisture retention effect shall last for a long period of time. Thus, after dissolving a water of an excess amount against a hydration water in the organic ammonium salt used in the present invention and then leaving the same in the air so as to observe a decrease in water, since the hydration water is suppressed from evaporating, a rate of decrease in water content shall decrease with time.

The organic ammonium salt used in the present invention may be either a liquid or a solid at 25°C. When an anhydride and hydrate thereof are liquids at 25°C, these liquid anhydride, hydrate or dilution of the organic ammonium salt will not cause any usage problems such as a problem that the crystals of the organic ammonium salt may be precipitated, aggregated or solidified. When the cosmetic compounding agent is a hair treatment agent, the effects of the present invention such as the water and moisture retention property, adherability, antistatic property (static protection property) and stabilization of the proteins of the hair; and the effects of an additive(s) dissolved therein can be exhibited immediately after the use of the agent in a more effective manner and for a long period of time, due to the fact that even after other solvents mixed therein and water have volatilized, the agent can still be evenly applied to and thus coat the surface of the hair as a liquid, since the organic ammonium salt itself is non-volatile. Further, in terms of the adherability of the hair treatment agent, it is preferred that a compounding molar ratio between the acid and base forming the organic ammonium salt be 1:5 to 5:1, more preferably 1:1. When the cosmetic compounding agent is a skin care agent, the effects of the present invention such as the water and moisture retention property, antistatic property (static protection property), the high affinity to skin and the high solubility of active ingredients; and the effects of an additive(s) dissolved therein can be exhibited immediately after the use of the agent in a more effective manner and for a long period of time, due to the fact that even after other solvents mixed therein and water have volatilized, the agent can still be evenly applied to and thus coat the surface of skin as a liquid, since the organic ammonium salt itself is non-volatile. Further, in terms of safety and damage control of the skin, hair or the like, it is preferred that the cosmetic compounding agent have a pH level of 3 to 10, more preferably 5 to 9, even more preferably 6 to 8. Although not particularly limited, for example, when the pH level thereof is 3 to 10, the compounding molar ratio between the acid and base forming the organic ammonium salt is 2:1 to 1:5; when the pH level is 5 to 9, such molar ratio is 1:2 to 1:1; when the pH level is 6

to 8, such molar ratio is 1:1.

The effects of the organic ammonium salt or the ingredients dissolved therein can be exerted on a target of the cosmetic, such as the hair and skin in a more effective manner and for a long period of time. For example, these effects can be sufficiently exhibited even when the agent is used in a small amount where a low-concentration composition is employed. If the organic ammonium salt is a liquid at 25°C, when used with other additive(s) (e.g. poorly-soluble active ingredients), the organic ammonium salt can be used as a base agent, solvent and a carrier toward the inner portion of a target. Further, let alone the organic ammonium salt, the active ingredients dissolved therein are also superior in permeability into the hair and skin. In terms of solubility of the active ingredients, it is preferred that the compounding molar ratio between the acid and base composing the organic ammonium salt of the cosmetic compounding agent be 1:1 to 2:1, more preferably 1:1. The melting point (freezing point) of the organic ammonium salt used in the present invention is preferably lower than 25°C, more preferably lower than - 5°C, particularly preferably lower than -10°C.

The organic ammonium salt used in the present invention is superior in permeability into targets such as the hair and skin, is useful in terms of the water and moisture retention effect, and can also be used as a carrier of the active ingredients.

The organic ammonium salt used in the present invention is preferable in terms of maintaining the water and moisture retention effect for a long period of time, because the organic ammonium salt does not volatilize even under a low-humidity environment and will thus remain so as to be able to retain the water content for a long period of time. In terms of imparting the water and moisture retention effect to the hair, since the surface of the hair is negatively charged, the cation of the organic ammonium salt of the present invention shall interact therewith such that the organic ammonium salt can then be immobilized on the surface of the hair for a long period of time. In addition, in terms of imparting the water and moisture retention effect to skin, the skin application of the organic ammonium salt is also preferable because the hydrogen-bonding functional groups in the compound of the present invention have a high affinity with (are bondable with) hydroxy groups, carbonyl groups and amino residues such as amino groups in the proteins on the skin surface such that coating can take place in a favorable manner, and an immobilized state will remain for a long period of time; and because a high permeability will be exhibited due to a small molecular size. Further, the organic ammonium salt is also preferable in a sense that when the skin surface is negatively charged, the cation of the organic ammonium salt of the present invention shall interact therewith such that the organic ammonium salt can then be immobilized on the skin surface for a long period of time.

Moreover, since the proteins on the surface of the hair has functional groups capable of interacting and bonding with hydrogen atoms; and with the hydrogen-bonding functional groups in the organic ammonium salt of the present invention, such as an oxygen-containing group, a nitrogen-containing group, a sulfur-containing group and a phosphorus-containing group, the organic ammonium salt having a hydrogen-bonding functional group(s) in a cation and/or an anion can bond and interact with, for example, the carboxyl groups, carbonyl groups and amino groups of the hair, and thus be favorably immobilized for a long period of time.

The organic ammonium salt used in the cosmetic compounding agent of the present invention is superior in hydrophilicity, and has a small molecular size and a high osmotic pressure, thereby being superior in, for example, permeability into the skin, hair or the like. Since the surface of skin or the like is either negatively or positively charged, and the surface of the hair or the like is negatively charged, the organic ammonium salt used in the present invention is favorable as it can be easily adsorbed thereto due to the salt structure thereof and the fact that it has a hydrogen-bonding functional group(s).

Since the cosmetic compounding agent of the present invention has a high water retention property and is non-volatile, it is superior in barrier property and can thus prevent drying for a long period of time. Particularly, a higher barrier property can be achieved if the agent has a low melting point and is a liquid at 25°C. Further, the cosmetic compounding agent of the present invention (hair treatment agent, skin care agent) may be (1) a cosmetic compounding agent (hair treatment agent, skin care agent) produced by preparing the organic ammonium salt by mixing a base with an acid and even with a solvent, if necessary, and then mixing the organic ammonium salt with other ingredients, if necessary; or (2) a cosmetic compounding agent (hair treatment agent, skin care agent) produced in a manner where the organic ammonium salt is not previously prepared, but formed in a system in which a base and an acid are present as individual ingredients and are separately mixed with a solvent as well as other ingredients, if necessary.

The cosmetic compounding agent of the present invention is mainly directed to one added as a raw material to a cosmetic as a product.

Although not particularly limited, examples of a cosmetic compounding agent include a hair treatment agent added as a raw material to a hair treatment composition as a product suitable for use in the hair or the like; and a skin care agent added as a raw material to a skin care composition as a product suitable for use in skin or the like.

When the cosmetic compounding agent of the present invention is a hair treatment agent, the hair treatment agent is added as a raw material to a hair treatment composition as a product suitable for use in the hair, such as a shampoo. The hair treatment agent of the present invention may be the aforementioned organic ammonium salt or a hydrate thereof; as the above raw material, the hair treatment agent may also be a composition containing this organic

ammonium salt. When the hair treatment agent of the present invention is a composition, it is preferred that the organic ammonium salt be contained in an amount of not smaller than 0.01% by mass, more preferably not smaller than 0.1% by mass, even more preferably not smaller than 1.0% by mass, particularly preferably not smaller than 10.0% by mass, in terms of anhydrates. While there are no particular restrictions on the mode of the composition, there may be employed, for example, a composition with the organic ammonium salt being dissolved or dispersed in, for example, water and/or a solvent.

When the cosmetic compounding agent of the present invention is a skin care agent, the skin care agent is added as a raw material to a skin care composition as a product suitable for use in skin, such as a lotion. The skin care agent of the present invention may be the aforementioned organic ammonium salt or a hydrate thereof; as the above compounding ingredient, the skin care agent may also be a composition containing this organic ammonium salt. When the skin care agent of the present invention is a composition, it is preferred that the organic ammonium salt be contained in an amount of not smaller than 0.001% by mass, more preferably not smaller than 0.01% by mass, even more preferably not smaller than 0.1% by mass, particularly preferably not smaller than 1.0% by mass, especially preferably not smaller than 10.0% by mass, in terms of anhydrates. While there are no particular restrictions on the mode of the composition, there may be employed, for example, a composition with the organic ammonium salt being dissolved or dispersed in, for example, water and/or a solvent.

The cosmetic compounding agent of the present invention may be a cosmetic compounding agent or cosmetic produced by preparing the organic ammonium salt in advance, and then mixing the organic ammonium salt with water and/or a solvent as well as with other ingredients; or a cosmetic compounding agent or cosmetic produced in a manner where the individual compounds as the raw materials of a cation and an anion are mixed with water and/or a solvent as well as with other ingredients.

Although not particularly limited, examples of the solvent include water, methanol, ethanol, propanol, isopropanol, butanol, ethylene glycol, propylene glycol, 1,3-butylene glycol, diethylene glycol, dipropylene glycol, isoprene glycol, hexylene glycol, glycerin, benzyl alcohol, methyl acetate, ethyl acetate, isopropyl acetate, ethyl ether, acetone, toluene, hexane, heptane and acetonitrile; if necessary, two or more of them may be used in combination. Further, the composition may also contain an additive(s); although not particularly limited, examples of such additive(s) include ingredients such as a pH adjuster, a colorant, resin particles, a surfactant, an oil agent, a viscosity regulator, a coloring agent, a preservative, a perfume, an ultraviolet absorber (organic and inorganic), a natural plant extract ingredient, a seaweed extract ingredient, a crude drug ingredient, an antioxidant and an insect repellent.

If necessary, a pH adjuster or the like may be added to the cosmetic compounding agent of the present invention to adjust the pH level thereof. Although not particularly limited, a natural compound is preferable as the pH adjuster. Examples of such natural pH adjuster include citric acid, lactic acid, malic acid, glycolic acid, tartaric acid, quinic acid, acetic acid, butyric acid, caproic acid, caprylic acid, capric acid, lauric acid, myristic acid, palmitic acid, stearic acid, succinic acid, oleic acid, linoleic acid, adipic acid, trisodium citrate, glucono delta lactone, gluconic acid, potassium gluconate, sodium gluconate, monosodium succinate, disodium succinate, sodium acetate, potassium hydrogen tartrate, sodium lactate, sodium dihydrogen pyrophosphate, phosphoric acid, sodium phosphate, potassium carbonate, sodium hydrogen carbonate, and amino acids such as alanine and glycine.

As for the organic ammonium salt used in the present invention, hydrophilic or lipophilic anions or cations may be appropriately selected depending on other ingredients to be mixed together as well as a required effect(s) of the invention. For example, in terms of water and moisture retention, and dissolving a water-soluble active ingredient(s), a hydrophilic organic ammonium salt is preferrable; in terms of the emollient effect, and dissolving an oil-soluble active ingredient(s), preferred is an organic ammonium salt employing a lipophilic anion or cation.

Among the organic ammonium salts used in the present invention, those that are liquids at room temperature shall also function as solvents or mediums for other compositions as well as the abovementioned additives.

Since the organic ammonium salt used in the present invention has a hydrogen-bonding functional group(s) such as an oxygen-containing group, a nitrogen-containing group, a sulfur-containing group and a phosphorus-containing group in a cation and/or an anion, the organic ammonium salt has an effect of stabilizing the higher-order structure of a protein contained in, for example, the hair, cuticle and nails, such as keratin. Due to changes in the secondary structure as the higher-order structure of a protein such as keratin, and due to changes in structures by bond breaking, the hair usually denatures, and will thus be susceptible to damages and exhibit an impaired texture accordingly. By having the hydrogen-bonding functional group(s) in the organic ammonium salt used in the present invention interact with a protein(s) in the hair such as keratin, the secondary structure can be maintained, and bond breaking can be suppressed, thereby making it possible to suppress the denaturalization of the proteins, thus allowing a texture after hair treatment to be maintained. Further, there can also be brought about an effect of stabilizing the proteins of the scalp.

The effects exerted on the proteins in the hair such as keratin by the organic ammonium salt used in the present invention can also be exhibited when the pH level is in an acidic region (hair manicure) and an alkaline region (hair color) in addition to a neutral region (e.g. hair shampoo).

When the cosmetic compounding agent of the present invention is a hair treatment agent and used on the hair,

due to the aforementioned effects of the organic ammonium salt of the invention, there will be imparted and exhibited sensory effects such as a flexibility, a finger-combing capability, an elasticity/resilience, a volume, a cohesiveness, a moist feeling, a surface smoothness, a gloss, an excellent texture and a low-stickiness. The hair treatment agent of the present invention has the abovementioned effects even with respect to a damaged hair that has been damaged by, for example, drying and coloring.

Since the organic ammonium salt used in the present invention has a hydrogen-bonding functional group(s) such as an oxygen-containing group, a nitrogen-containing group, a sulfur-containing group and a phosphorus-containing group in a cation and/or an anion, the organic ammonium salt is superior in affinity with the skin, cuticle, nails and mucous membranes, and is superior in short- or long-term water and moisture retention property and moisture barrier property due to the fact that the organic ammonium salt is non-volatile. Further, the organic ammonium salt provides a favorable feeling of use such as a moisture retention feeling, a low-stickiness and a fitting property, and has an excellent permeability as well as a high safety. Particularly, it is preferable to use, as raw materials, the compounds listed in Japanese standards of quasi-drug ingredients (JSQI), Japanese pharmacopoeia (JP), Japanese pharmaceutical codex (JPC), Japanese pharmaceutical excipients (JPE), Japanese standards of quasi-drug additives and Japan's specifications and standards for food additives (JSFA), because the safety of these compounds has been confirmed by these standards. Moreover, the solubility of the active ingredients is high, a whitening effect is imparted, and a superior antistatic property is imparted as well. As described above, by interacting with proteins such as keratin, the organic ammonium salt used in the present invention is capable of maintaining the secondary structure and suppressing bond breaking, thereby making it possible to suppress the denaturalization of the proteins. Thus, it can be used for the purpose of retaining water and moisture of the skin, cuticle, nails and mucous membranes (in oral and nasal cavities); and for the purpose of stabilizing the proteins.

By introducing, for example, a structure (acid, base) having a whitening effect into the cation and/or anion of the ammonium salt of the present application so as to turn the ammonium salt into the mode of an organic ammonium salt, these effects can then be achieved in a continuous and efficient manner in terms of non-volatility and permeability. Further, although ascorbic acid has a low stability, it will be stabilized in the mode of an ammonium salt, and will thus be able to further improve the effects thereof.

2. Addition to cosmetic

A method for producing the cosmetic of the present invention includes a step of adding the aforementioned cosmetic compounding agent of the present invention.

Here, the production method corresponds to producing the cosmetic by adding, as one ingredient, the cosmetic compounding agent of the present invention that has been previously prepared.

As another example, the method for producing the cosmetic of the present invention includes a step of adding a base forming an ammonium cation and an acid forming an anion to obtain an organic ammonium salt composed of the ammonium cation represented by the following formula (I) and an anion.

[Chemical formula 1]

$$N^{+}[R^{1}]_{n}[R^{2}]_{4-n} \qquad (I)$$

(In this formula, each $R^1$ independently represents a hydroxyalkyl group in which there is at least one hydroxy group, an alkyl moiety is a linear or branched moiety having 1 to 10 carbon atoms, and such alkyl moiety may contain an oxygen atom(s); a carboxyalkyl group in which there is at least one carboxy group, an alkyl moiety is a linear or branched moiety having 1 to 10 carbon atoms, and such alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and such alkyl moiety may contain an oxygen atom(s). Each $R^2$ independently represents a hydrogen atom or a linear or branched alkyl group having 1 to 18 carbon atoms, and at least one $R^2$ represents a hydrogen atom. n represents an integer of 0 to 4.)

The production method corresponds to (1) producing a cosmetic (hair treatment composition, skin care composition) by previously preparing an organic ammonium salt-containing cosmetic compounding agent (hair treatment agent, skin care agent) by mixing the abovementioned base with the abovementioned acid and even with a solvent as well as other ingredients, if necessary, and then mixing such cosmetic compounding agent with a solvent and other ingredients, if necessary; or (2) producing a cosmetic compounding agent (hair treatment agent, skin care agent) or a cosmetic (hair treatment composition, skin care composition) in a manner where the organic ammonium salt is not previously prepared, but formed in a system in which the abovementioned base and the abovementioned acid are present as individual ingredients and are separately mixed with a solvent as well as other ingredients, if necessary.

This production method includes, as described in (1), producing a cosmetic using the cosmetic compounding agent of the present invention obtained by separately adding a base forming a cation in which at least one $R^2$ represents a hydrogen atom (particularly, all $R^2$s represent hydrogen atoms) in the formula (I) and the abovementioned acid forming

anion.

In other words, the production method includes producing a cosmetic using the cosmetic compounding agent of the present invention obtained by separately adding a base forming the aforementioned organic ammonium salt in which at least one $R^2$ represents a hydrogen atom (particularly, all $R^2$s represent hydrogen atoms) in the formula (I) and the acid also forming the organic ammonium salt. In this case, it is preferred that n in the formula (I) represent an integer of 0 to 3, more preferably an integer of 1 to 3. $R^1$ may be a monohydroxyalkyl group or monocarboxyalkyl group whose alkyl moiety is a linear or branched moiety having 1 to 10 carbon atoms; at least one $R^1$ may be a polyhydroxyalkyl group in which there are at least two hydroxy groups, and an alkyl moiety is a linear or branched moiety having 1 to 10 carbon atoms.

Further, this production method includes, as described in (2), producing a cosmetic containing the organic ammonium salt by separately adding the abovementioned base and acid as individual ingredients without previously preparing the organic ammonium salt.

Specific examples of the abovementioned base include a base obtained by removing one proton corresponding to the hydrogen atom as $R^2$ from the ammonium cation described in "1-1. Cation." Further, a hydroxide of the cation in the organic ammonium salt of the present invention is also included.

Although not particularly limited, as an amine compound as the abovementioned base, there may be listed $NH_3$ and organic amines substituted by organic groups, examples of which include $NR_3$ (at least one R represents an organic group, while the rest of them represent hydrogen atoms), imidazole, pyridine, pyrrolidine, piperidine, pyrroline, pyrazine, triazine, isoquinoline, oxazoline, thiazoline, morpholine, guanidine, pyrimidine, piperazine, triazine, quinoline, indoline, quinoxaline, isooxazoline and an amino acid. Particularly, the amine compounds exemplified here are indicated as a collective term(s) encompassing those containing a substituent group such as a hydrogen-bonding functional group, in addition to the abovelisted amines having basic structures.

Specific examples of the abovementioned acid include an acid obtained by adding a proton(s) to the anion(s) described in "1-2. Anion"

Although not particularly limited, as the acid used in the present invention, there may be used compounds composed of various anions and protons; examples of such anions include a sulfur-based anion, a phosphorus-based anion, a nitrogen oxide-based anion and a carboxylic acid anion. Specific examples and preferable modes thereof are described as above.

## 3. Cosmetic

The cosmetic of the present invention contains the abovementioned cosmetic compounding agent of the present invention.

In the present invention, a cosmetic is defined as a material to be rubbed or dispersed on the body, or be used by a method(s) similar to these methods, for the purpose of cleaning and beautifying the human body, improving one’s charm, modifying looks or keeping the skin or hair healthy while mildly affecting the human body. Among such cosmetics, a cosmetic for use in the hair, skin or the like is preferred; particularly preferred are a hair treatment composition and a skin care composition.

### (Hair treatment composition)

The hair treatment composition of the present invention is mainly directed to a product that is used in the hair and contains the aforementioned hair treatment agent of the present invention. Although not particularly limited, examples of the hair include hairs of human body, such as scalp hairs, mustaches/beards, eyebrows, eyelashes, nasal hairs, ear hairs, underarm hairs and body hairs.

Although not particularly limited, examples of the hair treatment composition of the present invention include a hair shampoo, a scalp shampoo, a conditioner-integrated shampoo, a conditioning shampoo, a color shampoo, a hair soap, an anti-fading shampoo, a dry shampoo (non-rinse shampoo), a hair conditioner, a coloring conditioner, a hair cleansing product, a treatment product, a color treatment product, a non-rinse treatment product, an out bath treatment product, a conditioner, an out bath product, a styling agent, a set lotion agent, a hair manicure, a hair oil, a hair spray, a hair mist, a mousse, a foam, a hair gel, a hair cream, a hair wax, a hair liquid, a hair tonic, a hair growing agent, a hair restorer, a hair dyeing agent, a treatment product for scalp, a mascara, an eyelash cosmetic, a product for eyebrow and a product for an eyebrow pencil.

As for the abovelisted usages, in addition to the hair treatment agent of the present invention and a solvent(s), other ingredients may also be added to the hair treatment composition of the present invention provided that the effects of the present invention will not be impaired, in view of conventional combinations and common technical knowledge. There are no particular restrictions on the ingredients that are added as other ingredients, and those ingredients may be appropriately selected according to an intended purpose; examples of such ingredients include an anionic surfactant,

a nonionic surfactant, a cationic surfactant, an amphoteric surfactant, a cationic polymer, a water-soluble polymer, a viscosity regulator, a gloss imparting agent, a higher alcohol, a multivalent alcohol, a higher fatty acid, amidoamines, a hydrocarbon, a wax, esters, a silicone derivative, a physiologically active ingredient, extracts, an antioxidant, a sequestrant, a preservative, an ultraviolet absorber (organic and inorganic), a perfume, a moisturizer, carbons, metal oxides, minerals, salts, a neutralizer, a pH adjuster, a colorant, resin particles, a coloring agent, a natural plant extract ingredient, a seaweed extract ingredient, a crude drug ingredient, a refreshing agent an insect repellent and an enzyme.

Although not particularly limited, examples of the anionic surfactant include carboxylates such as a fatty acid soap, alkyl ether carboxylate, alkylene alkyl ether carboxylate, fatty acid amide ether carboxylate, acyl lactate, N-acylglutamate (e.g. triethanolamine cocoyl glutamate, sodium cocoyl glutamate), N-acylalaninate (e.g. sodium lauroylalaninate, sodium cocoylalaninate), N-acylmethyl-β-alaninate (e.g. sodium lauroylmethyl-β-alaninate), N-acylsarcosinate (e.g. sodium lauroylsarcosinate, triethanolamine lauroylsarcosinate), N-acylthreonine salt, N-acylglycine salt, N-acylaspartate, N-acylserine salt, N-acyl-ω-amino acid salt, alkyl sulfoacetate and alkenyl sulfoacetate; sulfonates such as alkane sulfonate, α-olefin sulfonate (e.g. sodium tetradecene sulfonate), α-sulfo fatty acid methyl ester salt, acyl isethionate, alkyl glycidyl ether sulfonate, alkyl sulfosuccinate, polyoxyalkylene alkyl sulfosuccinate (e.g. laureth sulfosuccinate 2Na, pareth sulfosuccinate 2Na), alkylbenzene sulfonate, alkylnaphthalene sulfonate, N-acyl taurate, N-acylmethyl taurate (e.g. sodium cocoyl methyl taurate), formalin condensation-based sulfonate, paraffin sulfonate, alkylamide sulfonate, alkenylamide sulfonate and alkylglyceryl ether sulfonate; sulfates such as alkyl sulfate, alkenyl sulfate, alkyl ether sulfate, alkenyl ether sulfate, polyoxyalkylene alkyl ether sulfate (e.g. sodium polyoxyethylene lauryl ether sulfate), alkylaryl ether sulfate, fatty acid alkanolamide sulfate, fatty acid monoglyceride sulfate, polyoxyalkylene aliphatic amide ether sulfate and alkylglyceryl ether sulfate; and phosphates such as polyoxyalkylene alkyl ether phosphate (e.g. sodium polyoxyethylene lauryl ether phosphate, potassium polyoxyethylene lauryl ether phosphate), alkyl phosphate, alkylaryl ether phosphate and fatty acid amide ether phosphate. Although not particularly limited, examples of the nonionic surfactant include polyoxyalkylene fatty acid ester; polyoxyalkylene alkyl ether; polyoxyalkylene alkyl phenyl ether; polyoxyalkylene (hardened) castor oil ester; sucrose fatty acid ester; polyglycerin alkyl ether; polyglycerin fatty acid ester; fatty acid alkanolamides such as alkyl alkanolamide, polyoxyethylene alkylalkanolamide and polyoxypropylene alkylalkanolamide; polyoxyalkylene glycerin fatty acid (mono/di/tri) ester; sorbitan fatty acid ester; polyoxyalkylene sorbitan fatty acid ester; and alkyl polyglycoside. Although not particularly limited, examples of the cationic surfactant include aliphatic amine salts and quaternary ammonium salts thereof, such as a primary amine salt, secondary amine salt, tertiary amine salt, aliphatic amide amine salt, aliphatic amide guanidium salt, quaternary ammonium salt (e.g. stearyltrimonium chloride, behentrimonium chloride), alkyl trialkylene glycol ammonium salt and alkyl ether ammonium salt; and cyclic quaternary ammonium salts such as benzalkonium salt, benzethonium salt, pyridinium salt and imidazolinium salt. Although not particularly limited, examples of the amphoteric surfactant include an alkylbetaine-type amphoteric surfactant, an amidebetaine-type amphoteric surfactant (e.g. coconut oil fatty acid amide propyl betaine, lauramidepropyl betaine, myristamidepropyl betaine, and palm kernel oil fatty acid amide propyl betaine), a sulfobetaine-type amphoteric surfactant (e.g. lauryl hydroxysulfobetaine), a phosphobetaine-type amphoteric surfactant, an imidazolinium betaine-type amphoteric surfactant (e.g. 2-alkyl-N-carboxymethyl-N-hydroxyethyl imidazolinium betaine), an alkylamine oxide-type amphoteric surfactant, an amino acid-type amphoteric surfactant and a phosphate ester-type amphoteric surfactant. Although not particularly limited, examples of the cationic polymer include a cationized starch, cationized cellulose, cationized hydroxyethyl cellulose, cationized guar gum, cationized locust bean gum, cationized tamarind gum, cationized tara gum, cationized fenugreek gum, (N,N-dimethyl-3,5-methylenepiperidium chloride)-acrylamide copolymer, poly(N,N-dimethyl-3,5-methylenepiperidium chloride), polyethyleneimine, quaternized vinylpyrrolidone-aminoethyl methacrylate copolymer, adipate-dimethylamino hydroxypropylene diethylenetriamine copolymer and acrylamide-β-methacryloxyethyltrimethylammonium copolymer. Although not particularly limited, examples of the water-soluble polymer include a hydroxyethyl cellulose, hydroxypropyl cellulose, hydroxypropyl methyl cellulose, dimethyldiallyl ammonium chloride-acrylamide copolymer, polyethylene glycol, highly polymerized polyethylene glycol, polyvinyl alcohol, polyglutamic acid, carboxyvinyl polymer, acrylate-alkyl methacrylate copolymer and alkyl acrylate copolymer. Although not particularly limited, examples of the viscosity regulator include alkyl alkanolamides such as coconut oil fatty acid monoethanolamide, coconut oil fatty acid diethanolamide, lauric acid diethanolamide and coconut oil fatty acid N-methylethanolamide; polyoxyethylene alkyl alkanolamides such as polyoxyethylene coconut oil fatty acid monoethanolamide; polyoxypropylene alkyl alkanolamides such as polyoxypropylene coconut oil fatty acid monoisopropanolamide; the abovementioned water-soluble polymers; polysaccharides; and the abovementioned cationic polymers. Although not particularly limited, examples of the gloss imparting agent include fatty acid ethylene glycol ester (e.g. ethylene glycol distearate), fatty acid polyethylene glycol ester and fatty acid monoethanolamide. Although not particularly limited, examples of the higher alcohol include cetyl alcohol, stearyl alcohol and behenyl alcohol. Although not particularly limited, examples of the multivalent alcohol include glycerin, 1,3-butanediol, propylene glycol, dipropylene glycol, propanediol, sorbitol, diglycerin, triglycerin and polyglycerin. Although not particularly limited, examples of the higher fatty acid include myristic acid, palmitic acid, stearic acid and behenic acid. Although not particularly limited, examples of the amidoamines include dimethylaminopropyl amide stearate. Although not particularly limited, examples of the hydrocarbon include a liquid paraffin, polyisobutene and

squalane. Although not particularly limited, examples of the wax include a candelilla wax, carnauba wax, paraffin wax, microcrystalline wax and polyethylene wax. Although not particularly limited, examples of the esters include animal and plant oils, isopropyl myristate, ethylhexyl palmitate, cetyl octanoate, cetyl ethylhexanoate and isononyl isononanoate. Although not particularly limited, examples of the silicone derivative include dimethylpolysiloxane, methylphenylpolysiloxane, fatty acid-modified silicone, alcohol-modified silicone, amino-modified silicone and dimethiconol. Although not particularly limited, examples of the physiologically active ingredient include a natural plant extract ingredient, a seaweed extract ingredient and a crude drug ingredient that are capable of imparting any sort of physiological activity to the skin when applied thereto. Although not particularly limited, examples of the extracts include various extracts derived from plants, animals and microorganisms. Although not particularly limited, examples of the antioxidant include tocopherols, dibutylhydroxytoluene, butylhydroxyanisole, gallic acid and gallic acid esters. Although not particularly limited, examples of the sequestrant include 1-hydroxyethane-1,1-diphosphonic acid; 1-hydroxyethane-1,1-diphosphonic acid tetrasodium salt; ethylenediamine tetraacetic acid and salts thereof (e.g. dipotassium dihydrate, disodium salt, disodium calcium salt, trisodium salt, tetrasodium salt, tetrasodium dihydrate, and tetrasodium tetrahydrate); hydroxyethylethylenediamine triacetic acid and its salt; phosphoric acid and its salt; ascorbic acid and its salt; succinic acid and its salt; gluconic acid and its salt; polyphosphoric acid and its salt; metaphosphoric acid and its salt; tartaric acid and its salt; phytic acid and its salt; citric acid and its salt; maleic acid and its salt; polyacrylic acid and its salt; isoamylene-maleic acid copolymer and its salt; silicic acid and its salt; hydroxybenzyliminodiacetic acid and its salt; iminodiacetic acid and its salt; diethylenetriaminepentaacetic acid and its salt; nitrilotriacetic acid and its salt; methylglycine diacetic acid and its salt; L-glutamic acid diacetic acid and its salt; and L-aspartate diacetic acid and its salt. Although not particularly limited, examples of the preservative include paraoxybenzoic acid ester (methylparaben, ethylparaben, and butylparaben); 1,2-alkanediol (carbon chain length 6 to 14) and its derivative; phenoxyethanol; isopropylmethylphenol; sodium benzoate; and hinokitiols. Although not particularly limited, examples of the ultraviolet absorber include a benzotriazole-based absorber, a PABA-based absorber, an anthranilic acid-based absorber, a cinnamic acid-based absorber, a salicylic acid-based absorber, a benzophenone-based absorber and a triazine-based absorber. Although not particularly limited, examples of the perfume include perfumes containing perfume ingredients as natural substances such as an extract, an essential oil, a resinoid, a resin, an attar and combinations thereof. Although not particularly limited, examples of the moisturizer include a mucopolysaccharide, hyaluronic acid, chondroitin sulfate, glutamic acid and chitosan. Although not particularly limited, examples of the carbons include carbon black, graphite, carbon fibers, activated carbon, bamboo charcoal, charcoal, single-walled carbon nanotubes, double-walled carbon nanotubes, multi-walled carbon nanotubes, carbon nanohorns, fullerene and carbon balloons. Although not particularly limited, examples of the metal oxides include silica, aluminum oxide (alumina), zirconium oxide, titanium oxide, magnesium oxide, indium tin oxide (ITO), cobalt blue ($CoO-Al_2O_3$), antimony oxide, zinc oxide, cesium oxide, zirconium oxide, yttrium oxide, tungsten oxide, vanadium oxide, cadmium oxide, tantalum oxide, niobium oxide, tin oxide, bismuth oxide, cerium oxide, copper oxide, iron oxide, indium oxide, boron oxide, calcium oxide, barium oxide, thorium oxide, indium tin oxide, magnesium silicate and ferrite. Although not particularly limited, examples of the salts include salts of organic acids such as citric acid, malic acid, succinic acid and lactic acid; and salts of inorganic acids such as hydrochloric acid, sulfuric acid, nitric acid, carbonic acid and phosphoric acid. Although not particularly limited, examples of the neutralizer and the pH adjuster include inorganic acids, organic acids, alkali metal salts and organic bases. Although not particularly limited, examples of the refreshing agent include L-menthol, L-menthyl lactate, menthyl glyceryl ether, menthanediol, camphor and a mint oil. Although not particularly limited, examples of the insect repellent include diethyltoluamide. Although not particularly limited, examples of the enzyme include proteases, cellulases, amylases, lipases and mannanases.

Although there are no particular restrictions on the amount of the hair treatment agent of the present invention contained in the hair treatment composition of the present invention, from the perspective of the effects of the present invention, it is preferred that the hair treatment agent be contained in an amount of not smaller than 0.01% by mass, more preferably not smaller than 0.1% by mass, even more preferably not smaller than 1.0% by mass, particularly preferably not smaller than 10.0% by mass, as a content of the organic ammonium salt expressed in terms of anhydride. As an example of the hair treatment composition of the present invention, for example, when the hair treatment composition of the invention is a shampoo, although not particularly limited, it is preferred that the hair treatment agent of the present invention be contained therein by an amount of not smaller than 0.1% by mass, more preferably not smaller than 0.5% by mass, even more preferably not smaller than 3.0% by mass. For example, when the hair treatment composition is a conditioner, although not particularly limited, it is preferred that the hair treatment agent of the present invention be contained therein by an amount of not smaller than 0.01% by mass, more preferably not smaller than 0.1% by mass, even more preferably not smaller than 0.5% by mass, particularly preferably not smaller than 3.0% by mass.

The hair treatment composition of the present invention can be prepared in a manner such that the content of the hair treatment agent of the present invention shall, for example, fall into the above ranges by performing dilution with a solvent. Although not particularly limited, examples of the solvent include water; alcohols such as methanol, ethanol, 1-propanol, 2-propanol, 1-butanol and benzyl alcohol; alkyl ethers such as diethylene glycol monomethyl ether and diethylene glycol dimethyl ether; and multivalent alcohols such as ethylene glycol, diethylene glycol, isoprene glycol,

hexylene glycol, glycerin, diglycerin, 1,3-butanediol, propylene glycol, dipropylene glycol, propanediol, sorbitol and maltitol. Any one of these solvents may be used alone, or two or more of them may be used in combination. Among them, water or an aqueous solution is preferred; particularly preferred is a water such as a purified water.

The hair treatment composition of the present invention can, for example, be prepared by adding and mixing the hair treatment agent of the present invention and the aforementioned other ingredients into a solvent. If necessary, solid raw materials will be heated and melted before being mixed, stirred and eventually uniformly blended.

As for the hair treatment composition of the present invention, not only that the organic ammonium salt is used, but it will also suffice if the composition contains a cation and an anion composing the organic ammonium salt. The hair treatment composition may be obtained by previously producing the organic ammonium salt used in the present invention, and then mixing it with water, a solvent and/or other ingredients; or by mixing, in any order, compounds (e.g. acid and base) as raw materials of a cation and an anion with water, a solvent and/or other ingredients.

When used on the hair, the hair treatment composition of the present invention is capable of imparting a cohesiveness, moist feeling, gloss and smoothness and thus allowing there to be achieved a favorable feeling with no stickiness, due to the effects owing to the non-volatility of the organic ammonium salt of the present invention that is contained in the composition, such as the short- or long-term water and moisture retention property, antistatic property (static protection property), adherability to and stabilization of the proteins of the hair and heat stability. Further, there can be obtained a hair treatment composition with an excellent solubility of active ingredients.

(Skin care composition)

The skin care composition of the present invention is mainly directed to a product that is used in the skin or the like and contains the aforementioned skin care agent of the present invention. Although not particularly limited, examples of the skin or the like include dermis, skin, cuticle, nails, and mucous membranes in oral and nasal cavities.

Although not particularly limited, examples of the skin care composition include skin cleansing agents such as a soap composition, a body soap, a hand soap and a facial cleanser; cleansing cosmetics such as a cleansing oil, a cleansing liquid, a cleansing lotion, a cleansing cream, a cleansing milk, a cleansing balm, a cleansing gel and a cleansing emulsion; basic cosmetics such as a lotion, a moisturizer, an emulsion, a beauty essence, a hand cream, a body lotion and a body cream; makeup cosmetics such as a powder foundation, a liquid foundation, a gel foundation, a face powder, a mousse powder, a concealer, a blusher, an eye shadow, an eyeliner, an overcoat agent, a lipstick, a lip cream and a makeup base; sunscreen cosmetics such as a sunscreen emulsion and a sunscreen cream; bath agents such as a bath oil, a bath milk and a bath essence; makeup films such as films for concealing flecks, acne and scars; treatment products for scalp; nail cosmetics; cuticle care products; oral care products; and sheet masks. The effects of the present invention shall be expressed on the skin or the like with regard to these usages.

There are no particular restrictions on the form of the skin care composition of the present invention; the composition may be either uniform or ununiform, for example, the composition may be in the state of a liquid to a solid, or even be an emulsification composition. Although not particularly limited, the emulsification composition may, for example, be a water-in-oil (W/O type) emulsification composition, an oil-in-water (O/W type) emulsification composition, and a composite emulsion (W/O/W type, O/W/O type).

The emulsification composition containing the skin care agent of the present invention is equally superior in feeling of use, such as a spreadability thereof when applied to the skin, a non-stickiness, a moisture retention feeling, a skin compatibility and a skin elasticity; and is particularly superior in a persistent moisturizer feeling due to the non-volatility of the organic ammonium salt used in the present invention. Especially, an emulsification composition containing an organic ammonium salt having a melting point of lower than 25°C is superior in feeling of use, such as the spreadability, non-stickiness, moisture retention feeling and skin compatibility, and is particularly superior in non-stickiness.

As for the abovelisted usages, in addition to the skin care agent of the present invention and a solvent(s), other ingredients may also be added to the skin care composition of the present invention provided that the effects of the present invention will not be impaired, in view of conventional combinations and common technical knowledge. There are no particular restrictions on the ingredients that are added as other ingredients, and those ingredients may be appropriately selected according to an intended purpose; examples of such ingredients include an anionic surfactant, a nonionic surfactant, a cationic surfactant, an amphoteric surfactant, an oil agent, a cationic polymer, a water-soluble polymer, a viscosity regulator, resin particles, a gloss imparting agent, a higher alcohol, a multivalent alcohol, a higher fatty acid, amidoamines, a hydrocarbon, a wax, esters, a silicone derivative, a physiologically active ingredient, extracts, an antioxidant, a preservative, an ultraviolet absorber (organic and inorganic), a perfume, a moisturizer, carbons, metal oxides, minerals, salts, a neutralizer, a pH adjuster, a refreshing agent, an insect repellent, an enzyme, a dye, an organic colorant, an inorganic colorant, a coloring agent, a pearling agent, a pearlizing agent, an anti-inflammatory agent, an antioxidant, a whitening agent, a wrinkle ameliorating agent, vitamins, amino acids, a hair growing agent, an antibacterial agent, a hormonal agent, a plant extract, a seaweed extract ingredient, a crude drug ingredient, an activator, a blood circulation promoter, a sequestrant and organic modified clay minerals.

Although not particularly limited, examples of the anionic surfactant include carboxylates such as a fatty acid soap, alkyl ether carboxylate, alkylene alkyl ether carboxylate, fatty acid amide ether carboxylate, acyl lactate, N-acylglutamate (e.g. triethanolamine cocoyl glutamate, sodium cocoyl glutamate), N-acylalaninate (e.g. sodium lauroylalaninate, sodium cocoylalaninate), N-acylmethyl-β-alaninate (e.g. sodium lauroylmethyl-β-alaninate), N-acylsarcosinate (e.g. sodium lauroylsarcosinate, triethanolamine lauroylsarcosinate), N-acylthreonine salt, N-acylglycine salt, N-acylaspartate, N-acylserine salt, N-acyl-ω-amino acid salt, alkyl sulfoacetate and alkenyl sulfoacetate; sulfonates such as alkane sulfonate, α-olefin sulfonate (e.g. sodium tetradecene sulfonate), α-sulfo fatty acid methyl ester salt, acyl isethionate, alkyl glycidyl ether sulfonate, alkyl sulfosuccinate, polyoxyalkylene alkyl sulfosuccinate (e.g. laureth sulfosuccinate 2Na, pareth sulfosuccinate 2Na), alkylbenzene sulfonate, alkylnaphthalene sulfonate, N-acyl taurate, N-acylmethyl taurate (e.g. sodium cocoyl methyl tauratee), formalin condensation-based sulfonate, paraffin sulfonate, alkylamide sulfonate, alkenylamide sulfonate and alkylglyceryl ether sulfonate; sulfates such as alkyl sulfate, alkenyl sulfate, alkyl ether sulfate, alkenyl ether sulfate, polyoxyalkylene alkyl ether sulfate (e.g. sodium polyoxyethylene lauryl ether sulfate), alkylaryl ether sulfate, fatty acid alkanolamide sulfate, fatty acid monoglyceride sulfate, polyoxyalkylene aliphatic amide ether sulfate and alkylglyceryl ether sulfate; and phosphates such as polyoxyalkylene alkyl ether phosphate (e.g. sodium polyoxyethylene lauryl ether phosphate, potassium polyoxyethylene lauryl ether phosphate), alkyl phosphate, alkylaryl ether phosphate and fatty acid amide ether phosphate. Although not particularly limited, examples of the nonionic surfactant include polyoxyalkylene fatty acid ester; polyoxyalkylene alkyl ether; polyoxyalkylene alkyl phenyl ether; polyoxyalkylene (hardened) castor oil ester; sucrose fatty acid ester; polyglycerin alkyl ether; polyglycerin fatty acid ester; fatty acid alkanolamides such as alkyl alkanolamide, polyoxyethylene alkylalkanolamide and polyoxypropylene alkylalkanolamide; polyoxyalkylene glycerin fatty acid (mono/di/tri) ester; sorbitan fatty acid ester; polyoxyalkylene sorbitan fatty acid ester; and alkyl polyglycoside. Although not particularly limited, examples of the cationic surfactant include aliphatic amine salts and quaternary ammonium salts thereof, such as a primary amine salt, secondary amine salt, tertiary amine salt, aliphatic amide amine salt, aliphatic amide guanidium salt, quaternary ammonium salt (e.g. stearyltrimonium chloride, behentrimonium chloride), alkyl trialkylene glycol ammonium salt and alkyl ether ammonium salt; and cyclic quaternary ammonium salts such as benzalkonium salt, benzethonium salt, pyridinium salt and imidazolinium salt. Although not particularly limited, examples of the amphoteric surfactant include an alkylbetaine-type amphoteric surfactant, an amidebetaine-type amphoteric surfactant (e.g. coconut oil fatty acid amide propyl betaine, lauramide propyl betaine, myristamide propyl betaine, and palm kernel oil fatty acid amide propyl betaine), a sulfobetaine-type amphoteric surfactant (e.g. lauryl hydroxysulfobetaine), a phosphobetaine-type amphoteric surfactant, an imidazolinium betaine-type amphoteric surfactant (e.g. 2-alkyl-N-carboxymethyl-N-hydroxyethyl imidazolinium betaine), an alkylamine oxide-type amphoteric surfactant, an amino acid-type amphoteric surfactant and a phosphate ester-type amphoteric surfactant. Although not particularly limited, examples of the oil agent include hydrocarbons, lipids, esters, fatty acids, higher alcohols, silicone oils, waxes, steroids, monomers, oligomers, fluid polymers (polymer compounds), a silicone oil, alcohols, glycols, glycol ethers and cellosolves; these oil agents may be liquids, gels or solids at normal temperature. Any one of them may be used alone, or two or more of them may be used in combination. Although not particularly limited, examples of the hydrocarbon include mineral oils such as a liquid paraffin, paraffin, solid paraffin, light isoparaffin, light liquid isoparaffin, liquid isoparaffin, ceresin, microcrystalline wax, vaseline, white vaseline and mineral oil; and synthetic oils such as squalane, alkylbenzene, polyethylene wax, polypropylene wax, hydrogenated polyisobutene, ethylene-α-olefin-cooligomer and ethylene propylene polymer. As other synthetic oils, although not particularly limited, examples of an aromatic oil include alkylbenzenes such as monoalkylbenzene and dialkylbenzene; or monoalkylnaphthalene, dialkylnaphthalene and polyalkylnaphthalene. Although not particularly limited, examples of an aliphatic oil include a normal paraffin, isoparaffin, polybutene, polyisobutylene, polyalphaolefin (e.g. 1-octene oligomer, 1-decene oligomer, and ethylene-propylene oligomer) and its hydride, and a cooligomer of alphaolefin and ethylene. Although not particularly limited, examples of an ester-based oil include diester oils such as dibutyl sebacate, di-2-ethylhexyl sebacate, dioctyl adipate, diisodecyl adipate, ditridecyl adipate, ditridecyl glutarate and methyl-acetylsinolate; or aromatic ester oils such as trioctyl trimellitate, tridecyl trimellitate and tetraoctyl pyromellitate; or even polyol ester oils such as trimethylolpropane caprylate, trimethylolpropane pelargonate, pentaerythritol-2-ethylhexanoate and pentaerythritol pelargonate; or yet a complex ester oil as an oligoester of a multivalent alcohol and a mixed fatty acid of dibasic acid-monobasic acid. Although not particularly limited, examples of an ether-based oil include phenyl ether oils such as monoalkyltriphenyl ether, alkyldiphenyl ether, dialkyldiphenyl ether, pentaphenyl ether, tetraphenyl ether, monoalkyl tetraphenyl ether and dialkyl tetraphenyl ether. Although not particularly limited, examples of the lipids include an avocado oil, almond oil, flaxseed oil, olive oil, cacao oil, perilla oil, camellia oil, castor oil, sesame oil, wheat germ oil, rice germ oil, rice bran oil, sasanqua oil, safflower oil, soybean oil, evening primrose oil, camellia oil, corn oil, rapeseed oil, persic oil, palm kernel oil, coconut oil, palm oil, beef fat, pork fat, horse fat, sheep fat, shea fat, cacao fat, turtle oil, mink oil, egg yolk oil, purcellin oil, castor oil, jojoba oil, grape seed oil, macadamia nut oil, cotton seed oil, meadowfoam oil, coconut oil, peanut oil, cod liver oil, rose hip oil, hardened oil of beef fat, extremely hardened oil of beef fat, hardened castor oil and extremely hardened palm oil. Although not particularly limited, examples of the esters include stearic acid alkyl ester, palmitic acid alkyl ester, myristic acid alkyl ester, lauric acid alkyl ester, behenic acid alkyl ester, oleic acid alkyl ester, isostearic acid alkyl ester, 12-hydroxystearic

acid alkyl ester, undecylenic acid alkyl ester, lanolin fatty acid alkyl ester, erucic acid alkyl ester, coconut oil fatty acid alkyl ester, stearoyloxystearic acid alkyl ester, isononanoic acid alkyl ester, dimethyloctanoic acid alkyl ester, octanoic acid alkyl ester, lactic acid alkyl ester, ethylhexanoic acid alkyl ester, neopentanoic acid alkyl ester, malic acid alkyl ester, phthalic acid alkyl ester, citric acid alkyl ester, malonic acid alkyl ester, adipic acid alkyl ester, ethylene glycol fatty acid ester, propanediol fatty acid ester, butanediol fatty acid ester, trimethylolpropane fatty acid ester, pentaerythritol fatty acid ester, polyglycerin fatty acid ester, trehalose fatty acid ester, pentylene glycol fatty acid ester and trimellitic acid tris(2-ethylhexyl). Although not particularly limited, examples of the fatty acids include stearic acid, palmitic acid, myristic acid, lauric acid, behenic acid, oleic acid, isostearic acid, 12-hydroxystearic acid, undecylenic acid, lanolin fatty acid, erucic acid and stearoyloxystearic acid. Although not particularly limited, examples of the higher alcohols include lauryl alcohol, cetyl alcohol, cetostearyl alcohol, stearyl alcohol, oleyl alcohol, behenyl alcohol, lanolin alcohol, hexyldecanol, myristyl alcohol, aralkyl alcohol, phytosterol, isostearyl alcohol and octyldodecanol. Although not particularly limited, examples of the silicone oils include an amino-modified silicone oil, epoxy-modified silicone oil, carboxyl-modified silicone oil, polyether-modified oil, polyglycerin-modified silicone oil, dimethylpolysiloxane, dimethylsilicone, polyether-modified silicone, methylphenylsilicone, alkyl-modified silicone, higher fatty acid-modified silicone, methylhydrogen silicone, fluorine-modified silicone, epoxy-modified silicone, carboxy-modified silicone, carbinol-modified silicone, amino-modified silicone, methylpolysiloxane, methylphenylpolysiloxane, silicone resin, dimethicone, methylhydrogenpolysiloxane, methylcyclopolysiloxane, octamethyltrisiloxane, tetramethylhexasiloxane and highly polymerized methylpolysiloxane. Although not particularly limited, examples of the waxes include Japan wax, beeswax, sumac wax, lacquer wax, sugarcane wax, palm wax, montan wax, carnauba wax, candelilla wax, rice bran wax, lanolin, spermaceti, reduced lanolin, liquid lanolin, hard lanolin, ceresin and ozokerite. Although not particularly limited, examples of the steroids include cholesterol, dihydrocholesterol and cholesterol fatty acid ester. Although not particularly limited, examples of the cationic polymer include a cationized starch, cationized cellulose, cationized hydroxyethyl cellulose, cationized guar gum, cationized locust bean gum, cationized tamarind gum, cationized tara gum, cationized fenugreek gum, (N,N-dimethyl-3,5-methylenepiperidium chloride)-acrylamide copolymer, poly(N,N-dimethyl-3,5-methylenepiperidium chloride), polyethyleneimine, quaternized vinylpyrrolidone-aminoethyl methacrylate copolymer, adipate-dimethylamino hydroxypropylene diethylenetriamine copolymer and acrylamide-$\beta$-methacryloxyethyltrimethylammonium copolymer. Although not particularly limited, examples of the water-soluble polymer include a hydroxyethyl cellulose, hydroxypropyl cellulose, hydroxypropyl methyl cellulose, dimethyldiallyl ammonium chloride-acrylamide copolymer, polyethylene glycol, highly polymerized polyethylene glycol, polyvinyl alcohol, polyglutamic acid, carboxyvinyl polymer, acrylate-alkyl methacrylate copolymer and alkyl acrylate copolymer. Although not particularly limited, examples of the viscosity regulator include alkyl alkanolamides such as coconut oil fatty acid monoethanolamide, coconut oil fatty acid diethanolamide, lauric acid diethanolamide and coconut oil fatty acid N-methylethanolamide; polyoxyethylene alkyl alkanolamides such as polyoxyethylene coconut oil fatty acid monoethanolamide; polyoxypropylene alkyl alkanolamides such as polyoxypropylene coconut oil fatty acid monoisopropanolamide; the abovementioned water-soluble polymers; polysaccharides; and the abovementioned cationic polymers. Although not particularly limited, examples of the gloss imparting agent include fatty acid ethylene glycol ester (e.g. ethylene glycol distearate), fatty acid polyethylene glycol ester and fatty acid monoethanolamide. Although not particularly limited, examples of the higher alcohol include cetyl alcohol, stearyl alcohol and behenyl alcohol. Although not particularly limited, examples of the multivalent alcohol include glycerin, 1,3-butanediol, propylene glycol, dipropylene glycol, propanediol, sorbitol, diglycerin, triglycerin and polyglycerin. Although not particularly limited, examples of the higher fatty acid include myristic acid, palmitic acid, stearic acid and behenic acid. Although not particularly limited, examples of the amidoamines include dimethylaminopropyl amide stearate. Although not particularly limited, examples of the hydrocarbon include a liquid paraffin, polyisobutene and squalane. Although not particularly limited, examples of the wax include a candelilla wax, carnauba wax, paraffin wax, microcrystalline wax and polyethylene wax. Although not particularly limited, examples of the esters include animal and plant oils, isopropyl myristate, ethylhexyl palmitate, cetyl octanoate, cetyl ethylhexanoate and isononyl isononanoate. Although not particularly limited, examples of the silicone derivative include dimethylpolysiloxane, methylphenylpolysiloxane, fatty acid-modified silicone, alcohol-modified silicone, amino-modified silicone and dimethiconol. Although not particularly limited, examples of the physiologically active ingredient include a natural plant extract ingredient, a seaweed extract ingredient and a crude drug ingredient that are capable of imparting any sort of physiological activity to the skin when applied thereto. Although not particularly limited, examples of the extracts include various extracts derived from plants, animals and microorganisms. Although not particularly limited, examples of the antioxidant include tocopherols, dibutylhydroxytoluene, butylhydroxyanisole, gallic acid and gallic acid esters. Although not particularly limited, examples of the sequestrant include 1-hydroxyethane-1,1-diphosphonic acid; 1-hydroxyethane-1,1-diphosphonic acid tetrasodium salt; ethylenediamine tetraacetic acid and salts thereof (e.g. dipotassium dihydrate, disodium salt, disodium calcium salt, trisodium salt, tetrasodium salt, tetrasodium dihydrate, and tetrasodium tetrahydrate); hydroxyethylethylenediamine triacetic acid and its salt; phosphoric acid and its salt; ascorbic acid and its salt; succinic acid and its salt; gluconic acid and its salt; polyphosphoric acid and its salt; metaphosphoric acid and its salt; tartaric acid and its salt; phytic acid and its salt; citric acid and its salt; maleic acid and its salt; polyacrylic acid and its salt; isoamylene-maleic acid copolymer and its salt; silicic acid

and its salt; hydroxybenzyliminodiacetic acid and its salt; iminodiacetic acid and its salt; diethylenetriaminepentaacetic acid and its salt; nitrilotriacetic acid and its salt; methylglycine diacetic acid and its salt; L-glutamic acid diacetic acid and its salt; and L-aspartate diacetic acid and its salt. Although not particularly limited, examples of the preservative include paraoxybenzoic acid ester (methylparaben, ethylparaben, and butylparaben); 1,2-alkanediol (carbon chain length 6 to 14) and its derivative; phenoxyethanol; isopropylmethylphenol; sodium benzoate; and hinokitiols. Although not particularly limited, examples of the ultraviolet absorber include a benzotriazole-based absorber, a PABA-based absorber, an anthranilic acid-based absorber, a cinnamic acid-based absorber, a salicylic acid-based absorber, a benzophenone-based absorber and a triazine-based absorber. Although not particularly limited, examples of the perfume include perfumes containing perfume ingredients as natural substances such as an extract, an essential oil, a resinoid, a resin, an attar and combinations thereof. Although not particularly limited, examples of the moisturizer include a mucopolysaccharide, hyaluronic acid, chondroitin sulfate, glutamic acid and chitosan. Although not particularly limited, examples of the carbons include carbon black, graphite, carbon fibers, activated carbon, bamboo charcoal, charcoal, single-walled carbon nanotubes, double-walled carbon nanotubes, multi-walled carbon nanotubes, carbon nanohorns, fullerene and carbon balloons. Although not particularly limited, examples of the metal oxides include silica, aluminum oxide (alumina), zirconium oxide, titanium oxide, magnesium oxide, indium tin oxide (ITO), cobalt blue ($CoO-Al_2O_3$), antimony oxide, zinc oxide, cesium oxide, zirconium oxide, yttrium oxide, tungsten oxide, vanadium oxide, cadmium oxide, tantalum oxide, niobium oxide, tin oxide, bismuth oxide, cerium oxide, copper oxide, iron oxide, indium oxide, boron oxide, calcium oxide, barium oxide, thorium oxide, indium tin oxide, magnesium silicate and ferrite. Although not particularly limited, examples of the salts include salts of organic acids such as citric acid, malic acid, succinic acid and lactic acid; and salts of inorganic acids such as hydrochloric acid, sulfuric acid, nitric acid, carbonic acid and phosphoric acid. Although not particularly limited, examples of the neutralizer and the pH adjuster include inorganic acids, organic acids, alkali metal salts and organic bases. Although not particularly limited, examples of the refreshing agent include L-menthol, L-menthyl lactate, menthyl glyceryl ether, menthanediol, camphor and a mint oil. Although not particularly limited, examples of the insect repellent include diethyltoluamide. Although not particularly limited, examples of the enzyme include proteases, cellulases, amylases, lipases and mannanases. Although not particularly limited, examples of the dye include Sudan red, D&C red No17, D&C green No6, β-carotene, soybean oil, Sudan brown, D&C yellow No11, D&C violet No2, D&C orange No5, quinoline yellow, annatto and bromic acids. Although not particularly limited, examples of the organic colorant include carbon black, D&C type colorants; and lakes based on cochineal carmine or barium, and on strontium, calcium or aluminum. Although not particularly limited, examples of the inorganic colorant include silicic anhydride, magnesium silicate, talc, kaolin, bentonite, mica, mica titanium, bismuth oxychloride, zirconium oxide, magnesium oxide, zinc oxide, titanium oxide, calcium carbonate, magnesium carbonate, yellow iron oxide, colcothar, black iron oxide, ultramarine, chromium oxide, chromium hydroxide, carbon black and calamine. Although not particularly limited, examples of the pearling agent include white pearling agents such as a mica coated with titanium or bismuth oxychloride; and colored pearling agents such as a titanium mica coated with an iron oxide, particularly a titanium mica coated with ferric blue or chromium oxide, a titanium mica coated with the aforementioned type(s) of organic colorant and likewise, a pearling agent based on bismuth oxychloride. Although not particularly limited, examples of the pearlizing agent include ethylene glycol distearate ester and mica. Although not particularly limited, examples of the anti-inflammatory agent include tranexamic acid, thiotaurine and hypotaurine. Although not particularly limited, examples of the antioxidant include glycyrrhizin, glycyrrhizate (e.g. dipotassium glycyrrhizinate, ammonium glycyrrhizinate), allantoin, thiotaurine, glutathione, catechin, albumin, ferritin and metallothionein. Although not particularly limited, examples of the whitening agent include a L-ascorbic acid (vitamin C) and its derivative; pantothenic acid derivative; tranexamic acid and its derivative; resorcinol derivative; salicylic acid derivative of, for example, potassium 4-methoxysalicylate; other phenol derivatives; arbutin; placenta extracts; and plant extracts (e.g. chamomile extract, saxifrage extract, and adlay extract). Although not particularly limited, examples of the wrinkle ameliorating agent include retinol, retinal, retinoic acid, tretinoin, isotretinoin, tocopherol retinoate, retinol palmitate, retinol acetate and ursolic acid benzyl ester, ursolic acid phosphate ester, betulinic acid benzyl ester, benzylic acid phosphate ester, sodium chondroitin sulfate, 2-methacryloyloxyethyl phosphorylcholine, and various extracts such as swertia japonica extract, alpinia speciosa leaf extract, hydrolyzed codonopsis pilosula root extract and cornflower extract. Although not particularly limited, examples of the vitamins include vitamin A and its derivative; vitamin B6 and its derivative such as vitamin B6 hydrochloride; nicotinic acid and a nicotinic acid derivative of nicotinic-acid amide; vitamin E and its derivative; and β-carotene. Although not particularly limited, examples of the amino acids include hydroxyproline, 1-serine, trimethyl glycine and 1-arginine. Although not particularly limited, examples of the hair growing agent include pantothenyl ethyl ether, adenosine, β-glycyrrhetinic acid and minoxidil. Although not particularly limited, examples of the antibacterial agent include resorcin, sulfur and salicylic acid. Although not particularly limited, examples of the hormonal agent include oxytocin, corticotropin, vasopressin, secretin, gastrin, calcitonin, hinokitiol and ethinyl estradiol. Although not particularly limited, examples of the plant extract include extracts of phellodendron bark, coptis japonica, lithospermum root, paeonia lactiflora, swertia japonica, birch, sage, loquat, carrot, aloe, mallow, iris, grape, coix seed, loofa, lily, saffron, cnidium rhizome, ginger, hypericum erectum, ononis, garlic, capsicum annum, citrus unshiu peel, angelica acutiloba and seaweed. Although not particularly limited, examples of the activator include

a royal jelly, photosensitizer and cholesterol derivative. Although not particularly limited, examples of the blood circulation promoter include capsaicin, zingerone, cantharides tincture, ichthammol, tannic acid, α-borneol, tocopherol nicotinate, inositol hexanicotinate, cyclandelate, cinnarizine, tolazoline, acetylcholine, verapamil, cepharanthin and γ-oryzanol. Although not particularly limited, examples of the sequestrant include 1-hydroxyethane-1,1-diphosphonic acid, 1-hydroxyethane-1,1-diphosphonate tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid and ethylenediaminehydroxyethyl triacetate 3 sodium. Although not particularly limited, examples of the organic modified clay minerals include those obtained by modifying clay minerals such as smectites (e.g. hectorite, bentonite, and montmorillonite), kaolinite, illite, marine clay minerals (sea mud), desert rose clay minerals and pascalite, with, for example, a quaternized amine group and carboxyl group.

When the skin care composition of the present invention is an emulsion containing a surfactant and an oil agent, in terms of a spreadability thereof when applied to the skin, non-stickiness, moisture retention feeling, persistent moisture retention feeling, skin compatibility and skin elasticity, it is preferred, although not particularly limited, that the oil agent be hydrocarbons, more preferably a mineral oil, even more preferably liquid paraffin and ceresin.

From the perspective of the effects of the present invention, although not particularly limited, it is preferred that the skin care agent of the present invention be contained in the skin care composition of the present invention by an amount of not smaller than 0.001% by mass, more preferably not smaller than 0.01% by mass, even more preferably not smaller than 0.1% by mass, particularly preferably not smaller than 1.0% by mass, more particularly preferably not smaller than 10.0% by mass, as a content of the aforementioned organic ammonium salt expressed in terms of anhydride.

The skin care composition of the present invention can be prepared in a manner such that the content of the skin care agent of the present invention shall, for example, fall into the above ranges by performing dilution with a solvent. Although not particularly limited, examples of the solvent include water; alcohols such as ethanol, 1-propanol, 2-propanol, 1-butanol, benzyl alcohol, hexenol, nonenol and methyldecenol; alkyl ethers such as diethylene glycol monomethyl ether and diethylene glycol dimethyl ether; and multivalent alcohols such as glycerin, diglycerin, 1,3-butanediol, propylene glycol, dipropylene glycol, isoprene glycol, propanediol, sorbitol and maltitol. Any one of these solvents may be used alone, or two or more of them may be used in combination. Among them, water or an aqueous solution is preferred; particularly preferred is a water such as a purified water.

The skin care composition of the present invention can, for example, be prepared by adding and mixing the skin care agent of the present invention and the aforementioned other ingredients into a solvent. If necessary, solid raw materials will be heated and melted before being mixed, stirred and eventually uniformly blended.

As for the skin care composition of the present invention, not only that the organic ammonium salt is used, but it will also suffice if the composition contains a cation and an anion composing the organic ammonium salt. The skin care composition may be obtained by previously producing the organic ammonium salt used in the present invention, and then mixing it with water, a solvent and/or other ingredients; or by mixing, in any order, amine and acidic compounds as raw materials of a cation and an anion forming the organic ammonium salt with water, a solvent and/or other ingredients.

When applied to the skin or the like, the skin care composition of the present invention is capable of imparting a moisture retention feeling, and allowing there to be achieved a favorable feeling with no stickiness, due to the effects owing to the non-volatility of the organic ammonium salt of the present invention that is contained in the composition, such as the short- or long-term water and moisture retention property or the antistatic property (static protection property). Further, due to the safety of the organic ammonium salt, and an affinity, permeability, low-irritating property and whitening effect thereof to the skin or the like, even as a skin care composition, there can be obtained a skin care composition with a high safety, a favorable skin compatibility, a favorable skin elasticity and being low-irritating to the skin or the like. Furthermore, since the aforementioned ammonium salt is useful as a base material for a skin care composition even in that there can be achieved a high solubility of the active ingredients, and since the ammonium salt is superior in permeability into the skin or the like, the ammonium salt can bring an excellent water and moisture retention effect to the skin or the like, and can thus also be used as a carrier of the active ingredients. Other than skin application, the composition of the invention may also be used in cuticles, nails, and inner regions of oral and nasal cavities where the effects of the present invention are sought.

## WORKING EXAMPLES

The present invention is described in greater detail hereunder with reference to working examples. However, the invention shall not be limited to the following working examples.

[1] Evaluation of cosmetic compounding agent

(Compound)

Compounds A1 to A54

Compounds A1 to A54 shown in Tables 1 to 8 were synthesized and obtained by a method(s) described below.
Compounds A1 to A51 were synthesized by a method described in JP-A-2014-131974.
Compound A52: A reagent (glycerin) produced by FUJIFILM Wako Pure Chemical Corporation was used.
Compound A53: An ion-exchange water was used.
Compound A54: A reagent (tetrabutylammonium bromide) produced by FUJIFILM Wako Pure Chemical Corporation was used.

1. Condition of cosmetic compounding agent (appearance)

Organic ammonium salts with molar ratios of anions and cations that are shown in Tables 1A to 1C were synthesized, and the conditions thereof at 25°C were then observed.

[Table 1A]

| | Compound | 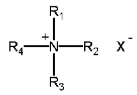 | | | | | Base | Acid | Compounding molar ratio (acid:base) | Condition at 25°C | n-hydrate |
|---|---|---|---|---|---|---|---|---|---|---|---|
| | | $R_1$ | $R_2$ | $R_3$ | $R_4$ | $X^-$ | | | | | |
| Working Example A1 | A1 | 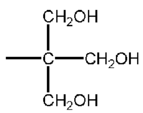 | H | H | H | $CH_3COO^-$ | 2-amino-2-hydroxymethyl-1,3-propanediol (tromethamine) | Acetic acid | 1:1 | Solid | Dihydrate |
| Working Example A2 | A2 | | | | | Isostearic acid anion | | Isostearic acid | 1:1 | Liquid | Dihydrate |
| Working Example A3 | A3 | | | | | Oleic acid anion | | Oleic acid | 1:1 | Liquid | Dihydrate |
| Working Example A4 | A4 | | | | | Linoleic acid anion | | Linoleic acid | 1:1 | Liquid | Dihydrate |
| Working Example A5 | A5 | | | | | $CH_3CH(OH)COO^-$ | | Lactic acid | 1:1 | Liquid | Dihydrate |
| Working Example A6 | A6 | | | | | $HOCH_2COO^-$ | | Glycolic acid | 1:1 | Solid | Dihydrate |
| Working Example A7 | A7 | | | | | $HOOC(CH_2)_2COO^-$ | | Succinic acid | 1:1 | Solid | Dihydrate |
| Working Example A8 | A8 | | | | | $^-OOC(CH_2)_2COO^-$ | | Succinic acid | 1:2 | Solid | Dihydrate |
| Working Example A9 | A9 | | | | | $HOOCCH_2CH(OH)COO^-$ | | Malic acid | 1:1 | Liquid | Dihydrate |
| Working Example A10 | A10 | | | | | $^-OOCCH_2CH(OH)COO^-$ | | Malic acid | 1:2 | Liquid | Dihydrate |
| Working Example A11 | A11 | | | | | $HOOCCH(OH)CH(OH)COO^-$ | | Tartaric acid | 1:1 | Solid | Dihydrate |
| Working Example A12 | A12 | | | | | $^-OOCCH(OH)CH(OH)COO^-$ | | Tartaric acid | 1:2 | Liquid | Dihydrate |
| Working Example A13 | A13 | | | | | $HOOCCHCHCOO^-$ | | Fumaric acid | 1:1 | Solid | Dihydrate |
| Working Example A14 | A14 | | | | | $^-OOCCHCHCOO^-$ | | Fumaric acid | 1:2 | Solid | Dihydrate |
| Working Example A15 | A15 | | | | | Benzoic acid anion | | Benzoic acid | 1:1 | Solid | Dihydrate |
| Working Example A16 | A16 | | | | | $CH_3COCH_2CH_2COO^-$ | | Levulinic acid | 1:1 | Liquid | Dihydrate |
| Working Example A17 | A17 | | | | | $Cl^-$ | | Hydrochloric acid | 1:1 | Solid | Dihydrate |
| Working Example A18 | A18 | | | | | $HOOCCH_2CH(COO^-)NH_2$ | | Aspartic acid | 1:1 | Liquid | Dihydrate |
| Working Example A19 | A19 | | | | | $^-OOCCH_2CH(COO^-)NH_2$ | | Aspartic acid | 1:2 | Solid | Dihydrate |
| Working Example A20 | A20 | | | | | $HOOC(CH_2)_2CH(COO^-)NHCOCH_3$ | | N-acetyl-L-glutamic acid | 1:1 | Solid | Dihydrate |
| Working Example A21 | A21 | | | | | $^-OOC(CH_2)_2CH(COO^-)NHCOCH_3$ | | N-acetyl-L-glutamic acid 2 | 1:2 | Solid | Dihydrate |
| Working Example A22 | A22 | | | | | $H_2N(CH_2)_3COO^-$ | | γ-aminobutyric acid | 1:1 | Solid | Monohydrate |
| Working Example A23 | A23 | | | | | $H_2N(CH_2)_5COO^-$ | | ε-aminocaproic acid | 1:1 | Solid | Monohydrate |
| Working Example A24 | A24 | | | | | Ascorbic acid anion | | Ascorbic acid | 1:1 | Liquid | Dihydrate |

[Table 1B]

| | Compound | 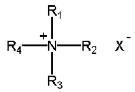 | | | | | Base | Acid | Compounding molar ratio (acid:base) | Condition at 25°C | n-hydrate |
|---|---|---|---|---|---|---|---|---|---|---|---|
| | | $R_1$ | $R_2$ | $R_3$ | $R_4$ | $X^-$ | | | | | |
| Working Example A25 | A25 | 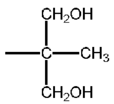 | H | H | H | $CH_3COO^-$ | 2-amino-2-methyl-1,3-propanediol | Acetic acid | 1:1 | Liquid | Dihydrate |
| Working Example A26 | A26 | | | | | Isostearic acid anion | | Isostearic acid | 1:1 | Liquid | Dihydrate |
| Working Example A27 | A27 | | | | | Oleic acid anion | | Oleic acid | 1:1 | Liquid | Dihydrate |
| Working Example A28 | A28 | | | | | Linoleic acid anion | | Linoleic acid | 1:1 | Liquid | Dihydrate |
| Working Example A29 | A29 | | | | | $CH_3CH(OH)COO^-$ | | lactic acid | 1:1 | Liquid | Dihydrate |
| Working Example A30 | A30 | | | | | $HOCH_2COO^-$ | | Glycolic acid | 1:1 | Solid | Dihydrate |
| Working Example A31 | A31 | | | | | $HOOC(CH_2)_2COO^-$ | | Succinic acid | 1:1 | Liquid | Dihydrate |
| Working Example A32 | A32 | | | | | $^-OOC(CH_2)_2COO^-$ | | Succinic acid | 1:2 | Solid | Dihydrate |
| Working Example A33 | A33 | | | | | $HOOCCH_2CH(OH)COO^-$ | | Malic acid | 1:1 | Liquid | Dihydrate |
| Working Example A34 | A34 | | | | | $^-OOCCH_2CH(OH)COO^-$ | | Malic acid | 1:2 | Liquid | Dihydrate |
| Working Example A35 | A35 | | | | | $HOOCCH(OH)CH(OH)COO^-$ | | Tartaric acid | 1:1 | Liquid | Dihydrate |
| Working Example A36 | A36 | | | | | $^-OOCCH(OH)CH(OH)COO^-$ | | Tartaric acid | 1:2 | Liquid | Dihydrate |
| Working Example A37 | A37 | | | | | $HOOCCHCHCOO^-$ | | Fumaric acid | 1:1 | Solid | Dihydrate |
| Working Example A38 | A38 | | | | | $^-OOCCHCHCOO^-$ | | Fumaric acid | 1:2 | Solid | Dihydrate |
| Working Example A39 | A39 | | | | | Benzoic acid anion | | Benzoic acid | 1:1 | Solid | Dihydrate |
| Working Example A40 | A40 | | | | | $CH_3COCH_2CH_2COO^-$ | | Levulinic acid | 1:1 | Liquid | Dihydrate |
| Working Example A41 | A41 | | | | | $Cl^-$ | | Hydrochloric acid | 1:1 | Solid | Dihydrate |
| Working Example A42 | A42 | | | | | $HOOCCH_2CH(COO^-)NH_2$ | | Aspartic acid | 1:1 | Solid | Dihydrate |
| Working Example A43 | A43 | | | | | $^-OOCCH_2CH(COO^-)NH_2$ | | Aspartic acid | 1:2 | Solid | Dihydrate |
| Working Example A44 | A44 | | | | | $HOOC(CH_2)_2CH(COO^-)NHCOCH_3$ | | N-acetyl-L-glutamic acid | 1:1 | Liquid | Dihydrate |
| Working Example A45 | A45 | | | | | $^-OOC(CH_2)_2CH(COO^-)NHCOCH_3$ | | N-acetyl-L-glutamic acid 2 | 1:2 | Solid | Dihydrate |
| Working Example A46 | A46 | | | | | $H_2N(CH_2)_3COO^-$ | | γ-aminobutyric acid | 1:1 | Solid | Monohydrate |
| Working Example A47 | A47 | | | | | $H_2N(CH_2)_5COO^-$ | | ε-aminocaproic acid | 1:1 | Solid | Monohydrate |
| Working Example A48 | A48 | | | | | Ascorbic acid anion | | Ascorbic acid | 1:1 | Solid | Dihydrate |

[Table 1C]

| | Compound | 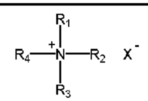 | | | | | Base | Acid | Compounding molar ratio (acid:base) | Condition at 25°C | n-hydrate |
|---|---|---|---|---|---|---|---|---|---|---|---|
| | | $R_1$ | $R_2$ | $R_3$ | $R_4$ | $X^-$ | | | | | |
| Working Example A49 | A49 | 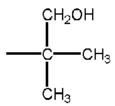 | H | H | H | CH3CH(OH)COO− | 2-amino-2-methyl-1-propanol | Lactic acid | 1 : 1 | Liquid | Dihydrate |
| Working Example A50 | A50 | $(CH_2)_3COO^-$ | H | H | H | $CH_3CH(OH)COO^-$ | γ-aminobutyric acid | Lactic acid | 1 : 1 | Liquid | Monohydrate |
| Working Example A51 | A51 | $(CH_2)_5COO^-$ | H | H | H | $CH_3CH(OH)COO^-$ | ε-aminocaproic acid | Lactic acid | 1 : 1 | Liquid | Monohydrate |

2. Evaluation on solubility of active ingredient

Evaluated were solubilities of active ingredients in a 20 wt% aqueous solution of each cosmetic compounding agent of working examples A52 to A92 and comparative examples A1 and A2 that are listed in Tables 2A to 2C. As the active ingredients, there were used a poorly-soluble gallic acid having an antioxidant effect, and a glutamic acid having a moisture retention effect. The solubility of the glutamic acid in the case of each of the comparative examples A1 (glycerin 20 wt% aqueous solution) and A2 (ion-exchange water) was lower than 1 wt%; the solubility of the glutamic acid in the cases of all the working examples was high. Further, similar tendencies were also observed with the gallic acid; a larger amount of the active ingredient was able to be dissolved. In this regard, the cosmetic compounding agent of the present invention can be used as, for example, a base agent, solvent or carrier of a cosmetic material.

[Table 2A]

| | Compound | 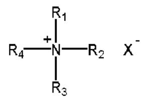 | | | | | Base | Acid | Compounding molar ratio (acid:base) | Compound concentration (wt%) | Solubility (g/100g) | |
|---|---|---|---|---|---|---|---|---|---|---|---|---|
| | | $R_1$ | $R_2$ | $R_3$ | $R_4$ | $X^-$ | | | | | Glutamic acid | Gallic acid |
| Working Example A52 | A1 | 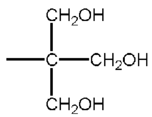 | H | H | H | $CH_3COO^-$ | 2-amino-2-hydroxymethyl-1,3-propanediol (tromethamine) | Acetic acid | 1:1 | 20 | 7 | 11 |
| Working Example A53 | A5 | | | | | $CH_3CH(OH)COO^-$ | | Lactic acid | 1:1 | | 3 | 4 |
| Working Example A54 | A6 | | | | | $HOCH_2COO^-$ | | Glycolic acid | 1:1 | | 3 | 7 |
| Working Example A55 | A7 | | | | | $HOOC(CH_2)_2COO^-$ | | Succinic acid | 1:1 | | 5 | 5 |
| Working Example A56 | A8 | | | | | $^-OOC(CH_2)_2COO^-$ | | Succinic acid | 1:2 | | 6 | 10 |
| Working Example A57 | A9 | | | | | $HOOCCH_2CH(OH)COO^-$ | | Malic acid | 1:1 | | 3 | 3 |
| Working Example A58 | A10 | | | | | $^-OOCCH_2CH(OH)COO^-$ | | Malic acid | 1:2 | | 5 | 5 |
| Working Example A59 | A11 | | | | | $HOOCCH(OH)CH(OH)COO^-$ | | Tartaric acid | 1:1 | | 2 | 2 |
| Working Example A60 | A12 | | | | | $^-OOCCH(OH)CH(OH)COO^-$ | | Tartaric acid | 1:2 | | 2 | 5 |
| Working Example A61 | A14 | | | | | $^-OOCCHCHCOO^-$ | | Fumaric acid | 1:2 | | 3 | 6 |
| Working Example A62 | A16 | | | | | $CH_3COCH_2CH_2COO^-$ | | Levulinic acid | 1:1 | | 6 | 11 |
| Working Example A63 | A17 | | | | | $Cl^-$ | | Hydrochloric acid | 1:1 | | 2 | 2 |
| Working Example A64 | A18 | | | | | $HOOCCH_2CH(COO^-)NH_2$ | | Aspartic acid | 1:1 | | 6 | 6 |
| Working Example A65 | A19 | | | | | $^-OOCCH_2CH(COO^-)NH_2$ | | Aspartic acid | 1:2 | | 11 | 12 |
| Working Example A66 | A20 | | | | | $HOOC(CH_2)_2CH(COO^-)NHCOCH_3$ | | N-acetyl-L-glutamic acid | 1:1 | | 3 | 3 |
| Working Example A67 | A21 | | | | | $^-OOC(CH_2)_2CH(COO^-)NHCOCH_3$ | | N-acetyl-L-glutamic acid 2 | 1:2 | | 6 | 8 |
| Working Example A68 | A22 | | | | | $H_2N(CH_2)_3COO^-$ | | γ-aminobutyric acid | 1:1 | | 15 | 20 |
| Working Example A69 | A23 | | | | | $H_2N(CH_2)_5COO^-$ | | ε-aminocaproic acid | 1:1 | | 15 | 25 |
| Working Example A70 | A24 | | | | | Ascorbic acid anion | | Ascorbic acid | 1:1 | | 4 | 8 |

[Table 2B]

| | Compound | 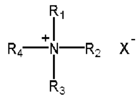 | | | | | Base | Acid | Compounding molar ratio (acid:base) | Compound concentration (wt%) | Solubility (g/100g) | |
|---|---|---|---|---|---|---|---|---|---|---|---|---|
| | | $R_1$ | $R_2$ | $R_3$ | $R_4$ | $X^-$ | | | | | Glutamic acid | Gallic acid |
| Working Example A71 | A25 | 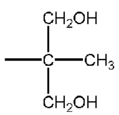 | H | H | H | $CH_3COO^-$ | 2-amino-2-methyl-1,3-propanediol) | Acetic acid | 1:1 | 20 | 8 | 10 |
| Working Example A72 | A29 | | | | | $CH_3CH(OH)COO^-$ | | Lactic acid | 1:1 | | 4 | 9 |
| Working Example A73 | A30 | | | | | $HOCH_2COO^-$ | | Glycolic acid | 1:1 | | 3 | 6 |
| Working Example A74 | A31 | | | | | $HOOC(CH_2)_2COO^-$ | | Succinic acid | 1:1 | | 3 | 10 |
| Working Example A75 | A32 | | | | | $^-OOC(CH_2)_2COO^-$ | | Succinic acid | 1:2 | | 7 | 13 |
| Working Example A76 | A33 | | | | | $HOOCCH_2CH(OH)COO^-$ | | Malic acid | 1:1 | | 3 | 6 |
| Working Example A77 | A34 | | | | | $^-OOCCH_2CH(OH)COO^-$ | | Malic acid | 1:2 | | 5 | 5 |
| Working Example A78 | A35 | | | | | $HOOCCH(OH)CH(OH)COO^-$ | | Tartaric acid | 1:1 | | 2 | 2 |
| Working Example A79 | A36 | | | | | $^-OOCCH(OH)CH(OH)COO^-$ | | Tartaric acid | 1:2 | | 3 | 4 |
| Working Example A80 | A38 | | | | | $^-OOCCHCHCOO^-$ | | Fumaric acid | 1:2 | | 3 | 9 |
| Working Example A81 | A40 | | | | | $CH_3COCH_2CH_2COO^-$ | | Levulinic acid | 1:1 | | 4 | 14 |
| Working Example A82 | A41 | | | | | $Cl^-$ | | Hydrochloric acid | 1:1 | | 2 | 2 |
| Working Example A83 | A42 | | | | | $HOOCCH_2CH(COO^-)NH_2$ | | Aspartic acid | 1:1 | | — | — |
| Working Example A84 | A43 | | | | | $^-OOCCH_2CH(COO^-)NH_2$ | | Aspartic acid | 1:2 | | 11 | 14 |
| Working Example A85 | A44 | | | | | $HOOC(CH_2)_2CH(COO^-)NHCOCH_3$ | | N-acetyl-L-glutamic acid | 1:1 | | 3 | 5 |
| Working Example A86 | A45 | | | | | $^-OOC(CH_2)_2CH(COO^-)NHCOCH_3$ | | N-acetyl-L-glutamic acid 2 | 1:2 | | 6 | 9 |
| Working Example A87 | A46 | | | | | $H_2N(CH_2)_3COO^-$ | | γ-aminobutyric acid | 1:1 | | 20 | 10 |
| Working Example A88 | A47 | | | | | $H_2N(CH_2)_5COO^-$ | | ε-aminocaproic acid | 1:1 | | 15 | 30 |
| Working Example A89 | A48 | | | | | Ascorbic acid anion | | Ascorbic acid | 1:1 | | 4 | 10 |

[Table 2C]

| | Compound | 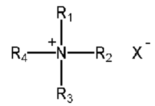 | | | | | Base | Acid | Compounding molar ratio (acid:base) | Compound concentration (wt%) | Solubility (g/100g) | |
|---|---|---|---|---|---|---|---|---|---|---|---|---|
| | | $R_1$ | $R_2$ | $R_3$ | $R_4$ | $X^-$ | | | | | Glutamic acid | Gallic acid |
| Working Example A90 | A49 | 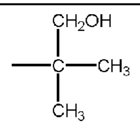 | H | H | H | CH3CH(OH)COO− | AMP | Lactic acid | 1:1 | 20 | 4 | 8 |
| Working Example A91 | A50 | $(CH_2)_3COO^-$ | H | H | H | $CH_3CH(OH)COO^-$ | γ-aminobutyric acid | Lactic acid | 1:1 | 20 | 2 | 7 |
| Working Example A92 | A51 | $(CH_2)_5COO^-$ | H | H | H | $CH_3CH(OH)COO^-$ | ε-aminocaproic acid | Lactic acid | 1:1 | 20 | 2 | 8 |
| Comparative Example A1 | A52 | Glycerin | | | | | | | - | 20 | <1 | 1 |
| Comparative Example A2 | A53 | Ion-exchange water | | | | | | | - | - | <1 | 1 |

3. Influence of molar ratio between cations and anions of organic ammonium salt on solubilities of active ingredients

Solubilities of active ingredients were evaluated via working examples A93 to A99 and comparative example A3 where the concentration of an ammonium salt, an ammonium salt composition, or a composition of an acid and/or a base was set to 20 wt%. As the active ingredients, there were used a poorly-soluble gallic acid having an antioxidant effect, and a glutamic acid having a moisture retention effect. A preparation method A of a test sample was such that after synthesizing an organic ammonium salt with an acid: lactic acid and a base: 2-amino-2-hydroxymethyl-1,3-propanediol (tromethamine), an ion-exchange water was added thereto to prepare an organic ammonium salt aqueous solution, followed by adding an active ingredient(s) thereto. A preparation method B was such that after preparing an organic ammonium salt aqueous solution by adding an acid: lactic acid and a base: tromethamine to an ion-exchange water, an active ingredient(s) was added thereto. A preparation method C was such that an acid: lactic acid, a base: tromethamine and an active ingredient(s) were added to water. Here, it was confirmed by $^1$H-NMR that an ammonium salt was formed in each of the preparation methods.

As for a compound A5, after examining the influence of the molar ratio between tromethamine and lactic acid as raw materials on solubilities, it was confirmed that as compared to a comparative example where only lactic acid was used in the test, in the working examples A93 to A99, the solubility of the gallic acid had improved, and the solubility of the glutamic acid was of the same level as or a level higher than that of the gallic acid; improvements in solubilities owing to the organic ammonium salt were confirmed. Particularly, as are the cases with the working examples A93, A94 and A95, an especially excellent solubility was observed when an ammonium salt whose molar ratio between anions and cations was 1:1 mol (molar ratio between acid and base 1:1) was present at a higher ratio. Further, as can be seen from the results of the working examples A93 and A95, it was confirmed that regardless of the preparation method, a similar level of solubility was able to be achieved as long as the molar ratio between acid and base was 1:1 mol.

As can be seen from the results shown in Table 3, in terms of the solubility of the gallic acid (not less than 3 g/100 g), it is preferred that the molar ratio between acid and base in the cosmetic compounding agent such as the hair treatment agent and the skin care agent be 1:1 mol to 2:1 mol; particularly, in terms of the solubilities of the glutamic acid (not less than 3 g/100 g) and the gallic acid (not less than 4 g/100 g), the molar ratio of 1:1 mol led to excellent solubilities. Further, other than the results shown in Table 3, a pH level was 8.4 when the molar ratio between acid and base was 1:2, and 9.1 when the molar ratio between acid and base was 1:5. The pH level, as far as safety is concerned, is preferably 3 to 10 with the molar ratio being 2:1 to 1:5, more preferably 5 to 9 with the molar ratio being 1:2 to 1:1, even more preferably 6 to 8 with the molar ratio being 1:1.

In this way, it was indicated that in the case of the cosmetic compounding agent of the present invention, excellent solubilities of the active ingredients were able to be achieved due to the structure of the organic ammonium salt, and that excellent solubilities were able to be achieved especially when the organic ammonium salt was such an organic ammonium salt that cations were present in a molar quantity equal to that of carboxylic acids in anions.

[Table 3]

| | Compound | Compound concentration (wt%) | Preparation method | Compounding molar ratio (acid:base) | | Solubility (g/100g) | | pH |
|---|---|---|---|---|---|---|---|---|
| | | | | Acid | Base | Glutamic acid | Gallic acid | |
| Working Example A93 | A5 | 20 | A | 1 | 1 | 3 | 4 | 6.2 |
| Working Example A94 | | | B | 1 | 1 | 3 | 4 | 6.2 |
| Working Example A95 | | | C | 1 | 1 | 3 | 4 | 6.2 |
| Working Example A96 | | | B | 100 | 1 | 2 | 2 | 1.4 |
| Working Example A97 | | | B | 10 | 1 | 2 | 2 | 2.4 |
| Working Example A98 | | | B | 5 | 1 | 2 | 2 | 2.9 |
| Working Example A99 | | | B | 2 | 1 | 2 | 3 | 3.6 |
| Comparative Example A3 | | | B | 1 | 0 | 2 | 1 | 1.1 |

4. Metal oxide dispersion test

Dispersibilities of metal oxides and carbon-based materials in the cosmetic material were observed. Here, samples in Tables 4 to 8 were prepared by the above preparation method B.

As for the compounds of the working and comparative examples shown in Table 4, 0.25 g of each compound,

0.50 g of an ion-exchange water and 0.10 g of zirconium oxide (IV) (by FUJIFILM Wako Pure Chemical Corporation) were mixed five times where the ingredients were mixed at a rate of 2,000 rpm for 1 min each time, using a rotation/revolution mixer (ARE-310 by THINKY CORPORATION); a dispersed state was then visually confirmed after mixing. Evaluation was performed by giving "○" to examples where the zirconium oxide was dispersed; and "×" to examples exhibiting a state where the zirconium oxide was not dispersed, but found to have precipitated. The results are shown in Table 4.

The zirconium oxide was able to be favorably dispersed with regard to each of the compounds of the working examples, and a dispersion liquid(s) were thus able to be obtained. In contrast, as for the compound of a comparative example A4, the zirconium oxide immediately precipitated and did not disperse.

[Table 4]

| | Compound | 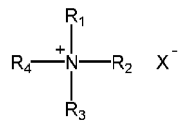 | | | | | Zirconium oxide dispersibility |
|---|---|---|---|---|---|---|---|
| | | $R_1$ | $R_2$ | $R_3$ | $R_4$ | x— | |
| Working Example A100 | A6 | 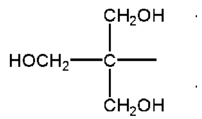 | H | H | H | $HOCH_2COO^-$ | ○ |
| Working Example A101 | A7 | | | | | $HOOC(CH_2)_2COO^-$ | ○ |
| Working Example A102 | A8 | | | | | $^-OOC(CH_2)_2COO^-$ | ○ |
| Comparative Example A4 | A54 | $CH_3(CH_2)_3$ | $CH_3(CH_2)_3$ | $CH_3(CH_2)_3$ | $CH_3(CH_2)_3$ | $Br^-$ | × |

Under an ingredient amount(s) and an operation that are similar to those described above, evaluation was performed using a titanium oxide (IV) (by FUJIFILM Wako Pure Chemical Corporation) instead of a zirconium oxide (Table 5).

The titanium oxide was able to be favorably dispersed with regard to each of the compounds of the working examples, and a dispersion liquid(s) were thus able to be obtained. In contrast, as for the compound of a comparative example A5, the titanium oxide precipitated and exhibited a low dispersibility.

That is, it is considered that in the case of the cosmetic compounding agent of the present invention, the titanium oxide was able to be favorably dispersed due to the fact that an affinity to the oxygen atoms in the hydrogen bond accepting titanium oxide had improved, and due to an affinity of the hydroxy groups in the quaternary ammonium salt to the metal atoms of the coordinating zirconium oxide and titanium oxide, as a result of utilizing the structural characteristics composed of cations having a large number of hydroxy groups as the hydrogen-bonding functional groups (hydrogen bond donating and coordinating groups).

This result indicated that the organic ammonium salt of the present invention was superior in affinity with inorganic materials such as hydrogen bond accepting functional group-containing metals and metal oxides, and that this organic ammonium salt was thus useful in a cosmetic compounding agent.

[Table 5]

| | Compound | 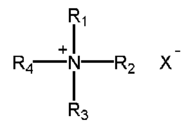 | | | | | Titanium oxide dispersibility |
|---|---|---|---|---|---|---|---|
| | | $R_1$ | $R_2$ | $R_3$ | $R_4$ | x— | |
| Working Example A103 | A6 | 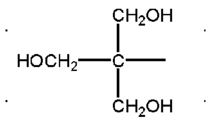 | H | H | H | $HOCH_2COO^-$ | ○ |
| Working Example A104 | A7 | | | | | $HOOC(CH_2)_2COO^-$ | ○ |
| Working Example A105 | A8 | | | | | $^-OOC(CH_2)_2COO^-$ | ○ |
| Comparative Example A5 | A54 | $CH_3(CH_2)_3$ | $CH_3(CH_2)_3$ | $CH_3(CH_2)_3$ | $CH_3(CH_2)_3$ | Br | × |

5. Redispersibility

A compound in a dry state was obtained by performing dehydration on the dispersion liquids of the zirconium oxide and titanium oxide that had been prepared in Tables 4 and 5, at 50°C and under a reduced pressure for three hours. There, 0.50 g of an ion-exchange water was added to the samples of the zirconium oxide and titanium oxide, followed by using a rotation/revolution mixer (ARE-310 by THINKY CORPORATION) to perform mixing five times where the ingredients were mixed at a rate of 2,000 rpm for 1 min each time; a dispersed state was then visually confirmed after mixing. As a result, a favorably dispersed state was observed with regard to each of the compounds of the working examples; uniform dispersion liquids were obtained. In contrast, as for a compound of a comparative example, precipitates were observed immediately after performing mixing with the mixer (Table 6).

From this result, it was confirmed that in the case of the organic ammonium salt of the present invention, a favorably dispersed state was able to be achieved even after drying a dispersed compound and then dispersing the same again, as the organic ammonium salt does not volatilize. Further, in addition to the dispersion method employing a system where a metal oxide(s) was added and then dispersed into the aqueous solution of the organic ammonium salt of the present invention, a favorably dispersed state was also able to be achieved in a system where water was added and then dispersed into a powder state; therefore, it was indicated that a redispersion of a hydrogen bond accepting functional group-containing material(s) contained in a cosmetic compounding agent was possible, thus being suitable for use in a cosmetic compounding agent.

[Table 6]

| | Compound | 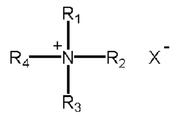 | | | | | Redispersibility | |
|---|---|---|---|---|---|---|---|---|
| | | $R_1$ | $R_2$ | $R_3$ | $R_4$ | x— | Zirconium oxide | Titanium oxide |
| Working Example A106 | A6 | 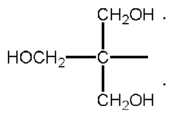 | H | H | H | $HOCH_2COO^-$ | ○ | ○ |
| Working Example A107 | A7 | | | | | $HOOC(CH_2)_2COO^-$ | ○ | ○ |
| Working Example A108 | A8 | | | | | $^-OOC(CH_2)_2COO^-$ | ○ | ○ |
| Comparative Example A6 | A54 | $CH_3(CH_2)_3$ | $CH_3(CH_2)_3$ | $CH_3(CH_2)_3$ | $CH_3(CH_2)_3$ | Br | × | × |

6. Metal oxide dispersion test 2

As for the compounds of the working and comparative examples shown in Table 7, 0.25 g of each compound, 0.50 g of an ion-exchange water and 0.10 g of zinc oxide (IV) (by ISHIHARA SANGYO KAISHA, LTD.) were mixed five times where the ingredients were mixed at a rate of 2,000 rpm for 1 min each time, using a rotation/revolution mixer (ARE-310 by THINKY CORPORATION); a dispersed state was then visually confirmed after mixing. Evaluation was performed by giving "○" to examples where the zinc oxide was dispersed; and "×" to examples exhibiting a state where the zinc oxide was not dispersed, but found to have precipitated. The results are shown in Table 7.

The zinc oxide was able to be favorably dispersed with regard to each of the compounds of the working examples, and a dispersion liquid(s) were thus able to be obtained. In contrast, as for the compound of a comparative example A7, the zinc oxide immediately precipitated and did not disperse.

That is, it is considered that in the case of the cosmetic compounding agent of the present invention, the zinc oxide was able to be favorably dispersed due to the fact that an affinity to the oxygen atoms in the hydrogen bond accepting zinc oxide had improved, and due to an affinity of the hydroxy groups in the quaternary ammonium salt to the metal atoms of the coordinating zinc oxide, as a result of utilizing the structural characteristics composed of cations having a large number of hydroxy groups as the hydrogen-bonding functional groups (hydrogen bond donating and coordinating groups).

[Table 7]

| | Compound | 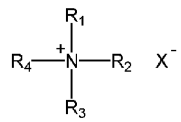 | | | | | Zinc oxide dispersibility |
|---|---|---|---|---|---|---|---|
| | | $R_1$ | $R_2$ | $R_3$ | $R_4$ | x— | |
| Working Example A109 | A6 | 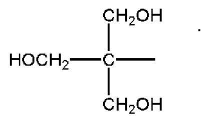 | H | H | H | $HOCH_2COO^-$ | ○ |
| Working Example A110 | A7 | | | | | $HOOC(CH_2)_2COO^-$ | ○ |
| Working Example A111 | A8 | | | | | $^-OOC(CH_2)_2COO^-$ | ○ |
| Comparative Example A7 | A54 | $CH_3(CH_2)_3$ | $CH_3(CH_2)_3$ | $CH_3(CH_2)_3$ | $CH_3(CH_2)_3$ | $Br^-$ | × |

7. Carbons (carbon nanotube) dispersibility test

As for the compounds of the working and comparative examples shown in Table 8, 0.25 g of each compound, 0.75 g of an ion-exchange water and 0.025 g of carbon nanotubes as carbons (multilayered, 3 to 20 nm) (by ISHIHARA SANGYO KAISHA, LTD.) were mixed five times where the ingredients were mixed at a rate of 2,000 rpm for 1 min each time, using a rotation/revolution mixer (ARE-310 by THINKY CORPORATION); a dispersed state was then visually confirmed after mixing. Evaluation was performed by giving "o" to examples where the carbon nanotubes were dispersed; and "×" to examples exhibiting a state where the carbon nanotubes were not dispersed, but found to have precipitated. The results are shown in Table 8.

As a result, the carbon nanotubes were able to be favorably dispersed with regard to each of the compounds of the working examples, and a low-viscosity dispersion liquid(s) with a favorable handling property were thus able to be obtained. In contrast, as for the compound of a comparative example A8, the carbon nanotubes precipitated and did not disperse.

That is, it is considered that in the case of the cosmetic compounding agent of the present invention, the carbon nanotubes were able to be favorably dispersed due to a favorable affinity to carbons, and due to the fact that in the case of such hydrogen bond accepting carbon nanotubes, an affinity to the carbon-carbon unsaturated bond(s) ($\pi$ electron system) had improved, as a result of utilizing the structural characteristics composed of cations having a large number of hydroxy groups as the hydrogen-bonding functional groups (hydrogen bond donating and coordinating groups).

This result indicated that the organic ammonium salt of the present invention was superior in affinity with carbons, and that this organic ammonium salt was thus useful in a cosmetic compounding agent using such a kind of material.

[Table 8]

<table>
<tr><td rowspan="2"></td><td rowspan="2">Compound</td><td colspan="5">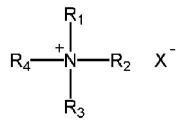</td><td rowspan="2">Carbon nanotube dispersibility</td></tr>
<tr><td>$R_1$</td><td>$R_2$</td><td>$R_3$</td><td>$R_4$</td><td>X—</td></tr>
<tr><td>Working Example A112</td><td>A6</td><td rowspan="2">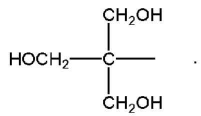</td><td rowspan="2">H</td><td rowspan="2">H</td><td rowspan="2">H</td><td>$HOCH_2COO^-$</td><td>○</td></tr>
<tr><td>Working Example A113</td><td>A7</td><td>$HOOC(CH_2)_2COO^-$</td><td>○</td></tr>
<tr><td>Comparative Example A8</td><td>A54</td><td>$CH_3(CH_2)_3$</td><td>$CH_3(CH_2)_3$</td><td>$CH_3(CH_2)_3$</td><td>$CH_3(CH_2)_3$</td><td>$Br^-$</td><td>×</td></tr>
</table>

[2] Evaluation of hair treatment agent

Hydrates of compounds B1 to B29 shown in Tables 9 to 21 were synthesized and obtained by the following methods.

Compounds B1 to B4: synthesized by a method described in JP-A-2014-131975.

Compounds B5 to B22: synthesized by a method described in JP-A-2014-131974.

Compound B23: synthesized by a method described in JP-A-2012-031137.

Compound B24: as tetrabutylammonium bromide, a reagent produced by KANTO CHEMICAL CO., INC. was used.

Compound B25: as 1-butyl-3-methylimidazolium chloride (BMI-Cl), a reagent produced by Tokyo Chemical Industry Co., Ltd. was used.

Compound B26: as glycerin, a reagent produced by FUJIFILM Wako Pure Chemical Corporation was used.

Compound B27: an ion-exchange water was used.

Compound B28: as a sodium lactate, a reagent produced by KANTO CHEMICAL CO., INC. was used.

Compound B29: 2-amino-2-hydroxymethyl-1,3-propanediol hydrochloride (tris-HCl) was synthesized with reference to a method described in JP-A-2014-131974.

Compound B30: 2-amino-2-hydroxymethyl-1,3-propanediol oleate was synthesized by a method described in JP-A-2014-131974.

Compound B31: 2-amino-2-hydroxymethyl-1,3-propanediol linoleate was synthesized by a method described in JP-A-2014-131974.

Compound B32: 2-amino-2-hydroxymethyl-1,3-propanediol isostearate was synthesized by a method described in JP-A-2014-131974.

Compound B33: 2-amino-2-methyl-1,3-propanediol oleate was synthesized by a method described in JP-A-2014-131974.

Compound B34: 2-amino-2-methyl-1,3-propanediol linoleate was synthesized by a method described in JP-A-2014-131974.

Compound B35: 2-amino-2-methyl-1,3-propanediol isostearate was synthesized by a method described in JP-A-2014-131974.

In the following evaluations, a human black hair produced by Beaulax Co., Ltd was used as a healthy hair. A damaged hair was prepared by repeatedly having the healthy hair permed and bleached three times.

1. Water retention property and adherability of hair treatment agent to hair of hair treatment agent

1-1. Water retention property and adherability with respect to healthy hair when using organic ammonium salt

An 80 wt% aqueous solution of each of the compounds B1 to B26 (content in terms of anhydride in the cases of the compounds B1 to B23) was prepared, followed by using a Karl Fischer moisture meter (KF-200 by Mitsubishi Chemical Analytech Co., Ltd.) to confirm that a moisture rate was 20.0 wt%. As a healthy hair, there was used a chemically untreated healthy hair (human black hair by Beaulax Co., Ltd). The moisture rate (pre-test moisture rate A) of the hair was measured by an infrared moisture meter (by Kett Electric Laboratory).

Here, 0.05 g (pre-test hair weight A) of the hair was dipped into and then kept immersed in 3.0 g of an 80 wt% aqueous solution of each of the compounds B1 to B26 for 60 min. After immersion, the hair was taken out, and KimWipes were then used to wipe off the compound until a change in weight was no longer observed, followed by measuring the

weight of the hair (post-test hair weight B). The hair from which the compound had been wiped off was then placed into a thermo-hygrostat set to 40°C and 35 to 40%RH. A moisture rate after 24 hours (post-test moisture rate B) was then measured, followed by calculating a rate of reduction in moisture with the following formula. Likewise, instead of the 80 wt% aqueous solution, evaluation was also performed in a similar manner using only an ion-exchange water (comparative example B4).

$$\text{Rate of reduction in moisture (\%)} = [(\text{pre-test moisture rate A} - \text{post-test moisture rate B}) / \text{pre-test moisture rate A}] \times 100$$

Further, an adhesion rate of the compound adhering to the hair was calculated with the following formula.

$$\text{Compound adhesion rate (\%)} = [(\text{post-test hair weight B (g)} - \text{pre-test hair weight A (g)}) / \text{pre-test hair weight A (g)}] \times 100$$

[Table 9A]

| | Compound | 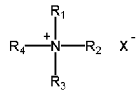 | | | | | Compounding molar ratio (acid:base) | Moisture content (wt%) | Melting point | Healthy hair | | | |
|---|---|---|---|---|---|---|---|---|---|---|---|---|---|
| | | $R_1$ | $R_2$ | $R_3$ | $R_4$ | $X^-$ | | | | Pre-test moisture rate | Post-test moisture rate | Rate of reduction in moisture | Compound adhesion rate |
| Working Example B1 | B1 | $(CH_2)_2OH$ | H | H | H | $CH_3CH(OH)COO^-$ | 1:1 | 10.7 | <-10 | 7.6% | 5.9% | 22% | 2.1% |
| Working Example B2 | B2 | $(CH_2)_2OH$ | $(CH_2)_2OH$ | H | H | $CH_3CH(OH)COO^-$ | 1:1 | 15.6 | <-10 | 7.6% | 6.1% | 20% | 2.0% |
| Working Example B3 | B3 | $(CH_2)_2OH$ | $(CH_2)_2OH$ | $(CH_2)_2OH$ | H | $CH_3CH(OH)COO^-$ | 1:1 | 18.4 | <-10 | 7.6% | 6.8% | 11% | 2.4% |
| Working Example B4 | B4 | $(CH_2)_2OH$ | $(CH_2)_2OH$ | $(CH_2)_2OH$ | H | $HOCH_2COO^-$ | 1:1 | 19.4 | <-10 | 7.6% | 6.8% | 11% | 2.4% |
| Working Example B5 | B5 | 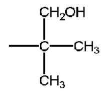 | H | H | H | $CH_3CH(OH)COO^-$ | 1:1 | 16.7 | <-10 | 7.6% | 6.4% | 16% | 1.1% |
| Working Example B6 | B6 | 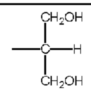 | H | H | H | $CH_3CH(OH)COO^-$ | 1:1 | 16.6 | <-10 | 7.6% | 6.8% | 11% | 2.8% |
| Working Example B7 | B7 | 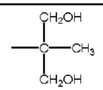 | H | H | H | Ascorbic acid anion | 1:1 | 12.0 | <-10 | 7.6% | 6.0% | 21% | 2.4% |
| Working Example B8 | B8 | 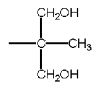 | H | H | H | $CH_3CH(OH)COO^-$ | 1:1 | 15.6 | <-10 | 7.6% | 6.1% | 20% | 1.3% |
| Working Example B9 | B9 | 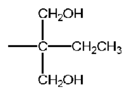 | H | H | H | $CH_3CH(OH)COO^-$ | 1:1 | 14.7 | <-10 | 7.6% | 5.9% | 22% | 1.8% |
| Working Example B10 | B10 | 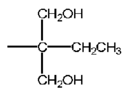 | H | H | H | $Cl^-$ | 1:1 | 18.8 | <-10 | 7.6% | 5.4% | 29% | 1.5% |
| Working Example B11 | B11 | 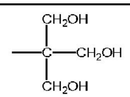 | H | H | H | $CH_3CH(OH)COO^-$ | 1:1 | 13.2 | <-10 | 7.6% | 6.8% | 11% | 2.8% |
| Working Example B12 | B12 | 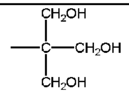 | H | H | H | $HOCH_2COO^-$ | 1:1 | 15.5 | >25 | 7.6% | 5.4% | 29% | 1.6% |

[Table 9B]

| | | | | | | | | | | | | | |
|---|---|---|---|---|---|---|---|---|---|---|---|---|---|
| Working *Example B13* | B13 | 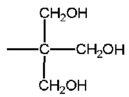 | H | H | H | $C(OH)(CH_2COOH)_2COO^-$ | 1:1 | 10.3 | <-10 | 7.6% | 6.5% | 15% | 2.3% |
| Working Example B14 | B14 | 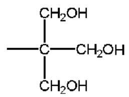 | H | H | H | $HOCH_2COO^-$ | 1:1 | 15.5 | >25 | 7.6% | 7.4% | 3% | 1.3% |
| Working Example B15 | B15 | 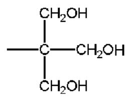 | H | H | H | $HOOCCH_2CH(OH)COO^-$ | 1:1 | 12.4 | <-10 | 7.6% | 5.8% | 23% | 2.0% |
| Working Example B16 | B16 | 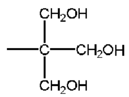 | H | H | H | $^-OOCCH_2CH(OH)COO^-$ | 1:2 | 8.7 | <-10 | 7.6% | 5.5% | 28% | 2.1% |
| Working Example B17 | B17 | 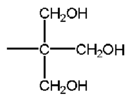 | H | H | H | $CH_3COCH_2CH_2COOH^-$ | 1:1 | 13.2 | <-10 | 7.6% | 6.5% | 15% | 6.9% |
| Working Example B18 | B18 | 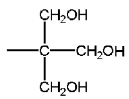 | H | H | H | $HOOCCH_2CH(COO^-)NH_2$ | 1:1 | 12.4 | <-10 | 7.6% | 5.6% | 26% | 5.1% |
| Working Example B19 | B19 | 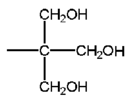 | H | H | H | $^-OOC(CH_2)_2CH(COO^-)$ $NHCOCH_3$ | 1:2 | 7.7 | >25 | 7.6% | 5.6% | 26% | 9.4% |
| Working Example B20 | B20 | 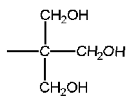 | H | H | H | Ascorbic acid anion | 1:1 | 10.8 | <-10 | 7.6% | 6.6% | 14% | 2.1% |
| Working Example B21 | B21 | $(CH_2)_3COOH$ | H | H | H | $CH_3CH(OH)COO^-$ | 1:1 | 7.4 | <-10 | 7.6% | 5.8% | 24% | 1.1% |
| Working Example B22 | B22 | $(CH_2)_5COOH$ | H | H | H | $CH_3CH(OH)COO^-$ | 1:1 | 6.7 | <-10 | 7.6% | 5.8% | 24% | 1.1% |
| Working Example B23 | B23 | $CH_3$ | $CH_3$ | $CH_3$ | $(CH_2)_2OH$ | $CH_3CH(OH)COO^-$ | 1:1 | 11.2 | <-10 | 7.6% | 5.5% | 28% | 1.8% |

(continued)

| | | | | | | | | | | | | | |
|---|---|---|---|---|---|---|---|---|---|---|---|---|---|
| Comparative Example B1 | B24 | $CH_3(CH_2)_3$ | $CH_3(CH_2)_3$ | $CH_3(CH_2)_3$ | $CH_3(CH_2)_3$ | $Br^-$ | 1:1 | <1 | >25 | 7.6% | 4.0% | 47% | 1.0% |
| Comparative Example B2 | B25 | BMI-Cl | | | | | — | <1 | >25 | 7.6% | 4.0% | 47% | 1.0% |
| Comparative Example B3 | B26 | Glycerin | | | | | — | <1 | >-5 | 7.6% | 5.3% | 30% | 1.0% |
| Comparative Example B4 | B27 | Ion-exchange water | | | | | - | — | 0 | 7.6% | 3.9% | 49% | 0.0% |

As can be seen from the results shown in Table 9, it was confirmed that the healthy hairs treated with the compounds B1 to B23 of the working examples exhibited rates of reduction in moisture that were smaller than those of the healthy hairs treated with the compounds B24 to B27 of the comparative examples; and that the compounds B1 to B23 of the working examples were thus superior in water retention property for healthy hairs. Further, as a result of measuring the hair adhesion rate based on the change in weight, adhesion was not observed in the comparative example B4 where an ion-exchange water was used, and an adhesion rate of 1.0% was observed with regard to each of the compounds B24, B25 and B26 of the comparative examples B1, B2 and B3; whereas the adhesion rate was 1.1 to 9.4% with regard to the compounds of the working examples. In this sense, it was confirmed that the compounds of the working examples were superior in adherability to the hair; and the structure of the organic ammonium salt involving the hydrogen-bonding functional group(s) and cations thus exhibited superiorities in water retention property and adherability to the hair. Further, although having the same cation(s), the compound B11 being a liquid at 25°C exhibited a rate of reduction in moisture that was smaller than and an adhesion rate that was larger than those of the compound B12 being a solid at 25°C; the compound being a liquid at 25°C was superior in water retention property and adherability. Moreover, although having the same anion(s), since the compound B3 exhibited a rate of reduction in moisture that was smaller than and an adhesion rate was larger than those of the compound B23, cations only composed of the hydrogen-bonding functional group(s) and having no alkyl groups in ammonium cations were superior in water retention property and adherability. In addition, although having the same anion(s), since the compounds B6 and B11 exhibited rates of reduction in moisture that were smaller than and adhesion rates that were larger than those of the compound B9, a superior water retention property and adherability were able to be brought about by a type of compound that was only composed of the hydrogen-bonding functional group(s) in a way such that in the structure of the hydrogen-bonding functional group-containing $R^1$, the terminals were all hydroxy groups.

As can be seen from these results, it was indicated that since the hair treatment agent containing the organic ammonium salt of the present invention was superior in adherability to a healthy hair, the water and moisture retention property of a healthy hair could be maintained for a long period of time. Further, it was also indicated that these effects were high if the compound was such a compound being a liquid at 25°C.

1-2. Water retention property and adherability with respect to damaged hair when using organic ammonium salt

An 80 wt% aqueous solution of each of the compounds B1 to B4, B6, B7, B9 to B13, and B23 to B26 (content in terms of anhydride in the cases of the compounds B1 to B4, B6, B7, B9 to B13, and B23) was prepared, followed by using a Karl Fischer moisture meter to confirm that a moisture rate was 20.0 wt%. A damaged hair was prepared by the following method.

<Preparation of damaged hair>

As a perm treatment, a healthy hair was dipped into and kept immersed in a 6.5% thioglycolic acid ammonium aqueous solution (pH 9.5) for 10 min, and then dipped into and kept immersed in a 6% sodium bromate aqueous solution (pH 6.5) for another 10 min, followed by rinsing the hair, drying the hair with a dryer, and then brushing the hair 100 times. Next, as a bleach treatment, the hair was further dipped into and kept immersed in a 1:1 mixed solution of a 5% hydrogen peroxide water and a 2.5% ammonia water for 30 min, followed by rinsing the hair, drying the hair with a dryer, and then brushing the hair 100 times. A damaged hair was produced by performing these perm treatment and bleach treatment three times.

Using the aforementioned sample(s) and only an ion-exchange water (comparative example B8), the rate of reduction in moisture, hair adhesion rate and compound adhesion rate were calculated by a method(s) similar to those described in "1-1.".

[Table 10]

| | Compound No. | 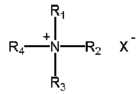 | | | | | Compounding molar ratio (acid:base) | Moisture content (wt%) | Melting point | Damaged hair | | | |
|---|---|---|---|---|---|---|---|---|---|---|---|---|---|
| | | $R_1$ | $R_2$ | $R_3$ | $R_4$ | X— | | | | Pre-test moisture rate | Post-test moisture rate | Rate of reduction in moisture | Compound adhesion rate |
| Working Example B24 | Compound B1 | $(CH_2)_2OH$ | H | H | H | $CH_3CH(OH)COO^-$ | 1:1 | 10.7 | <-10 | 8.0% | 3.7% | 53% | 1.6% |
| Working Example B25 | Compound B2 | $(CH_2)_2OH$ | $(CH_2)_2OH$ | H | H | $CH_3CH(OH)COO^-$ | 1:1 | 15.6 | <-10 | 8.0% | 3.9% | 51% | 16% |
| Working Example B26 | Compound B3 | $(CH_2)_2OH$ | $(CH_2)_2OH$ | $(CH_2)_2OH$ | H | $CH_3CH(OH)COO^-$ | 1:1 | 18.4 | <-10 | 8.0% | 5.2% | 35% | 2.2% |
| Working Example B27 | Compound B4 | $(CH_2)_2OH$ | $(CH_2)_2OH$ | $(CH_2)_2OH$ | H | $HOCH_2COO^-$ | 1:1 | 19.4 | <-10 | 8.0% | 3.9% | 51% | 1.8% |
| Working Example B28 | Compound B6 | 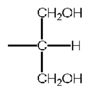 | H | H | H | $CH_3CH(OH)COO^-$ | 1:1 | 16.6 | <-10 | 8.0% | 5.2% | 35% | 2.3% |
| Working Example B29 | Compound B7 | 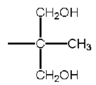 | H | H | H | Ascorbic acid anion | 1:1 | 12.0 | <-10 | 8.0% | 3.6% | 55% | 1.6% |
| Working Example B30 | Compound B9 | 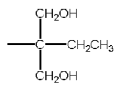 | H | H | H | $CH_3CH(OH)COO^-$ | 1:1 | 14.7 | <-10 | 8.0% | 3.6% | 55% | 1.8% |
| Working Example B31 | Compound B10 | 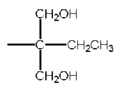 | H | H | H | $Cl^-$ | 1:1 | 18.8 | <-10 | 8.0% | 3.5% | 56% | 1.5% |
| Working Example B32 | Compound B11 | 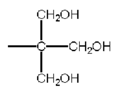 | H | H | H | $CH_3CH(OH)COO^-$ | 1:1 | 13.2 | <-10 | 8.0% | 5.9% | 26% | 2.8% |
| Working Example B33 | Compound B12 | 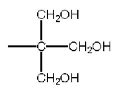 | H | H | H | $HOCH_2COO^-$ | 1:1 | 15.5 | >25 | 8.0% | 3.0% | 63% | 1.4% |

(continued)

| | Compound No. | 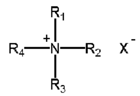 | | | | | Compounding molar ratio (acid:base) | Moisture content (wt%) | Melting point | Damaged hair | | | |
|---|---|---|---|---|---|---|---|---|---|---|---|---|---|
| | | $R_1$ | $R_2$ | $R_3$ | $R_4$ | X— | | | | Pre-test moisture rate | Post-test moisture rate | Rate of reduction in moisture | Compound adhesion rate |
| Working Example B34 | Compound B13 | 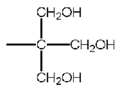 | H | H | H | $C(OH)(CH_2COOH)_2COO^-$ | 1:1 | 10.3 | <-10 | 8.0% | 5.4% | 33% | 2.4% |
| Working Example B35 | Compound B23 | $CH_3$ | $CH_3$ | $CH_3$ | $(CH_2)_2OH$ | $CH_3CH(OH)COO^-$ | 1:1 | 11.2 | <-10 | 8.0% | 5.0% | 38% | 2.0% |
| Comparative Example B5 | Compound B24 | $CH_2(CH_2)_3$ | $CH_2(CH_2)_3$ | $CH_3(CH_2)_3$ | $CH_3(CH_2)_3$ | $Br^-$ | 1:1 | <1 | >25 | 8.0% | 2.0% | 75% | 1.0% |
| Comparative Example B6 | Compound B25 | BMI-Cl | | | | | — | <1 | >25 | 8.0% | 1.9% | 76% | 0.9% |
| Comparative Example B7 | Compound B26 | Glycerin | | | | | — | <1 | >-5 | 8.0% | 2.6% | 68% | 1.2% |
| Comparative Example B8 | Compound B27 | Ion-exchange water | | | | | — | — | 0 | 8.0% | 1.9% | 76% | 0.0% |

As can be seen from the results shown in Table 10, it was confirmed that the damaged hairs treated with the compounds B1 to B4, B6, B7, B9 to B13 and B23 of the working examples exhibited rates of reduction in moisture that were smaller than those of the damaged hairs treated with the compounds B24 to B27 of the comparative examples; the damaged hairs treated with those compounds of the working examples exhibited superior water retention properties. As for the hair adhesion rate, while adhesion was not observed with regard to the comparative example B8 where an ion-exchange water was used, and while the comparative examples B5 to B7 exhibited hair adhesion rates of 0.9 to 1.2%, the compounds of the working examples exhibited adhesion rates of 1.4 to 2.8% i.e. it was indicated that the compounds of the working examples were superior in adherability to the hair.

Further, as a result of using the present invention's 2-amino-2-hydroxymethyl-1,3-propanediol oleate (compound B30), 2-amino-2-hydroxymethyl-1,3-propanediol linoleate (compound B31), 2-amino-2-hydroxymethyl-1,3-propanediol isostearate (compound B32), 2-amino-2-methyl-1,3-propanediol oleate (compound B33), 2-amino-2-methyl-1,3-propanediol linoleate (compound B34) and 2-amino-2-methy1-1,3-propanediol isostearate (compound B35) to the healthy and damaged hairs, and then confirming the adherability thereof to the healthy hair by a method similar to the method described above, a higher adherability was observed as compared to the compounds of the comparative examples B1 to B8. As for the moisture barrier property of the hair, as a result of evaluating the compound B31 (working example) by a method described in (4) Water confinement property test in [3] Evaluation of skin care agent, a water evaporation inhibition rate was confirmed to be 48.1% which was higher than the water evaporation inhibition rates of comparative examples C16 to 18 i.e. it was indicated that the compound of the working example was superior in moisture barrier property of the hair.

1-3. Influence of molar ratio between cations and anions of organic ammonium salt on water retention property

The rates of reduction in moisture, hair adhesion rates and compound adhesion rates of the healthy and damaged hairs were measured via working examples B36 to B43 and comparative examples B9 and B10 where as shown in Table 11, the concentration of an ammonium salt, an ammonium salt composition, or a composition of an acid and/or a base was set to 50 wt%. The damaged hair was prepared by the method described in 1-2.. Further, the rate of reduction in moisture, hair adhesion rate and compound adhesion rate were calculated by methods similar to the methods shown in 1-1.. A preparation method A of a test sample was such that after synthesizing an organic ammonium salt with an acid: lactic acid and a base: tromethamine, an ion-exchange water was added thereto to prepare an organic ammonium salt aqueous solution. A preparation method B was carried out by adding an acid: lactic acid and a base: tromethamine to an ion-exchange water (formation of ammonium salt was confirmed by $^{1}$H-NMR). Here, among the samples of Tables 9, 10, 12 to 17 and 21, compounds other than the compound B23 were prepared by the preparation method B.

[Table 11]

| | Compound | Compound concentration (wt%) | Preparation method of organic ammonium salt | Compounding molar ratio (acid:base) | | Water retention property (healthy hair) | | | | Water retention property (damaged hair) | | | |
|---|---|---|---|---|---|---|---|---|---|---|---|---|---|
| | | | | Acid | Base | Pre-test moisture rate (%) | Post-test moisture rate (%) | Rate of reduction in moisture (%) | Compound adhesion rate (%) | Pre-test moisture rate (%) | Post-test moisture rate (%) | Rate of reduction in moisture (%) | Compound adhesion rate (%) |
| Working Example B36 | B11 | 50 | A | 1 | 1 | 7.6% | 6.8% | 11% | 2.8 | 8.0% | 5.9% | 26% | 2.8 |
| Working Example B37 | | | B | 1 | 1 | 7.6% | 6.8% | 11% | 2.8 | 8.0% | 5.9% | 26% | 2.8 |
| Working Example B38 | | | B | 100 | 1 | 7.6% | 5.0% | 34% | 2.3 | 8.0% | 4.1% | 49% | 1.6 |
| Working Example B39 | | | B | 10 | 1 | 7.6% | 4.6% | 39% | 1.7 | 8.0% | 4.3% | 46% | 1.7 |
| Working Example B40 | | | B | 5 | 1 | 7.6% | 4.6% | 39% | 2.3 | 8.0% | 4.5% | 44% | 2.4 |
| Working Example B41 | | | B | 2 | 1 | 7.6% | 4.2% | 45% | 2.3 | 8.0% | 4.5% | 44% | 2.3 |
| Working Example B42 | | | B | 1 | 2 | 7.6% | 4.5% | 41% | 2.0 | 8.0% | 3.6% | 55% | 2.1 |
| Working Example B43 | | | B | 1 | 5 | 7.6% | 4.5% | 41% | 1.8 | 8.0% | 3.1% | 61% | 2.0 |
| Comparative Example B9 | | | B | 1 | — | 7.6% | 4.0% | 47% | 1.1 | 8.0% | 3.0% | 63% | 1.1 |
| Comparative Example B10 | B27 (Ion-exchange water) | | | | | 7.6% | 3.9% | 49% | 0.0 | 8.0% | 1.9% | 76% | 0.0 |

As can be seen from the results shown in Table 11, it was confirmed that the healthy and damaged hairs treated with the working examples B36 to B43 exhibited rates of reduction in moisture that were smaller than those of the healthy and damaged hairs treated with the comparative examples; the healthy and damaged hairs treated with those compounds of the working examples exhibited superior water retention properties. As for the hair adhesion rate, while adhesion was not observed with regard to the comparative example B10 where an ion-exchange water was used, and while both the healthy and damaged hairs in the comparative example B9 where lactic acid was used exhibited a hair adhesion rate of 1.1%, the adhesion rates of the healthy hairs in the working examples were 1.7 to 2.8%, and the adhesion rates of the damaged hairs in the working examples were 1.6 to 2.8%, i.e. it was indicated that the healthy and damaged hairs in the working examples were superior in adherability.

As for the compound B11, as a result of studying the influence of the molar ratio between 2-amino-2-hydroxymethyl-1,3-propanediol (tromethamine) and lactic acid as materials on the water retention property of the healthy and damaged hairs, it was confirmed that in the working examples B36 to B43, the water retention property had improved as compared to the comparative example where only lactic acid was used in the test; a water retention effect by the organic ammonium salt was confirmed. In terms of adherability to the hair, it is preferred that a compounding ratio between acid and base be 1:5 to 5:1 mol. Particularly, when the molar ratio between anions and cations was 1:1 mol, an excellent adherability, the lowest rate of reduction in moisture and thereby an especially superior water retention property was observed. Further, as can be seen from the results of the working examples B36 and B37, it was confirmed that regardless of the preparation method, a similar level of water retention effect was able to be achieved as long as the molar ratio between anions and cations was 1:1 mol.

In this way, it was indicated that in the case of the hair treatment agent of the present invention, excellent water retention properties were able to be achieved due to the structure of the organic ammonium salt, and that excellent water retention properties were able to be achieved especially when the organic ammonium salt was such an organic ammonium salt that cations were present in a molar quantity equal to that of carboxylic acids in anions.

1-4. Surface resistance measurement of organic ammonium salt

Here, 0.7 g (content in terms of anhydride) of each of the compounds B11 and B26 was applied to an artificial leather made of polyurethane, followed by using a high resistance meter (Stack TR-2 by Tokyo Electronics Co., Ltd.) to measure a surface resistance value. While the surface resistance value was greater than $4\times10^{11}\Omega$ when nothing had yet been applied thereto, a surface resistance value of lower than $1\times10^{8}\Omega$ was observed in the case of the compound B11, and a surface resistance value of $5\times10^{8}\Omega$ was observed in the case of the compound B26, provided that the compounds were both in a liquid state at the time of measurement; it was indicated that as compared to the compound B26, the compound B11 was able to bring about a more favorable electrical conductivity on the coating surface, suppress static electricity and impart an antistatic effect, thus showing a superiority of the structural characteristics of the hair treatment agent of the present invention.

Further, 0.7 g (content in terms of anhydride) of the compound B28 (sodium lactate) was applied to an artificial leather made of polyurethane. After drying the same to volatilize water, the coating surface turned into a solid state, and a surface resistance value measured in a similar manner as above was $2\times10^{8}\Omega$; confirmed were a non-volatility of the compound B11 (lower than $1\times10^{8}\Omega$) and an effect of liquidity at 25°C (capability of evenly coating an object to be coated).

In this way, it was indicated that the hair treatment agent of the present invention was superior in antistatic property and contributed to senses such as hair cohesiveness.

2. Use of hair treatment agent on healthy and damaged hairs

2-1. Sensory evaluation on healthy hair after using organic ammonium salt

Here, an 80 wt% aqueous solution(s) of each of the compounds B11, B12, B23, B26 and B27 (content in terms of anhydride in the cases of the compounds B11, B12 and B23) were prepared and used as samples. Next, one minute was spent in evenly applying 10.0 g of each sample to a bundle of the healthy hairs weighing about 10 g, using a brush; the bundle of the healthy hairs was then left for 5 min. After leaving it for 5 min, the bundle of the healthy hairs was rinsed with a hot water of 40°C for 30 sec, and evaluated was a finger-combing capability at the time of performing washing. The hair bundle that had been rinsed was then dried with a towel and further subjected to air drying for a day, followed by evaluating items such as flexibility, finger-combing capability, texture, elasticity/resilience (feeling by bare hands), volume increase (visual evaluation), cohesiveness, moist feeling, surface smoothness, non-stickiness (feeling by bare hands) and gloss (visual evaluation), from the perspective of superiority or inferiority to a test hair bundle(s) uncoated with the sample.

The following four criteria were used for evaluation.

◎: Superior
○: Favorable
Δ: No change
×: Inferior

[Table 12]

| | Evaluation item | Working example B44 | Working example B45 | Working example B46 | Comparative example B11 | Comparative example B12 |
|---|---|---|---|---|---|---|
| | | Compound B11 | Compound B12 | Compound B23 | Compound B26 | Compound B27 |
| When washing | Finger-combing capability | ◎ | ◎ | ◎ | ○ | △ ~ × |
| After air drying | Flexibility | ○ | ○ ~ △ | ○ | △ ~ × | △ ~ × |
| | Finger-combing capability | ○ | ○ ~ △ | ○ | △ ~ × | △ ~ × |
| | Texture | ○ | ○ ~ △ | ○ | △ ~ × | △ ~ × |
| | Elasticity / Resilience | ○ ~ △ | ○ ~ △ | ○ ~ △ | △ ~ × | △ ~ × |
| | Volume increase | ○ | ○ ~ △ | ○ ~ △ | △ ~ × | △ ~ × |
| | Cohesiveness of hair | ○ | ○ ~ △ | ○ | △ ~ × | △ ~ × |
| | Moist feeling | ○ | ○ ~ △ | ○ | △ ~ × | △ ~ × |
| | Gloss of hair | ○ | ○ ~ △ | ○ | △ ~ × | △ ~ × |
| | Smoothness of hair surface | ○ | ○ ~ △ | ○ | △ ~ × | △ ~ × |
| | Non-stickiness of hair surface | ○ | ○ ~ △ | ○ | △ ~ × | △ ~ × |

As can be seen from the results shown in Table 12, in the cases of the compounds B11, B12 and B23 of the working examples, superior finger-combing capabilities at the time of performing washing were observed as compared to the comparative examples. Further, even after air drying, the compounds of the working examples exhibited favorable effects in all the items evaluated, as compared to the comparative examples. From these results, it was confirmed that a compound(s) composed of the salt structure of cations and anions, such as the salt structure of the working examples, were capable of exhibiting favorable effects as a hair treatment agent. In addition, as compared to the compound B12, the compounds B11 and B23 of the working examples were superior in flexibility, finger-combing capability, texture, cohesiveness, moist feeling, gloss, surface smoothness and non-stickiness. From these results, it was confirmed that excellent results were able to be obtained in various sensory evaluations due to the fact that since the quaternary ammonium salt was a liquid, active ingredients would not be precipitated when water used as a solvent had volatilized. Particularly, as compared to the compound B23 containing alkyl groups in the quaternary ammonium cations, the compound B11 only composed of the hydrogen-bonding functional group(s) was able to allow the product of the present invention to be favorably immobilized on the hair for a long period of time; the compound B11 exhibited a favorable result in the evaluation of volume increase, and the evaluation result thereof was such that cations could be selected based on a need for increase in volume.

Further, as a result of performing a sensory evaluation(s) on the healthy hair by a method similar to that described above, using 2-amino-2-hydroxymethyl-1,3-propanediol oleate (compound B30), 2-amino-2-hydroxymethyl-1,3-propanediol linoleate (compound B31), 2-amino-2-hydroxymethyl-1,3-propanediol isostearate (compound B32), 2-amino-2-methyl-1,3-propanediol oleate (compound B33), 2-amino-2-methyl-1,3-propanediol linoleate (compound B34) and 2-amino-2-methyl-1,3-propanediol isostearate (compound B35), the evaluation results were such that as compared to the compounds of the comparative examples, there were observed a superior flexibility, finger-combing capability, texture, elasticity/resilience, volume increase, cohesiveness, moist feeling, surface smoothness, non-stickiness and gloss.

From these results, it was indicated that the hair treatment agent of the present invention was capable of bringing about a favorable feeling and appearance by imparting to the hair a superior flexibility, finger-combing capability, texture, elasticity/resilience, volume increase, cohesiveness, moist feeling, surface smoothness, non-stickiness and gloss, after rinsing the hair.

2-2. Sensory evaluation on damaged hair after using organic ammonium salt

Here, an 80 wt% aqueous solution(s) of each of the compounds B11, B12, B23, B26 and B27 (content in terms of anhydride in the cases of the compounds B11, B12 and B23) were prepared and used as samples. Next, one minute was spent in evenly applying 10.0 g of each sample to a bundle of the damaged hairs weighing about 10 g, using a brush; the bundle of the damaged hairs was then left for 5 min. After leaving it for 5 min, the bundle of the damaged hairs was rinsed with a hot water of 40°C for 30 sec, and evaluated was a finger-combing capability at the time of performing washing. The hair bundle that had been rinsed was then dried with a towel and further subjected to air drying for a day, followed by evaluating items such as elasticity/resilience (feeling by bare hands), cohesiveness, surface smoothness and non-waviness, from the perspective of superiority or inferiority to a test hair bundle(s) uncoated with the sample.

The following four criteria were used for evaluation.

◎: Superior
○: Favorable
△: No change
×: Inferior

[Table 13]

| | Evaluation item | Working example B47 | Working example B48 | Working example B49 | Comparative example B13 | Comparative example B14 |
|---|---|---|---|---|---|---|
| | | Compound B11 | Compound B12 | Compound B23 | Compound B26 | Compound B27 |
| When washing | Finger-combing capability | ○~△ | ○~△ | ○~△ | △~× | △~× |
| After air drying | Elasticity / Resilience | ○ | ○~△ | ○ | △~× | △~× |
| | Cohesiveness of hair | ○~△ | ○~△ | ○~△ | △~× | △~× |
| | Smoothness of hair surface | ○~△ | ○~△ | ○~△ | △~× | △~× |
| | Suppression of hair waviness | ○ | ○ | ○ | △~× | △~× |

As can be seen from the results shown in Table 13, as compared to the comparative examples, the compounds B11, B12 and B23 of the working examples were superior in evaluation results on finger-combing capability at the time of performing washing, elasticity/resilience after performing air drying, cohesiveness, surface smoothness and non-waviness; it was confirmed that the treatment effects on the damaged hair were higher than those observed in the comparative examples. From these results, it was confirmed that a compound(s) composed of the salt structure of cations and anions, such as the salt structure of the working examples, were capable of exhibiting favorable effects as a hair treatment agent.

Further, as compared to the compound B12, the compounds B11 and B23 of the working examples brought about a superior elasticity/resilience. From these results, it was indicated that since the quaternary ammonium salt was a liquid and would not volatilize, it could evenly coat the surface of the hair and even infiltrate the inner portion thereof.

Further, as a result of performing a sensory evaluation(s) on the damaged hair by a method similar to that described above, using 2-amino-2-hydroxymethyl-1,3-propanediol oleate (compound B30), 2-amino-2-hydroxymethyl-1,3-propanediol linoleate (compound B31), 2-amino-2-hydroxymethyl-1,3-propanediol isostearate (compound B32), 2-amino-2-methyl-1,3-propanediol oleate (compound B33), 2-amino-2-methyl-1,3-propanediol linoleate (compound B34)

and 2-amino-2-methyl-1,3-propanediol isostearate (compound B35), the evaluation results were such that as compared to the compounds of the comparative examples, there were observed a superior elasticity/resilience, cohesiveness, surface smoothness and suppression of waviness, thus bringing about a favorable feeling and appearance.

From these results, it was indicated that even in the case of the damaged hair, the hair treatment agent of the present invention was capable of bringing about a favorable feeling and appearance by imparting to the hair a superior elasticity/resilience, cohesiveness, surface smoothness and suppression of waviness, after rinsing the hair.

3. Use of hair treatment composition on healthy and damaged hairs

3-1. Sensory evaluation on healthy hair after using shampoo agent containing organic ammonium salt

Preparation of hair treatment composition (shampoo agent)

As an example of the hair treatment composition using the hair treatment agent of the present invention, a shampoo agent below was produced.

After uniformly mixing a 0.5% by mass cationized hydroxyethyl cellulose (CATINAL HC-200 by TOHO Chemical Industry Co., Ltd.) with a purified water at 70 to 80°C, a POE (3) sodium laurylether sulfate (SPAMIN SA by Miyoshi Oil & Fat Co., Ltd.) and a coconut oil fatty acid amide propyl betaine (AMPHOREX CB-1 by Miyoshi Oil & Fat Co., Ltd.) were added thereto so that they would be in amounts of respectively 11.25% by mass (effective amount) and 3.75% by mass (effective amount), followed by adding thereto each of the compounds B11, B7, B23, B26, B27 and B28 so that the amount of the compound B11 would be 0.5, 3.0 and 10.0% by mass, and that the amounts of the compounds B7, B23, B26, B27 and B28 would be 3.0% by mass each (content in terms of anhydride in the cases of the compounds B7, B11 and B23), and then stirring the ingredients at 70 to 80°C for 20 min so as to uniformly mix them. Next, the mixture was cooled to a temperature of 30°C or lower, and a purified water of an evaporated amount was then added thereto so that the whole hair treatment composition would be 100% by mass, followed by uniformly mixing them so as to obtain a hair treatment composition(s). The pH level of the hair treatment composition was adjusted to pH 6.0 by adding a citric acid or sodium hydroxide.

Evaluation on healthy hair treated repeatedly

About 5 g of the healthy hair was taken and dipped into 350 ml of a seven-time dilute aqueous solution of each of the abovementioned hair treatment compositions (shampoo agents) at 35 to 40°C so as to be washed. The healthy hair was then subjected to a rinsing treatment five times using 500 ml of a warm water of 35 to 40°C, followed by drying the same at 80°C, and then performing air drying at 25°C. Such series of treatment involving hair washing, rinsing and drying was repeated 10 times to then evaluate a cohesiveness of the hair, a moist feeling of the hair, a gloss of the hair, a surface smoothness of the hair and a non-stickiness on the surface of the hair, after performing air drying.

The following four criteria were used for evaluation.

◎: Superior
○: Favorable
Δ: Slightly inferior
×: Inferior

Further, a scanning electron microscope (SEM) (JCM-5000 by JEOL Ltd.) was used to observe the surface of the hair after air drying. Photographs of the appearances of the hairs after air drying and SEM photographs of the surfaces of the hairs are shown in FIG.1.

[Table 14]

| | Evaluation item | Working example B50<br>Compound B11 added (3.0% by mass) | Working example B51<br>Compound B23 added (3.0% by mass) | Working example B52<br>Compound B7 added (3.0% by mass) | Working example B53<br>Compound B11 added (0.5% by mass) | Working example B54<br>Compound B11 added (10.0% by mass) |
|---|---|---|---|---|---|---|
| After air drying | Cohesiveness of hair | ○ | ◎ | ○ | ○ | ○ |
| | Moist feeling | ◎ | ◎ | ○ | ○~◎ | ◎ |
| | Gloss of hair | ◎ | ◎ | ○ | ○~◎ | ◎ |
| | Smoothness of hair surface | ◎ | ◎ | ○ | ◎ | ○~◎ |
| | Non-stickiness of hair surface | ◎ | ○ | ◎ | ◎ | ○ |

| | Evaluation item | Comparative example B15<br>Compound B26 added (3.0% by mass) | Comparative example B16<br>Compound B27 added (3.0% by mass) | Comparative example B17<br>Compound B28 added (3.0% by mass) |
|---|---|---|---|---|
| After air drying | Cohesiveness of hair | △ | × | △ |
| | Moist feeling | △ | × | △ |
| | Gloss of hair | △ | × | △ |
| | Smoothness of hair surface | △ | × | △ |
| | Non-stickiness of hair surface | △~○ | × | △ |

As can be seen from the results shown in Table 14, in the cases of the healthy hairs of the working examples B50 to B52 that had been treated with shampoo agents each containing 3.0% by mass of the compound B11, B23 or B7, there were observed a favorable cohesiveness, moist feeling and gloss of the hair, and the surface of the hair exhibited a favorable smoothness as being non-sticky. As for the appearance of the healthy hair that had been treated, while the hairs in the comparative examples spread and exhibited no cohesiveness, the hairs of the working examples were in a condition showing a favorable cohesiveness. As can be seen from the observation results by SEM, no cuticle peeling was observed on the surfaces of the hairs of the working examples B50 to B52 i.e. a smooth condition was confirmed (FIG.1). Further, the evaluation results were all favorable with regard to the healthy hairs of the working examples B53 and B54 that had been treated with shampoo agents respectively containing 0.5% by mass and 10% by mass of the compound B11. In contrast, in the case of the hair of the comparative example B16 that had been treated with a shampoo agent containing the compound B27 (ion-exchange water), the evaluation results were all inferior; in the cases of the comparative examples B15 and B17 using the compound B26 (glycerin) and the compound B28 (sodium lactate), the evaluation results tended to be inferior in general as compared to the working examples B50 to B52.

It was confirmed that favorable effects as a shampoo agent were exhibited by adding a compound(s) composed of the salt structure of cations and anions, such as the salt structure of the working examples. Further, since the compounds of the working examples exhibited favorable results in sensory evaluations as compared to the compound B28 (sodium lactate), it was indicated that an organic ammonium salt having a hydrogen-bonding functional group(s) in cations and anions and being a liquid at 25°C was able to bring about high effects.

In addition, as compared to the results shown in Table 12, since the evaluation items of moist feeling, gloss and smoothness turned "◎" (Superior) by compounding a shampoo agent containing the compound(s) of the working examples, it was confirmed that the product of the present invention was able to express higher treatment effects by interacting with a shampoo agent.

From these results, it was indicated that a hair treatment composition using the hair treatment agent of the present invention was able to impart a cohesiveness, moist feeling, gloss and smoothness to the hair and bring about a favorable feeling with no stickiness.

3-2. Sensory evaluation on damaged hair after using shampoo agent containing organic ammonium salt

Evaluation on damaged hair treated repeatedly

The damaged hair was repeatedly treated 10 times in a similar manner as 3-1; evaluated were a cohesiveness of the hair, surface smoothness of the hair and waviness of the hair after performing air drying.

[Table 15]

| | Evaluation item | Working example B55 | Working example B56 | Comparative example B18 | Comparative example B19 | Comparative example B20 |
|---|---|---|---|---|---|---|
| | | Compound B11 added (3.0% by mass) | Compound B23 added (3.0% by mass) | Compound B26 added (3.0% by mass) | Compound B27 added (3.0% by mass) | Compound B28 added (3.0% by mass) |
| After air drying | Cohesiveness of hair | ◎ | ◎ | ◎ | ◎ | ◎ |
| | Smoothness of hair surface | ◎ | ◎ | △ | ◎ | △ |
| | Suppression of hair waviness | ○ | ○ | ○ | △ | ○ |

As can be seen from the results shown in Table 15, in the cases of the damaged hairs of the working examples B55 and B56 that had been treated with shampoo agents respectively containing the compounds B11 and B23, there were observed a favorable cohesiveness and surface smoothness of the hair, and the hair showed no waviness. In contrast, in the case of the hair of the comparative example B19 that had been treated with a shampoo agent containing the compound B27 (ion-exchange water), the hair showed waviness; in the cases of the comparative examples B18 and B20 using the compound B26 (glycerin) and the compound B28 (sodium lactate), the hair exhibited an inferior surface smoothness.

From these results, it was indicated that even in the case of a damaged hair, a hair treatment composition using the hair treatment agent of the present invention was able to impart a cohesiveness of the hair, smoothness of the hair and suppression of hair waviness, thus bringing about a favorable feeling.

3-3. Sensory evaluation on healthy hair after using shampoo agent containing organic ammonium salt (evaluation using various anionic surfactants)

Preparation of hair treatment composition (shampoo agent)

After uniformly mixing a 0.5% by mass cationized hydroxyethyl cellulose (CATINAL HC-200 by TOHO Chemical Industry Co., Ltd.) with a purified water at 70 to 80°C, any one of the anionic surfactants shown in Table 16 and a coconut oil fatty acid amide propyl betaine (AMPHOREX CB-1 by Miyoshi Oil & Fat Co., Ltd.) were added thereto so that they would be in amounts of respectively 11.25% by mass (effective amount) and 3.75% by mass (effective amount), followed by adding thereto the compound B11 or B27 so that the amount thereof would be 3.0% by mass (content in terms of anhydride in the case of the compound B11), and then stirring the ingredients at 70 to 80°C for 20 min so as to uniformly mix them. Next, the mixture was cooled to a temperature of 30°C or lower, and a purified water of an evaporated amount was then added thereto so that the whole hair treatment composition would be 100% by mass, followed by uniformly mixing them so as to obtain a hair treatment composition(s). The pH level of the hair treatment composition was adjusted to pH 6.0 by adding a citric acid or sodium hydroxide.

With regard to the anionic surfactants, as lauroylmethyl-β-alanine sodium, ENAGICOL L-30AN (by Lion Corporation) was used; as cocoyl glutamate triethanolamine, AMISOFT CT-12S (by Ajinomoto Co., Inc.) was used; as sodium tetradecenesulfonate, LIPOLAN LJ-441 (by Lion Corporation) was used.

Evaluation on healthy hair treated repeatedly

About 5 g of the healthy hair was taken and dipped into 350 ml of a seven-time dilute aqueous solution of each of the abovementioned hair treatment compositions (shampoo agents) at 35 to 40°C so as to be washed. The healthy hair was then subjected to a rinsing treatment five times using 500 ml of a warm water of 35 to 40°C, followed by drying the same at 80°C, and then performing air drying at 25°C. Such series of treatment involving hair washing, rinsing and drying was repeated five times to then evaluate a gloss of the hair, softness of the hair and cohesiveness of the hair, after performing air drying. Photographs of the appearances of the hairs after air drying are shown in FIG.2.

[Table 16]

| | | Working example B57 | Comparative example B21 | Working example B58 | Comparative example B22 |
|---|---|---|---|---|---|
| | Anionic surfactant | Lauroylmethyl-β-alanine sodium | | Triethanolamine cocoyl glutamate | |
| | Evaluation item | Compound B11 added (3.0% by mass) | Compound B27 added (3.0% by mass) | Compound B11 added (3.0% by mass) | Compound B27 added (3.0% by mass) |
| After air drying | Gloss of hair | ◎ | △ | ◎ | △ |
| | Flexibility of hair | ◎ | △ | ○ | △ |
| | Cohesiveness of hair | ◎ | △ | ◎ | △ |

| | | Working example B59 | Comparative example B23 |
|---|---|---|---|
| | Anionic surfactant | Sodium tetradecene sulfonate | |
| | Evaluation item | Compound B11 added (3.0% by mass) | Compound B27 added (3.0% by mass) |
| After air drying | Gloss of hair | ○ | △ |
| | Flexibility of hair | ○ | △ |
| | Cohesiveness of hair | ○ | △ |

As can be seen from the results shown in Table 16, in the cases of the healthy hairs of the working examples B57 to B59 that had been treated with shampoo agents containing the compound B11, there were observed a favorable gloss, softness and cohesiveness of the hair. In contrast, in the cases of the hairs of the comparative examples B21 to B23 that had been treated with shampoo agents containing the compound B27 (ion-exchange water), there were observed a slightly inferior gloss, softness and cohesiveness of the hair.

In addition, as compared to the results shown in Table 12, since any one of the evaluation items of gloss, softness and cohesiveness turned "◎" (Superior) in the working examples B57 and B58 by compounding a shampoo composition containing the compound(s) of the working examples and various anionic surfactants, it was confirmed that the product of the present invention was able to express higher treatment effects by interacting with a shampoo composition containing the various anionic surfactants shown in Table 16.

3-4. Sensory evaluation on healthy hair after using conditioner containing organic ammonium salt

Preparation of hair treatment composition (conditioner)

Here, 4.5% by mass of a stearyl alcohol (KALCOL 8688 by Kao Corporation), 2.0% by mass of a behenyl alcohol (KALCOL 220-80 by Kao Corporation) and 0.5% by mass of a phenoxyethanol (Hisolve EPH by TOHO Chemical Industry Co., Ltd.) were mixed together and heated to 75°C to obtain a liquid A. Further, 3.0% by mass of a behentrimonium chloride (CATINAL DC-80 by TOHO Chemical Industry Co., Ltd.), 5.0% by mass of dipropylene glycol and 3.0% by mass of each of the compounds B11, B23, B26, B27 and B28 (content in terms of anhydride in the cases of the compounds B11 and B23) were added together, followed by adding a purified water thereto, and then melting them at 80°C, thus obtaining a liquid B. The liquid B was then gradually added to the liquid A, and stirring was performed at 75°C for 20 min. Next, the mixture was cooled to a temperature of 40°C or lower, and a purified water of an evaporated amount was then added thereto so that the whole hair treatment composition would be 100% by mass, followed by uniformly mixing them so as to obtain a hair treatment composition(s).

Evaluation on healthy hair after being immersed in conditioner

About 5 g of the healthy hair was dipped into and kept immersed in 150 g of a seven-time dilute aqueous solution of each of the abovementioned hair treatment compositions (conditioners) at 40°C for 30 min. The healthy hair was then subjected to a rinsing treatment twice using 500 ml of a warm water of 35 to 40°C, followed by drying the same at 80°C, and then performing air drying at 25°C. After air drying, a combing capability, cohesiveness and non-stickiness of the hair were evaluated. Photographs of the appearances of the hairs after air drying are shown in FIG.3.

[Table 17]

| | Evaluation item | Working example B60 | Working example B61 | Comparative example B24 | Comparative example B25 | Comparative example B26 |
|---|---|---|---|---|---|---|
| | | Compound B11 added (3.0% by mass) | Compound B23 added (3.0% by mass) | Compound B26 added (3.0% by mass) | Compound B27 added (3.0% by mass) | Compound B28 added (3.0% by mass) |
| After air drying | Combing capability of hair | ◎ | ◎ | ○ | △ | ○ |
| | Cohesiveness of hair | ○ | ◎ | △ | × | △ |
| | Non-stickiness of hair surface | ○ | ○ | △ | ◎ | × |

As can be seen from the results shown in Table 17 and FIG.3, in the cases of the healthy hairs of the working examples B60 and B61 that had been treated with conditioners containing the compounds B11 and B23, there were observed a favorable combing capability and cohesiveness of the hair, and no hair stickiness was felt. In contrast, in the case of the hair of the comparative example B25 that had been treated with a conditioner containing the compound B27 (ion-exchange water), a slightly poor combining capability of the hair was observed, and the hair cohesiveness was inferior. In the case of the comparative example B24 using the compound B26 (glycerin), the cohesiveness and stickiness of the hair were slightly inferior. In the case of the comparative example B26 using the compound B28 (sodium lactate), a poor hair cohesiveness was observed, and a hair stickiness was felt.

From these results, it was confirmed that favorable effects as a conditioner were exhibited by adding a compound(s) composed of the salt structure of cations and anions, such as the salt structure of the working examples. Further, since the compounds of the working examples exhibited favorable results in sensory evaluations as compared to the compound B28 (sodium lactate), it was indicated that an organic ammonium salt having a hydrogen-bonding functional group(s) in cations and anions and being a liquid at 25°C was able to bring about high effects.

4. Evaluation on adherability to and stabilization of protein (keratin) of hair and skin

4-1. Keratin adherability

Here, 1 g of a powdery keratin (by Tokyo Chemical Industry Co., Ltd.) (pre-test keratin) was added to 2 g of a 50 wt% aqueous solution of each of the compounds B11, B23, B26 and B27, followed by performing stirring at 25°C for 24 hours. After stirring, filtration was performed, and a powdery keratin obtained was then dried so as to obtain a keratin that had been treated (post-test keratin).

A rate of compound adhesion to keratin was calculated with the following formula.

$$\text{Compound adhesion rate (\%)} = [(\text{post-test keratin weight (g)} - \text{pre-test keratin weight (g)}) / \text{pre-test keratin weight (g)}] \times 100$$

As can be seen from the results shown in Table 18, while adhesion was not observed in a comparative example where an ion-exchange water was used in the compound B27, and while the adhesion rate was 11.0% in the case of the compound B26 using glycerin, the compounds of the working examples exhibited adhesion rates of 13.2 to 17.0% i.e. it was indicated that the cation structure as well as the hydrogen-bonding functional group(s) of the hair treatment agent of the present invention were able to bring about a superior adherability to a protein(s) (keratin) of the hair and skin such as cuticles and nails.

4-2. Evaluation on stabilization effect of hair treatment agent on keratin

Here, 0.3 g of the keratin that had been treated in 4-1 was placed into a thermostatic device of 130°C and left to stand still therein for seven days. After seven days, an IR spectrum of the keratin obtained was measured; by measuring

an absorption derived from the α-helix secondary structure of amide, a stabilization effect of the hair treatment agent on the structure of keratin was evaluated.

As a result of measuring the powder of the keratin before heating by IR, absorption was observed at 1654 $cm^{-1}$ which was derived from the α-helix secondary structure of amide. Next, IR measurement was carried out after performing the heating test at 130°C to obtain peaks derived from each amide and an intensity ratio(s) thereof to a reference peak (reference peak (derived from C-N) 1086 $cm^{-1}$, peak 1654 $cm^{-1}$ derived from the α-helix secondary structure of amide). The intensity ratio (X) was evaluated as "intensity of reference peak : intensity of peak derived from amide = 1 : X," by reading the absorption intensity of the reference peak and the intensity of the peak derived from amide.

As can be seen from the results shown in Table 18, in the case of a comparative example where an ion-exchange water was used in the compound B27, it was confirmed that the peak derived from the α-helix secondary structure of amide had disappeared i.e. the α-helix secondary structure of keratin failed to be maintained. In contrast, the α-helix secondary structure of keratin was confirmed to have been maintained in the cases of the compounds B11 and B23 of the working examples and the compound B26 using glycerin, because a peak(s) derived from the α-helix secondary structure of amide were observed. However, in the case of the compound B26 of the comparative example using glycerin, the intensity ratio of the peak derived from the α-helix secondary structure of amide to the reference peak was 0.3. In this respect, in the cases of the compounds of the working examples, the intensity ratio(s) of the peak derived from the α-helix secondary structure of amide were as high as 0.6 to 0.9; there was indicated a high effect in stabilizing the α-helix secondary structure of keratin. In this way, since the hair treatment agent of the present invention has an effect of stabilizing keratin, there can be realized a water and moisture retention of the hair, health and quality maintenance of the hair, suppression of hair damages caused by heat from a dryer or the like, and even, for example, water and moisture retention of skin proteins such as cuticles and nails as well as health maintenance thereof.

[Table 18]

| | Compound | Compound adhesion rate | Presence of amide peak (Peak wavelength) | Intensity ratio to reference peak (X) |
|---|---|---|---|---|
| Working example B62 | B11 | 13.2% | Present (1654$cm^{-1}$) | 0.6 |
| Working example B63 | B23 | 17.0% | Present (1654$cm^{-1}$) | 0.9 |
| Comparative example B27 | B26 | 11.0% | Present (1654$cm^{-1}$) | 0.3 |
| Comparative example B28 | B27 | 0.0% | None | 0 |

5. Evaluation on solubility of active ingredient

Solubilities of active ingredients were evaluated with regard to the compositions shown in Table 19. As the active ingredients, there were used a poorly-soluble gallic acid having an antioxidant effect, and a glutamic acid having a moisture retention effect. While the solubility was <0.08 g in the cases of the comparative examples B29 (glycerin aqueous solution) and B30 (ion-exchange water) in which the glutamic acid had been dissolved, the working example B64 exhibited a high solubility of 0.24 g. Further, similar tendencies were observed with regard to the gallic acid; the compound B11 was able to dissolve a larger amount of the active ingredient. In this sense, the hair treatment agent of the present invention can be used as a base agent or solvent of a hair treatment composition and as a carrier toward the inner portion of the hair.

[Table 19]

| | | Working example B64 | Comparative example B29 | Comparative example B30 | Working example B65 | Comparative example B31 | Comparative example B32 |
|---|---|---|---|---|---|---|---|
| Composition | Compound B11 (g) | 1.8 | | | 1.8 | | |
| | Compound B26(g) | | 1.8 | | | 1.8 | |
| | Compound B27(g) | 6.2 | 6.2 | 8 | 6.2 | 6.2 | 8 |
| Solubility | Glutamic acid (g) | 0.24 | <0.08 | <0.08 | | | |
| | Gallic acid (g) | | | | 0.32 | 0.08 | 0.08 |

6. Evaluation on affinity to hair (contact angle)

A hair(s) was dipped into and kept immersed in a 50 wt% aqueous solution of each of the compounds B11, B12, B29 and B24 listed in Table 20 for 5 min, followed by once rinsing the hair with a warm water, and then drying the same with a towel. The hair thus treated and a hair as an untreated product were then dried with a dryer (about 100°C) (condition A), followed by dropping 10 μL of water onto each type of hair so as to measure the contact angle of a droplet(s) thereon. The contact angle measurement was performed using Drop Shape Analyzer DSA30 (by KRUSS GmbH); and a measurement method was such that 20 treated hairs were fixed to a glass slide with Scotch Tape (registered trademark) in a way such that the hairs were evenly and flatly arranged thereon, followed by dropping 10 μL of water thereonto from above, and then measuring the contact angle of a droplet(s) thereon. Further, a hair treated in a similar manner as above using a 50 wt% aqueous solution of each of the compounds B11, B12, B29, B24 and B26; and a hair as an untreated product were dried with a hair iron of a temperature (160°C) higher than that of a dryer (condition B), followed by measuring the contact angles under a similar condition(s) as above.

Under the condition A, as compared to the comparative examples B33 and B34, each of the compounds of the working examples B66 to B68 resulted in a small contact angle and was thus confirmed to have an excellent affinity to the hair. Particularly, the compound B11 of the working example B66 exhibited an affinity so high that it infiltrated the hair, thus exerting an effect in retaining the moisture of the hair and maintaining the health and quality thereof.

Under the condition B, as compared to the comparative examples B35 to B37, each of the compounds of the working examples B69 to B71 also resulted in a small contact angle and was thus confirmed to have an excellent affinity to the hair. Particularly, while the compounds B11 and B12 of the working examples B69 and B70 exhibited an affinity so high that they infiltrated the hair, the comparative example B36 brought about a result not indifferent from that of the untreated product as the glycerin had volatilized. From these results, it was confirmed that due to the non-volatile organic ammonium salt, the compound(s) had remained even under the condition B as a heating condition, and were able to exert the effect in retaining the moisture of the hair and maintaining the health and quality thereof.

[Table 20A]

| | Compound | 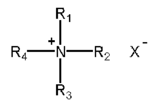 | | | | | Compund concentration (%) | Condition | Contact angle immediately after delivering droplet on hair (°) |
|---|---|---|---|---|---|---|---|---|---|
| | | $R_1$ | $R_2$ | $R_3$ | $R_4$ | X— | | | |
| Working example B66 | B11 | 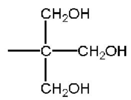 | H | H | H | $CH_3CH(OH)COO^-$ | 50 | A | (Soaked) |
| Working example B67 | B12 | | H | H | H | $HOCH_2COO^-$ | 50 | | 115 |
| Working example B68 | B29 | | H | H | H | $Cl^-$ | 50 | | 115 |
| Comparative example B33 | — | Untreated | | | | | — | | 117 |
| Comparative example B34 | B24 | TBAB | | | | | 50 | | 121 |

[Table 20B]

| | Compound | 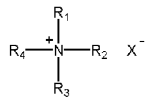 | | | | | Compound concentration (%) | Condition | Contact angle immediately after delivering droplet on hair (°) |
|---|---|---|---|---|---|---|---|---|---|
| | | $R_1$ | $R_2$ | $R_3$ | $R_4$ | X— | | | |
| Working example B69 | B11 | 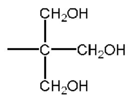 | H | H | H | $CH_3CH(OH)COO^-$ | 50 | B | — (Soaked) |
| Working example B70 | B12 | | H | H | H | $HOCH_2COO^-$ | 50 | | (Soaked) |
| Working example B71 | B29 | | H | H | H | $Cl^-$ | 50 | | 105 |
| Comparative example B35 | - | Untreated | | | | | - | | 108 |
| Comparative example B36 | B26 | Glycerin | | | | | 50 | | 108 |
| Comparative example B37 | B24 | TBAB | | | | | 50 | | 119 |

In this way, it was indicated that the organic ammonium salt of the present invention was superior in affinity to the hair, capable of exerting its effects without volatilizing even after performing heating with a dryer or the like, and thus causing less damage to the cuticle.

7. Evaluation on stability of protein (DSC)

A hair(s) was dipped into and kept immersed in a 50 wt% aqueous solution of each of the compounds B11, B12, B29 and B26 for 5 min, followed by once rinsing the hair with a warm water, and then drying the same with a towel. The hair thus treated and a hair as an untreated product were then dried with a dryer (about 100°C) (condition A). Further, a hair treated in a similar manner as above and a hair as an untreated product were dried with a hair iron of a temperature (160°C) higher than that of a dryer (condition B). Thus, samples of each type of hair were prepared. Each hair sample was shredded into a powder of which 5 mg was then accurately weighed and put into a SUS container. The container was then sealed after adding 10 μL of a distilled water thereinto, and was left for two days to sufficiently soak the hair sample with water. This sample was then subjected to a DSC measurement under a condition(s) of: nitrogen flow 60 ml/min; temperature range 30 to 210°C; rate of temperature rise 10°C/min, to calculate an endothermic peak area. The endothermic peak area (ΔH) is a difference obtained by subtracting a post-treatment endothermic peak area from an endothermic peak area of the untreated product. The evaluation results indicate that the smaller a difference from a numerical value before performing treatment under the condition A or B (endothermic peak area 34.5 (J/g-hair)) was, the smaller a change would be in the cross-linking strength of keratin protein as a main hair component under the microfibril state in the inner portion of the hair, and the lesser the degree of the damage to the hair would be accordingly.

As compared to the endothermic peak areas in the comparative examples B38 and B39, the peak areas in the working examples B72 to B77 were confirmed to be closer to the value before treatment; the results indicated that less damage was caused to the hair in the working examples. The peak areas in the working examples B72 and B75 failed to be calculated because the peak derived from the compound(s) and the endothermic peak overlapped. From the values of the endothermic peak areas (ΔH), it was confirmed that as compared to the comparative example B38 using glycerin, the organic ammonium salt of the present invention having the salt structure was superior in damage suppression effect, and that even among such organic ammonium salts, those having a hydroxy group(s) in anions were more preferred. In this sense, it was indicated that in the case of the hair treatment agent of the present invention, a texture after performing the hair treatment could be maintained, because the hydrogen-bonding functional group-containing organic ammonium salt would interact with the proteins in the hair so that it became possible to inhibit protein denaturation by maintaining the secondary structure and inhibiting bond breaking. Further, it was also indicated that there could be brought about an effect of stabilizing the proteins of the scalp.

[Table 21]

| | Compound | 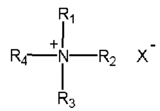 | | | | | Condition at 25°C | Compound concentration (%) | Condition | Endothermic peak area ⊿H (J/g-hair) |
|---|---|---|---|---|---|---|---|---|---|---|
| | | $R_1$ | $R_2$ | $R_3$ | $R_4$ | $X^-$ | | | | |
| Working example B72 | B11 | 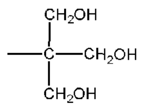 | H | H | H | $CH_3CH(OH)COO^-$ | Liquid | 50 | (A) | — |
| Working example B73 | B12 | | H | H | H | $HOCH_2COO^-$ | Solid | 50 | | 4.6 |
| Working example B74 | B29 | | H | H | H | $Cl^-$ | Solid | 50 | | 11.2 |
| Comparative example B38 | B26 | Glycerin | | | | | Liquid | 50 | | 21.0 |
| Working example B75 | B11 | 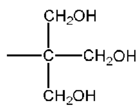 | H | H | H | $CH_3CH(OH)COO^-$ | Liquid | 50 | (B) | — |
| Working example B76 | B12 | | H | H | H | $HOCH_2COO^-$ | Solid | 50 | | 7.1 |
| Working example B77 | B29 | | H | H | H | $Cl^-$ | Solid | 50 | | 8.8 |
| Comparative example B39 | B26 | Glycerin | | | | | Liquid | 50 | | 17.7 |

[3] Evaluation of skin care agent

(Compound)

Compounds C1 to C101

Compounds C1 to C101 shown in Tables 22 to 41 were synthesized and obtained by the following method(s).

Compounds C1 to C5 were synthesized by a method described in JP-A-2014-131975.

Compounds C6 to C26 were synthesized by a method described in JP-A-2014-131974.

Compounds C27 to C39 were synthesized by a method described in JP-A-2012-031137.

Compounds C40 to C61, and C65 to C95 were synthesized by a method described in JP-A-2018-136893.

Compound C62: A reagent (tetrabutylammonium bromide) produced by FUJIFILM Wako Pure Chemical Corporation was used.

Compound C63: A reagent (1-butyl-3-methylimidazolium tetrafluoroborate) produced by Tokyo Chemical Industry Co., Ltd. was used.

Compound C64: A reagent (glycerin) produced by FUJIFILM Wako Pure Chemical Corporation was used.

Compound C96: A reagent (sodium lactate) produced by FUJIFILM Wako Pure Chemical Corporation was used.

Compound C97: A reagent (potassium lactate) produced by FUJIFILM Wako Pure Chemical Corporation was used.

Compound C98: A reagent (ascorbic acid) produced by Tokyo Chemical Industry Co., Ltd. was used.

Compound C99: A reagent (ascorbic acid glucoside) produced by Tokyo Chemical Industry Co., Ltd. was used.

Compound C100: A reagent (sodium ascorbyl phosphate) produced by Tokyo Chemical Industry Co., Ltd. was used.

Compound C101: Sodium ascorbyl phosphate was neutralized in hydrochloric acid; after desalting, the ascorbyl phosphate obtained was neutralized with 2-amino-2-hydroxymethyl-1,3-propanediol to obtain the compound.

Here, among the organic ammonium salts of the present invention in the working examples shown in Tables 22 to 42, compounds other than C25 to C39 and C57 to C59 were prepared by the preparation method B described in 1-3. of [2] Evaluation of hair treatment agent.

(Evaluation method)

(1) Water retention property test 1

An 80 wt% aqueous solution of each of the compounds C1 to C61 and the compounds C62 to C64 of the comparative examples was prepared, followed by using a Karl Fischer moisture meter (CA-200 by Mitsubishi Chemical Analytech Co., Ltd.) to confirm that a moisture rate was 20.0 wt% (pre-test moisture rate: A). Next, 1.0 g of each of these samples was added to a screw tube, and this screw tube, while remaining unsealed, was then left to stand still for 24 hours in a thermo-hygrostat (KCL-2000W by Tokyo Rikakikai Co., Ltd.) set to 40°C and 25%RH. A moisture rate after 24 hours was again measured (post-test moisture rate: B), followed by using the formula below to calculate a rate of reduction in moisture so as to evaluate the water retention property.

Pre-test moisture rate: A (%)

Post-test moisture rate: B (%)

$$\text{Rate of reduction in moisture (\%)} = [(A\,(\%) - B\,(\%))\,/\,A\,(\%)] \times 100$$

[Table 22]

| | Compound | 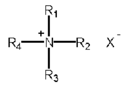 | | | | | Presence of hydration water | n-hydrate | Appearance at 25°C | | Freezing point | Water retention property test 1 80 wt% aqueous solution | | |
|---|---|---|---|---|---|---|---|---|---|---|---|---|---|---|
| | | $R_1$ | $R_2$ | $R_3$ | $R_4$ | $X^-$ | | | Hydrate | Anhydride | Hydrate | Pre-test moisture rate | Post-test moisture rate | Rate of reduction in moisture |
| Working example C1 | C1 | $(CH_2)_2OH$ | H | H | H | $CH_3CH(OH)COO^-$ | Present | Monohydrate | Liquid | Liquid | <-10 | 20.0% | 19.3% | 3.5% |
| Working example C2 | C2 | $(CH_2)_2OH$ | $(CH_2)_2OH$ | H | H | $CH_3CH(OH)COO^-$ | Present | Dihydrate | Liquid | Liquid | <-10 | 20.0% | 19.0% | 4.8% |
| Working example C3 | C3 | | | | | $HOCH_2COO$ | Present | Dihydrate | Liquid | Liquid | <-10 | 20.0% | 19.2% | 4.0% |
| Working example C4 | C4 | | | | | $HOOC(CH_2)_2COO^-$ | Present | Dihydrate | Liquid | Liquid | <-10 | 20.0% | 18.6% | 7.2% |
| Working example C5 | C5 | $(CH_2)_2OH$ | $(CH_2)_2OH$ | $(CH_2)_2OH$ | H | $CH_3CH(OH)COO^-$ | Present | Trihydrate | Liquid | Liquid | <-10 | 20.0% | 19.2% | 3.9% |
| Working example C6 | C6 | 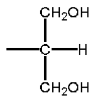 | H | H | H | $CH_3COO^-$ | Present | Dihydrate | Liquid | Liquid | <-10 | 20.0% | 19.3% | 3.5% |
| Working example C7 | C7 | | | | | $HOCH_2COO$ | Present | Dihydrate | Liquid | Liquid | <-10 | 20.0% | 19.6% | 2.1% |
| Working example C8 | C8 | | | | | $CH_3CH(OH)COO^-$ | Present | Dihydrate | Liquid | Liquid | <-10 | 20.0% | 19.2% | 4.0% |
| Working example C9 | C9 | | | | | $HOOC(CH_2)_2COO^-$ | Present | Dihydrate | Liquid | Liquid | <-10 | 20.0% | 18.9% | 5.6% |
| Working example C10 | C10 | | | | | $C(OH)(CH_2COOH)_2COO^-$ | Present | Dihydrate | Liquid | Liquid | <-10 | 20.0% | 18.9% | 5.6% |
| Working example C11 | C11 | | | | | $CH_3SO_3$ | Present | Dihydrate | Liquid | Liquid | <-10 | 20.0% | 19.9% | 0.5% |
| Working example C12 | C12 | | | | | $BF_4^-$ | Present | Dihydrate | Liquid | Liquid | <-5 | 20.0% | 18.8% | 5.8% |
| Working example C13 | C13 | | | | | $Br^-$ | Present | Dihydrate | Liquid | Liquid | <-10 | 20.0% | 18.0% | 9.8% |
| Working example C14 | C14 | | | | | $Cl^-$ | Present | Dihydrate | Liquid | Liquid | <-10 | 20.0% | 18.0% | 9.9% |
| Working example C15 | C15 | 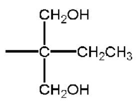 | H | H | H | $CH_3CH(OH)COO^-$ | Present | Dihydrate | Liquid | Liquid | <-10 | 20.0% | 19.2% | 4.0% |
| Working example C16 | C16 | | | | | $C(OH)(CH_2COOH)_2COO^-$ | Present | Dihydrate | Liquid | Liquid | <-10 | 20.0% | 19.0% | 5.0% |
| Working example C17 | C17 | | | | | $CH_3SO_3^-$ | Present | Dihydrate | Liquid | Liquid | <-10 | 20.0% | 19.8% | 1.0% |
| Working example C18 | C18 | | | | | $Br^-$ | Present | Dihydrate | Liquid | Liquid | <-10 | 20.0% | 18.2% | 9.0% |
| Working example C19 | C19 | | | | | $Cl^-$ | Present | Dihydrate | Liquid | Liquid | <-10 | 20.0% | 18.1% | 9.5% |

[Table 23]

| | Compound | 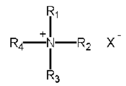 | | | | | Presence of hydration water | n-hydrate | Appearance at 25°C | | Freezing point | Water retention property test 1 80 wt% aqueous solution | | |
|---|---|---|---|---|---|---|---|---|---|---|---|---|---|---|
| | | $R_1$ | $R_2$ | $R_3$ | $R_4$ | $X^-$ | | | Hydrate | An-hydride | Hydrate | Pre-test moisture rate | Post-test moisture rate | Rate of reduction in moisture |
| Working example C20 | C20 | 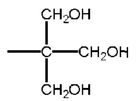 | H | H | H | $CH_3CH(OH)COO^-$ | Present | Dihydrate | Liquid | Liquid | <-10 | 20.0% | 19.5% | 2.7% |
| Working example C21 | C21 | | | | | $C(OH)(CH_2COOH)_2COO^-$ | Present | Dihydrate | Liquid | Liquid | <-10 | 20.0% | 19.0% | 5.1% |
| Working example C22 | C22 | | | | | $CH_3SO_3^-$ | Present | Dihydrate | Liquid | Liquid | <-10 | 20.0% | 19.8% | 1.2% |
| Working example C23 | C23 | 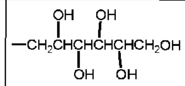 | H | H | H | $CH_3CH(OH)COO^-$ | Present | Dihydrate | Liquid | Liquid | <-10 | 20.0% | 19.3% | 3.4% |
| Working example C24 | C24 | H | H | H | H | $CH_3CH(OH)COO^-$ | Present | Monohydrate | Solid | Solid | ≥25 | 20.0% | 19.0% | 4.9% |
| Working example C25 | C25 | 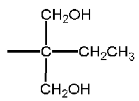 | 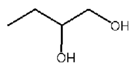 | 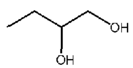 | 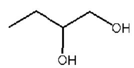 | $CH_3CH(OH)COO^-$ | Present | Dihydrate | Liquid | Liquid | <-5 | 20.0% | 19.0% | 5.0% |
| Working example C26 | C26 | $(CH_2)_2OH$ | $(CH_2)_2OH$ | $(CH_2)_2OH$ | $(CH_2)_2OH$ | $CH_3CH(OH)COO^-$ | Present | Dihydrate | Liquid | Liquid | <-5 | 20.0% | 19.0% | 5.0% |
| Working example C27 | C27 | $CH_3$ | $CH_3$ | $CH_3$ | $(CH_2)_2OH$ | $CH_3COO^-$ | Present | Monohydrate | Solid | Solid | ≥25 | 20.0% | 19.0% | 5.0% |
| Working example C28 | C28 | | | | | $HOCH_2COO^-$ | Present | Monohydrate | Solid | Solid | ≥25 | 20.0% | 19.4% | 3.0% |
| Working example C29 | C29 | | | | | $CH_3CH(OH)COO^-$ | Present | Monohydrate | Liquid | Liquid | <-10 | 20.0% | 19.0% | 5.1% |
| Working example C30 | C30 | | | | | $HOOCCH=CHCOO^-$ | Present | Monohydrate | Solid | Solid | ≥25 | 20.0% | 19.0% | 5.5% |
| Working example C31 | C31 | | | | | $HOOC(CH_2)_2COO^-$ | Present | Monohydrate | Liquid | Liquid | <-10 | 20.0% | 19.0% | 5.5% |
| Working example C32 | C32 | | | | | $CH_3SO_3^-$ | Present | Monohydrate | Solid | Solid | ≥25 | 20.0% | 19.6% | 2.2% |
| Working example C33 | C33 | | | | | $H_2PO_4^-$ | Present | Monohydrate | Solid | Solid | ≥25 | 20.0% | 19.4% | 3.0% |
| Working example C34 | C34 | | | | | $H_2PO_2^-$ | Present | Monohydrate | Liquid | Liquid | <-10 | 20.0% | 19.4% | 3.0% |
| Working example C35 | C35 | | | | | $Cl^-$ | Present | Monohydrate | Solid | Solid | ≥25 | 20.0% | 18.8% | 6.0% |

[Table 24A]

| | Compound | 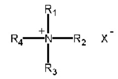 | | | | | Compounding molar ratio (acid:base) | Presence of hydration water | n-hydrate | Appearance at 25°C | | Freezing point | | Water retention property test 1 80 wt% aqueous solution | | |
|---|---|---|---|---|---|---|---|---|---|---|---|---|---|---|---|---|
| | | $R_1$ | $R_2$ | $R_3$ | $R_4$ | X | | | | Hydrate | An-hydride | Hydrate | An-hydride | Pre-test moisture rate | Post-test moisture rate | Rate of reduction in moisture |
| Working example C36 | C36 | $CH_3$ | $CH_3$ | $CH_3CH_2$ | $(CH_2)_2OH$ | $CH_3SO_3^-$ | 1:1 | Present | Mono-hydrate | Liquid | Liquid | <-10 | – | 20.0% | 19.6% | 2.0% |
| Working example C37 | C37 | $CH_2CH(OH)CH_2OH$ | $CH_3$ | $CH_3$ | $(CH_2)_2OH$ | $Cl^-$ | 1:1 | Present | Di-hydrate | Liquid | Liquid | <-10 | – | 20.0% | 18.3% | 8.3% |
| Working example C38 | C38 | $CH_2CH(OH)CH_2-N^+(CH_3)_2(CH_2CH_2OH)$ | $CH_3$ | $CH_3$ | $(CH_2)_2OH$ | Cl | 1:1 | Present | Di-hydrate | Solid | Solid | ≥25 | – | 20.0% | 18.1% | 9.5% |
| Working example C39 | C39 | $CH_3(CH_2)_8$ | $CH_3(CH_2)_8$ | $CH_3(CH_2)_8$ | $CH_3(CH_2)_3$ | $CH_3CH(OH)COO$ | 1:1 | None | — | — | Solid | – | ≥25 | 20.0% | 18.0% | 9.9% |
| Working example C40 | C40 | $(CH_2)_2OH$ | H | H | H | Ascorbic acid anion | 1:1 | Present | Di-hydrate | Solid | Solid | ≥25 | – | 20.0% | 19.5% | 2.3% |
| Working example C41 | C41 | $(CH_2)_2OH$ | $(CH_2)_2OH$ | H | H | Ascorbic acid anion | 1:1 | Present | Di-hydrate | Liquid | Liquid | <-10 | – | 20.0% | 19.2% | 4.1% |
| Working example C42 | C42 | $(CH_2)_2OH$ | $(CH_2)_2OH$ | $(CH_2)_2OH$ | H | Ascorbic acid anion | 1:1 | Present | Di-hydrate | Liquid | Liquid | <-10 | – | 20.0% | 19.1% | 4.4% |
| Working example C43 | C43 | 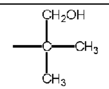 | H | H | H | $CH_3CH(OH)COO^-$ | 1:1 | Present | Di-hydrate | Liquid | Liquid | <-10 | – | 20.0% | 19.3% | 4.4% |
| Working example C44 | C44 | 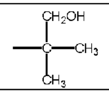 | H | H | H | Ascorbic acid anion | 1:1 | Present | Di-hydrate | Liquid | Liquid | <-10 | – | 20.0% | 19.2% | 4.7% |
| Working example C45 | C45 | 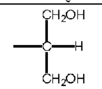 | H | H | H | Ascorbic acid anion | 1:1 | Present | Di-hydrate | Solid | Solid | ≥25 | – | 20.0% | 19.5% | 2.5% |
| Working example C46 | C46 | 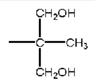 | H | H | H | $CH_3CH(OH)COO$ | 1:1 | Present | Di-hydrate | Liquid | Liquid | <-10 | – | 20.0% | 19.2% | 4.7% |
| Working example C47 | C47 | 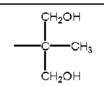 | H | H | H | Ascorbic acid anion | 1:1 | Present | Di-hydrate | Solid | Solid | ≥25 | – | 20.0% | 19.2% | 4.7% |
| Working example C48 | C48 | 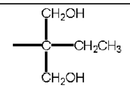 | H | H | H | Ascorbic acid anion | 1:1 | Present | Di-hydrate | Solid | Solid | ≥25 | – | 20.0% | 19.4% | 2.9% |
| Working example C49 | C49 | 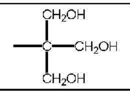 | H | H | H | Ascorbic acid anion | 1:1 | Present | Di-hydrate | Solid | Solid | ≥25 | – | 20.0% | 19.4% | 3.0% |

[Table 24B]

| | Compound | $R_4-\overset{R_1}{\underset{R_3}{N^+}}-R_2\ X^-$ R1 | R2 | R3 | R4 | $X^-$ | Compounding molar ratio (acid:base) | Presence of hydration water | n-hydrate | Appearance at 25°C Hydrate | Appearance at 25°C An-hydride | Freezing point Hydrate | Freezing point An-hydride | Water retention property test 1 80 wt% aqueous solution Pre-test moisture rate | Post-test moisture rate | Rate of reduction in moisture |
|---|---|---|---|---|---|---|---|---|---|---|---|---|---|---|---|---|
| Working example C50 | C50 | 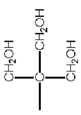 | H | H | H | $HOCH_2COO^-$ | 1:1 | Present | Di-hydrate | Solid | Solid | ≥25 | — | 20.0% | 19.1% | 4.4% |
| Working example C51 | C51 | 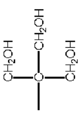 | H | H | H | $HOOCCH_2CH(OH)COO$ | 1:1 | Present | Di-hydrate | Liquid | Liquid | <-10 | — | 20.0% | 19.1% | 4.5% |
| Working example C52 | C52 | 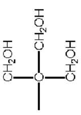 | H | H | H | $OOCCH_2CH(OH)COO$ | 1:2 | Present | Di-hydrate | Liquid | Liquid | <-10 | — | 20.0% | 18.6% | 7.1% |
| Working example C53 | C53 | 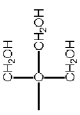 | H | H | H | $CH_3COCH_2CH_2COOH^-$ | 1:1 | Present | Di-hydrate | Liquid | Liquid | <-10 | — | 20.0% | 19.1% | 4.4% |
| Working example C54 | C54 | 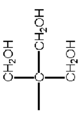 | H | H | H | $HOOCCH_2CH(NH_2)COO^-$ | 1:1 | Present | Di-hydrate | Liquid | Liquid | <-10 | — | 20.0% | 18.8% | 5.8% |
| Working example C55 | C55 | 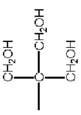 | H | H | H | $^-OOC(CH_2)_2CH(COO^-)NHCOCH_3$ | 1:2 | Present | Di-hydrate | Solid | Solid | ≥25 | — | 20.0% | 18.4% | 7.9% |
| Working example C56 | C56 | 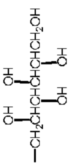 | H | H | H | Ascorbic acid anion | 1:1 | Present | Di-hydrate | Solid | Solid | ≥25 | — | 20.0% | 19.7% | 1.7% |
| Working example C57 | C57 | $CH_3$ | $CH_3$ | $CH_3$ | $(CH_2)_2OH$ | Ascorbic acid anion | 1:1 | Present | Di-hydrate | Solid | Solid | ≥25 | — | 20.0% | 19.0% | 5.0% |
| Working example C58 | C58 | $CH_3$ | $CH_3$ | $CH_3$ | $CH_2CH(OH)CH_2COOH$ | Ascorbic acid anion | 1:1 | Present | Di-hydrate | Liquid | Liquid | <-10 | — | 20.0% | 19.2% | 4.1% |
| Working example C59 | C59 | $CH_3$ | $CH_3$ | $CH_3$ | $(CH_2)_2OC(=O)CH_3$ | Ascorbic acid anion | 1:1 | Present | Di-hydrate | Liquid | Liquid | <-10 | — | 20.0% | 19.3% | 3.5% |
| Working example C60 | C60 | $(CH_2)_3COOH$ | H | H | H | $CH_3CH(OH)COO^-$ | 1:1 | Present | Mono-hydrate | Liquid | Liquid | <-10 | — | 20.0% | 18.1% | 9.9% |
| Working example C61 | C61 | $(CH_2)_5COOH$ | H | H | H | $CH_3CH(OH)COO^-$ | 1:1 | Present | Mono-hydrate | Liquid | Liquid | <-10 | — | 20.0% | 18.1% | 9.9% |
| Comparative example C1 | C62 | $CH_3(CH_2)_3$ | $CH_3(CH_2)_3$ | $CH_3(CH_2)_3$ | $CH_3(CH_2)_3$ | $Br^-$ | 1:1 | None | — | — | Solid | — | ≥25 | 20.0% | 15.3% | 23.6% |
| Comparative example C2 | C63 | $BMI-BF_4$ | | | | | — | None | — | — | Liquid | — | <-10 | 20.0% | 11.7% | 41.7% |
| Comparative example C3 | C64 | Glycerin | | | | | — | None | — | — | Liquid | — | >-5 | 20.0% | 18.0% | 10.0% |

As can be seen from the results shown in Tables 22 to 24, with the 80 wt% aqueous solution, the compounds C1 to C61 exhibited rates of reduction in moisture that were smaller than those of the compounds C62 to C64 of the comparative examples; the compounds C1 to C61 were superior in water retention property. Further, a liquidity was

maintained. Also, the compounds in a hydrate condition were turned into anhydrides via a decompression treatment so as to then observe the appearances thereof in an anhydride state at 25°C; the results were then listed in the Tables.

In the cases of the compounds C1 to C61 of the organic salts having the hydrogen-bonding functional group(s) (hydroxy group) in cations and/or anions, smaller rates of reduction in moisture were observed as compared to the compound C64 which likewise had the hydrogen-bonding functional group(s), but did not have a salt structure; it was indicated that the organic ammonium salts had a high water retention property.

In the cases of the compounds C12 to C14, C18, C19, C35, C37 and C38 having the hydrogen-bonding functional group(s) only in cations, and in the case of the compound 39 having the hydrogen-bonding functional group(s) only in anions, smaller rates of reduction in moisture were observed as compared to the compounds C62 and C63 having no hydrogen-bonding functional group; the organic ammonium salts having the hydrogen-bonding functional group(s) in either cations or anions were superior in water retention property.

As a result of making comparisons under a similar anion(s), in the cases of the compounds C1, C2, C5, C8, C15, C20, C23, C24, C25, C26, C29, C43 and C46 with the hydrogen-bonding functional group(s) already introduced into cations and anions, smaller rates of reduction in moisture were observed as compared to the compound C39 having the hydrogen-bonding functional group(s) only in anions. Further, as a result of making comparisons under a similar cation(s), in the cases of the compounds C6 to C11 with the hydrogen-bonding functional group(s) already introduced into cations and anions, smaller rates of reduction in moisture were observed as compared to the compounds C12 to C14 having the hydrogen-bonding functional group(s) only in cations. That is, it was confirmed that a more excellent water retention effect could be achieved if the hydrogen-bonding functional group(s) were present in both cations and anions.

As for a combination of substituent groups of cations, as a result of making comparisons under a similar anion(s) (lactic acid anion), in the cases of the compounds C1, C2, C5, C8, C15, C20, C23, C24, C25, C26, C29, C43 and C46 that had at least one hydroxyalkyl group and/or hydrogen atom directly bonded to nitrogen and could have an alkyl group(s) in cations, smaller rates of reduction in moisture were observed as compared to the compound C39 only composed of an alkyl group(s); the compounds having a hydroxyalkyl group(s) and a hydrogen atom(s) directly bonded to nitrogen in cations were superior in water retention property.

Next, as a result of comparing the hydrogen-bonding functional group(s) of cations, as compared to the compound C29 whose cations were composed of a hydroxyalkyl group(s) and an alkyl group(s), smaller rates of reduction in moisture were observed in the cases of the compounds C1, C2, C5, C8, C15, C20, C23, C24, C25, C26, C43 and C46 whose cations were only composed of a hydroxyalkyl group(s) and/or a hydrogen atom(s) directly bonded to nitrogen; it was confirmed that a superior water retention effect could be achieved when the cation structure was only composed of a hydroxyalkyl group(s) and/or a hydrogen atom(s) directly bonded to nitrogen.

Further, as compared to the compounds C25, C26 and C39 having no hydrogen atom(s) directly bonded to nitrogen, smaller rates of reduction in moisture were observed in the cases of the compounds C1, C2, C5, C8, C15, C20, C23, C24, C43 and C46 containing at least one hydrogen atom directly bonded to nitrogen; the compounds containing at least one hydrogen atom directly bonded to nitrogen were superior in water retention property. Particularly, as compared to the compound C24 only composed of a hydrogen atom(s) directly bonded to nitrogen, the compounds C1, C2, C5, C8, C15, C20, C23, C43 and C46 exhibited especially superior water retention properties. That is, it was indicated that a superior water retention property could be achieved especially when the cation structure was composed of a hydroxyalkyl group(s) and a hydrogen atom(s) directly bonded to nitrogen.

As for anion species, comparisons were made on anions using organic salts of similar cations. As a result of comparing the compounds C6 to C11, C15 to C17, C18 and C27 to C34 as compounds having a hydrogen-bonding functional group(s) in anions with the compounds C12 to C14, C19 and C35 as compounds having no hydrogen-bonding functional group(s) in anions, it was confirmed that the compounds C6 to C11, C15 to C17, C18 and C27 to C34 having a hydrogen-bonding functional group(s) in anions exhibited smaller rates of reduction in moisture. That is, as compared to halogen-based anions having no hydrogen-bonding functional group(s), compounds of anions having a hydrogen-bonding functional group-containing group(s) such as a hydroxy group, carboxy group, carboxylate group, sulfonyl group, phosphate group and phosphinic acid group were superior in water retention property. Further, as a result of making comparisons using halogen-based anions, superior water retention properties were observed in the cases of the compounds C12 (boron-based anion), C13 (bromide ion) and C14 (chloride ion) that are listed here in descending order of superiority.

(2) Water retention property test 2

The water retention property of the hydrate of each compound was evaluated. Hydrates were used in the cases of the compounds C1 to C38, C40 to C49 and C56 to C59; however, since the compounds C39 and C62 to C64 were anhydrides, there were used aqueous solutions prepared by adding water so that a concentration would be that of monohydrate to trihydrate. The moisture rate of the sample(s) was confirmed using a Karl Fischer moisture meter (pre-test moisture rate: A). Next, 1.0 g of each of these samples was added to a screw tube, and this screw tube, while remaining unsealed, was then left to stand still for 24 hours in a thermo-hygrostat set to 40°C and 25%RH. A moisture rate after 24 hours was again measured (post-test moisture rate: B), followed by calculating a rate of reduction in moisture in a similar manner as above so as to evaluate the water retention property. Here, a comparative evaluation was performed on each hydrate of the compounds C1 to C38, C40 to C49 and C56 to C59 together with an aqueous solution(s) of the compound C39 having a concentration(s) corresponding to the numbers of these hydration waters.

(monohydrates in compounds C1 to C38, C40 to C49 and C56 to C59 of working examples C62 to C99, C103 to C116 with compound C39 of working example C100, compound C62 of comparative example C4, compound C63 of comparative example C7 and compound C64 of comparative example C10)

(dihydrates in compounds C1 to C38, C40 to C49 and C56 to C59 of working examples C62 to C99, C103 to C116 with compound C39 of working example C101, compound C62 of comparative example C5, compound C63 of comparative example C8 and compound C64 of comparative example C11)

(trihydrates in compounds C1 to C38, C40 to C49 and C56 to C59 of working examples C62 to C99, C103 to C116 with compound C39 of working example C102, compound C62 of comparative example C6, compound C63 of comparative example C9 and compound C64 of comparative example C12)

[Table 25]

[Table 26]

| | Compound | 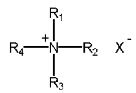 | | | | | Presence of hydration water | n-hydrate | Appearance at 25°C | | Freezing point | Water retention property test 2 Hydrate | | | |
|---|---|---|---|---|---|---|---|---|---|---|---|---|---|---|---|
| | | $R_1$ | $R_2$ | $R_3$ | $R_4$ | $X^-$ | | | Hydrate | An-hydride | Hydrate | n-hydrate | Pre-test moisture rate | Post-test moisture rate | Rate of reduction in moisture |
| Working example C62 | C1 | $(CH_2)_2OH$ | H | H | H | $CH_3CH(OH)COO^-$ | Present | Mono-hydrate | Liquid | Liquid | <-10 | Mono-hydrate | 10.7% | 10.5% | 1.9% |
| Working example C63 | C2 | | | | | $CH_3CH(OH)COO^-$ | Present | Di-hydrate | Liquid | Liquid | <-10 | Di-hydrate | 15.6% | 14.9% | 4.6% |
| Working example C64 | C3 | $(CH_2)_2OH$ | $(CH_2)_2OH$ | H | H | $HOCH_2COO^-$ | Present | Di-hydrate | Liquid | Liquid | <-10 | Di-hydrate | 16.6% | 16.1% | 3.0% |
| Working example C65 | C4 | | | | | $HOOC(CH_2)_2COO^-$ | Present | Di-hydrate | Liquid | Liquid | <-10 | Di-hydrate | 13.9% | 13.1% | 6.0% |
| Working example C66 | C5 | $(CH_2)_2OH$ | $(CH_2)_2OH$ | $(CH_2)_2OH$ | H | $CH_3CH(OH)COO^-$ | Present | Tri-hydrate | Liquid | Liquid | <-10 | Tri-hydrate | 18.4% | 17.7% | 3.8% |
| Working example C67 | C6 | | | | | $CH_3COO^-$ | Present | Di-hydrate | Liquid | Liquid | <-10 | Di-hydrate | 19.3% | 18.8% | 2.8% |
| Working example C68 | C7 | | | | | $HOCH_2COO^-$ | Present | Di-hydrate | Liquid | Liquid | <-10 | Di-hydrate | 17.7% | 17.3% | 2.0% |
| Working example C69 | C8 | | | | | $CH_3CH(OH)COO^-$ | Present | Di-hydrate | Liquid | Liquid | <-10 | Di-hydrate | 16.6% | 16.1% | 2.8% |
| Working example C70 | C9 | | | | | $HOOC(CH_2)_2COO^-$ | Present | Di-hydrate | Liquid | Liquid | <-10 | Di-hydrate | 14.7% | 13.9% | 5.4% |
| Working example C71 | C10 | 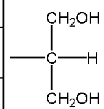 | H | H | H | $C(OH)(CH_2COOH)_2COO^-$ | Present | Di-hydrate | Liquid | Liquid | <-10 | Di-hydrate | 11.3% | 10.7% | 5.4% |
| Working example C72 | C11 | | | | | $CH_3SO_3^-$ | Present | Di-hydrate | Liquid | Liquid | <-10 | Di-hydrate | 16.1% | 16.0% | 0.4% |
| Working example C73 | C12 | | | | | $BF_4^-$ | Present | Di-hydrate | Liquid | Liquid | <-5 | Di-hydrate | 16.8% | 15.9% | 5.5% |
| Working example C74 | C13 | | | | | $Br^-$ | Present | Di-hydrate | Liquid | Liquid | <-10 | Di-hydrate | 17.3% | 15.7% | 9.5% |
| Working example C75 | C14 | | | | | $Cl^-$ | Present | Di-hydrate | Liquid | Liquid | <-10 | Di-hydrate | 22.0% | 19.7% | 10.5% |
| Working example C76 | C15 | | | | | $CH_3CH(OH)COO^-$ | Present | Di-hydrate | Liquid | Liquid | <-10 | Di-hydrate | 14.7% | 14.3% | 3.0% |
| Working example C77 | C16 | | | | | $C(OH)(CH_2COOH)_2COO^-$ | Present | Di-hydrate | Liquid | Liquid | <-10 | Di-hydrate | 10.4% | 9.9% | 4.9% |
| Working example C78 | C17 | 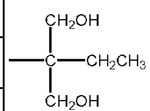 | H | H | H | $CH_3SO_3^-$ | Present | Di-hydrate | Liquid | Liquid | <-10 | Di-hydrate | 14.3% | 14.2% | 0.9% |
| Working example C79 | C18 | | | | | $Br^-$ | Present | Di-hydrate | Liquid | Liquid | <-10 | Di-hydrate | 15.3% | 13.9% | 8.9% |
| Working example C80 | C19 | | | | | $Cl^-$ | Present | Di-hydrate | Liquid | Liquid | <-10 | Di-hydrate | 18.8% | 17.0% | 9.8% |

| | Compound | 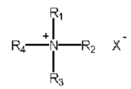 | | | | | Presence of hydration water | n-hydrate | Appearance at 25°C | | Freezing point | Water retention property test 2 Hydrate | | | |
|---|---|---|---|---|---|---|---|---|---|---|---|---|---|---|---|
| | | $R_1$ | $R_2$ | $R_3$ | $R_4$ | $X^-$ | | | Hydrate | An-hydride | Hydrate | n-hydrate | Pre-test moisture rate | Post-test moisture rate | Rate of reduction in moisture |
| Working example C81 | C20 | 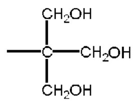 | H | H | H | $CH_3CH(OH)COO^-$ | Present | Di-hydrate | Liquid | Liquid | <-10 | Di-hydrate | 13.2% | 12.9% | 2.6% |
| Working example C82 | C21 | | | | | $C(OH)(CH_2COOH)_2COO^-$ | Present | Di-hydrate | Liquid | Liquid | <-10 | Di-hydrate | 10.3% | 9.8% | 5.0% |
| Working example C83 | C22 | | | | | $CH_3SO_3^-$ | Present | Di-hydrate | Liquid | Liquid | <-10 | Di-hydrate | 14.2% | 14.0% | 1.1% |
| Working example C84 | C23 | 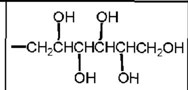 | H | H | H | $CH_3CH(OH)COO^-$ | Present | Di-hydrate | Liquid | Liquid | <-10 | Di-hydrate | 11.7% | 11.4% | 2.8% |
| Working example C85 | C24 | H | H | H | H | $CH_3CH(OH)COO^-$ | Present | Mono-hydrate | Solid | Solid | ≥25 | Mono-hydrate | 14.4% | 13.7% | 4.7% |
| Working example C86 | C25 | 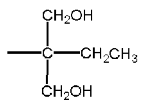 | 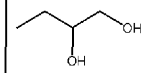 | 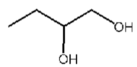 | 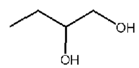 | $CH_3CH(OH)COO^-$ | Present | Di-hydrate | Liquid | Liquid | <-5 | Di-hydrate | 7.9% | 7.5% | 4.8% |
| Working example C87 | C26 | $(CH_2)_2OH$ | $(CH_2)_2OH$ | $(CH_2)_2OH$ | $(CH_2)_2OH$ | $CH_3CH(OH)COO^-$ | Present | Di-hydrate | Liquid | Liquid | <-5 | Di-hydrate | 11.2% | 10.7% | 4.8% |
| Working example C88 | C27 | $CH_3$ | $CH_3$ | $CH_3$ | $(CH_2)_2OH$ | $CH_3COO^-$ | Present | Mono-hydrate | Solid | Solid | ≥25 | Mono-hydrate | 9.9% | 9.5% | 4.0% |
| Working example C89 | C28 | | | | | $HOCH_2COO^-$ | Present | Mono-hydrate | Solid | Solid | ≥25 | Mono-hydrate | 9.1% | 8.9% | 2.6% |
| Working example C90 | C29 | | | | | $CH_3CH(OH)COO^-$ | Present | Mono-hydrate | Liquid | Liquid | <-10 | Mono-hydrate | 8.5% | 8.1% | 4.9% |
| Working example C91 | C30 | | | | | $HOOCCH=CHCOO^-$ | Present | Mono-hydrate | Solid | Solid | ≥25 | Mono-hydrate | 7.6% | 7.2% | 5.0% |
| Working example C92 | C31 | | | | | $HOOC(CH_2)_2COO^-$ | Present | Mono-hydrate | Liquid | Liquid | <-10 | Mono-hydrate | 7.5% | 7.1% | 5.0% |
| Working example C93 | C32 | | | | | $CH_3SO_3^-$ | Present | Mono-hydrate | Solid | Solid | ≥25 | Mono-hydrate | 8.3% | 8.1% | 2.0% |
| Working example C94 | C33 | | | | | $H_2PO_4^-$ | Present | Mono-hydrate | Solid | Solid | ≥25 | Mono-hydrate | 8.2% | 8.0% | 2.8% |
| Working example C95 | C34 | | | | | $H_2PO_2^-$ | Present | Mono-hydrate | Liquid | Liquid | <-10 | Mono-hydrate | 9.6% | 9.3% | 2.9% |
| Working example C96 | C35 | | | | | $Cl^-$ | Present | Mono-hydrate | Solid | Solid | ≥25 | Mono-hydrate | 11.4% | 10.7% | 6.0% |

[Table 27A]

| | Compound | 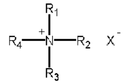 | | | | | Presence of hydration water | n-hydrate | Appearance at 25°C | | Freezing point | | Water retention property test 2 Hydrate | | | |
|---|---|---|---|---|---|---|---|---|---|---|---|---|---|---|---|---|
| | | $R_1$ | $R_2$ | $R_3$ | $R_4$ | $X^-$ | | | Hydrate | An-hydride | Hydrate | An-hydride | n-hydrate | Pre-test moisture rate | Post-test moisture rate | Rate of reduction in moisture |
| Working example C97 | C36 | $CH_3$ | $CH_3$ | $CH_3CH_2$ | $(CH_2)_2OH$ | $CH_3SO_3^-$ | Present | Mono-hydrate | Liquid | Liquid | <-10 | — | Mono-hydrate | 7.8% | 7.7% | 1.9% |
| Working example C98 | C37 | $CH_2CH(OH)CH_2OH$ | $CH_3$ | $CH_3$ | $(CH_2)_2OH$ | $Cl^-$ | Present | Di-hydrate | Liquid | Liquid | <-10 | — | Di-hydrate | 15.3% | 14.0% | 8.7% |
| Working example C99 | C38 | $CH_2CH(OH)CH_2-N^-(CH_3)_2(CH_2CH_2OH)$ | $CH_3$ | $CH_3$ | $(CH_2)_2OH$ | $Cl^-$ | Present | Di-hydrate | Solid | Solid | ≥25 | — | Di-hydrate | 10.5% | 9.4% | 10.2% |
| Working example C100 | C39 | $CH_3(CH_2)_3$ | $CH_3(CH_2)_3$ | $CH_3(CH_2)_3$ | $CH_3(CH_2)_3$ | $CH_3CH(OH)COO^-$ | None | — | — | Solid | — | ≥25 | Mono-hydrate | 5.2% | 4.9% | 6.4% |
| Working example C101 | | | | | | | | | | | | | Di-hydrate | 9.8% | 8.9% | 9.2% |
| Working example C102 | | | | | | | | | | | | | Tri-hydrate | 14.0% | 12.3% | 12.0% |
| Working example C103 | C40 | $(CH_2)_2OH$ | H | H | H | Ascorbic acid anion | Present | Di-hydrate | Solid | Solid | ≥25 | — | Di-hydrate | 13.2% | 13.0% | 1.5% |
| Working example C104 | C41 | $(CH_2)_2OH$ | $(CH_2)_2OH$ | H | H | Ascorbic acid anion | Present | Di-hydrate | Liquid | Liquid | <-10 | — | Di-hydrate | 11.4% | 11.0% | 3.5% |
| Working example C105 | C42 | $(CH_2)_2OH$ | $(CH_2)_2OH$ | $(CH_2)_2OH$ | H | Ascorbic acid anion | Present | Di-hydrate | Liquid | Liquid | <-10 | — | Di-hydrate | 10.0% | 9.8% | 2.0% |
| Working example C106 | C45 | 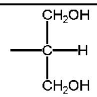 | H | H | H | Ascorbic acid anion | Present | Di-hydrate | Solid | Solid | ≥25 | — | Di-hydrate | 11.9% | 11.7% | 1.7% |
| Working example C107 | C48 | 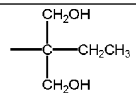 | H | H | H | Ascorbic acid anion | Present | Di-hydrate | Solid | Solid | ≥25 | — | Di-hydrate | 10.9% | 10.6% | 2.8% |
| Working example C108 | C49 | 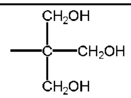 | H | H | H | Ascorbic acid anion | Present | Di-hydrate | Solid | Solid | ≥25 | — | Di-hydrate | 10.8% | 10.5% | 2.8% |
| Working example C109 | C56 | 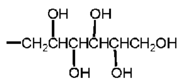 | H | H | H | Ascorbic acid anion | Present | Di-hydrate | Solid | Solid | ≥25 | — | Di-hydrate | 9.2% | 9.1% | 1.1% |

[Table 27B]

| | Compound | 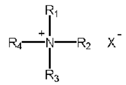 | | | | | Presence of hydration water | n-hydrate | Appearance at 25°C | | Freezing point | | Water retention property test 2 Hydrate | | | |
|---|---|---|---|---|---|---|---|---|---|---|---|---|---|---|---|---|
| | | $R_1$ | $R_2$ | $R_3$ | $R_4$ | $X^-$ | | | Hydrate | An-hydride | Hydrate | An-hydride | n-hydrate | Pre-test moisture rate | Post-test moisture rate | Rate of reduction in moisture |
| Working example C110 | C57 | $CH_3$ | $CH_3$ | $CH_3$ | $(CH_2)_2OH$ | Ascorbic acid anion | Present | Di-hydrate | Solid | Solid | ≥25 | — | Di-hydrate | 11.4% | 11.2% | 1.8% |
| Working example C111 | C58 | $CH_3$ | $CH_3$ | $CH_3$ | $CH_2CH(OH)CH_2COOH$ | Ascorbic acid anion | Present | Di-hydrate | Liquid | Liquid | <-10 | — | Di-hydrate | 9.7% | 9.6% | 1.0% |
| Working example C112 | C59 | $CH_3$ | $CH_3$ | $CH_3$ | $(CH_2)_2OC(=O)CH_3$ | Ascorbic acid anion | Present | Di-hydrate | Liquid | Liquid | <-10 | — | Di-hydrate | 10.1% | 9.9% | 2.0% |
| Working example C113 | C43 | 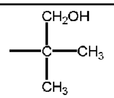 | H | H | H | $CH_3CH(OH)COO^-$ | Present | Di-hydrate | Liquid | Liquid | <-10 | — | Di-hydrate | 16.7% | 16.3% | 2.5% |
| Working example C114 | C46 | 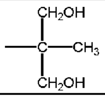 | H | H | H | $CH_3CH(OH)COO^-$ | Present | Di-hydrate | Liquid | Liquid | <-10 | — | Di-hydrate | 15.6% | 15.2% | 2.8% |
| Working example C115 | C44 | 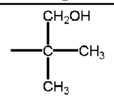 | H | H | H | Ascorbic acid anion | Present | Di-hydrate | Liquid | Liquid | <-10 | — | Di-hydrate | 12.0% | 11.7% | 2.9% |
| Working example C116 | C47 | 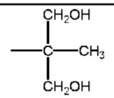 | H | H | H | Ascorbic acid anion | Present | Di-hydrate | Liquid | Liquid | <-10 | — | Di-hydrate | 11.4% | 11.1% | 2.9% |
| Comparative example C4 | C62 | $CH_3(CH_2)_3$ | $CH_3(CH_2)_3$ | $CH_3(CH_2)_3$ | $CH_3(CH_2)_3$ | $Br^-$ | None | — | — | Solid | — | ≥25 | Mono-hydrate | 5.3% | 4.9% | 8.1% |
| Comparative example C5 | | | | | | | | | | | | | Di-hydrate | 10.1% | 8.6% | 15.1% |
| Comparative example C6 | | | | | | | | | | | | | Tri-hydrate | 14.4% | 10.8% | 25.1% |
| Comparative example C7 | C63 | $BMI-BF_4$ | | | | | None | — | — | Liquid | — | <-10 | Mono-hydrate | 7.4% | 3.9% | 47.3% |
| Comparative example C8 | | | | | | | | | | | | | Mono-hydrate | 13.8% | 7.1% | 48.6% |
| Comparative example C9 | | | | | | | | | | | | | Tri-hydrate | 19.3% | 11.2% | 42.1% |
| Comparative example C10 | C64 | Glycerin | | | | | None | — | — | Liquid | — | >-5 | Mono-hydrate | 16.4% | 15.3% | 6.7% |
| Comparative example C11 | | | | | | | | | | | | | Di-hydrate | 28.1% | 24.4% | 13.3% |
| Comparative example C12 | | | | | | | | | | | | | Tri-hydrate | 37.0% | 31.9% | 13.7% |

As can be seen from the results shown in Tables 25 o 27, the compounds C1 to C49 and C56 to C59 of the working examples exhibited rates of reduction in moisture that were smaller than those of the compounds C62 to C64 of the comparative examples, and were thus superior in water retention property.

Further, as for a correlation(s) between the molecular structures of cations and anions and the water retention property, there were observed tendencies similar to those of the water retention property test 1.

As for the compounds C1 to C13, C15 to C18, C20 to C34, C36, C40 to C49 and C56 to C59 of the working examples, smaller rates of reduction in moisture tended to be observed with regard to the hydrates in the water retention property test 2 as compared to the 80 wt% aqueous solutions in the water retention property test 1. The pre- and post-test moisture rates of the 80 wt% aqueous solutions in the water retention property test 1 were larger than the moisture rates of the hydrates (e.g. post-test moisture rate of 80 wt% aqueous solution in the case of compound C8: 19.2%, moisture rate of hydrate in the case of compound C8: 16.6%); free water had volatilized, whereas in the water retention property test 2, hydration water had volatilized to cause a reduction in moisture. That is, it was indicated that in the cases of the compounds of hydrates, in a range(s) not higher than the moisture rates of the hydrates, the hydration water had a low volatility, which resulted in smaller rates of reduction in moisture and in a favorable water retention property accordingly. However, as for the compounds C14, C19, C35, C37 and C38 employing chloride anions, the rates of reduction in moisture of the hydrates were either equal to or larger than the rates of reduction in moisture of the 80 wt% aqueous solutions; the aforementioned tendencies were not exhibited.

Further, evaluations were performed on tendencies of rates of reduction in moisture depending on whether hydrates had been formed. As a result of comparing each of the mono-, di- and trihydrates under a similar anion(s), in the cases of the compounds C1, C2, C5, C8, C15, C20, C23, C24, C25, C26, C29, C43 and C46 forming hydrates, smaller rates of reduction in moisture were observed as compared to the compound C39 as an anhydride not forming a hydrate; it was confirmed that the compounds forming hydrates were superior in water retention property.

(3) Water retention property test 3

In a water retention property test 3, an 80 wt% aqueous solution of each of the compounds C8, C11, C62, C63 and C64 was prepared, followed by using a Karl Fischer moisture meter to confirm that a moisture rate was 20.0 wt%. Next, 1.0 g of each of these samples was added to a screw tube, and this screw tube, while remaining unsealed, was then left to stand still in a thermo-hygrostat set to 40°C and 25%RH. After 24, 96, 120, 144, 168 and 192 hours, the moisture rate was again measured by the Karl Fischer moisture meter. Also, by measuring weights, amounts of moisture reduction were calculated for each time period (0 to 24 h, 24 to 96 h, 96 to 120 h, 120 to 144 h, 144 to 168 h and 168 to 192 h). In addition, the water retention property was evaluated by calculating an amount of moisture reduction per hour (mg/h) based on each amount of moisture reduction (mg).

[Table 28A]

| | Compound | 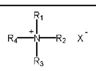 | | | | | Water retention property test 3 80 wt% aqueous solution | | | | | | | | | | | |
|---|---|---|---|---|---|---|---|---|---|---|---|---|---|---|---|---|---|---|
| | | | | | | | Presence of hydration water | Moisture rate of hydrate (%) | Pre-test moisture rate (%) | 0～24h | | | 24～96h | | | 96～120h | | |
| | | $R_1$ | $R_2$ | $R_3$ | $R_4$ | $X^-$ | | | | Moisture rate after 24h (%) | Amount of moisture reduction in 0 to 24h (mg) | Amount of moisture reduction per hour (mg/h) | Moisture rate after 96h (%) | Amount of moisture reduction in 24 to 96h (mg) | Amount of moisture reduction per hour (mg/h) | Moisture rate after 120h (%) | Amount of moisture reduction in 96 to 120h (mg) | Amount of moisture reduction per hour (mg/h) |
| Working example C117 | C8 | 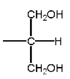 | H | H | H | $CH_3CH(OH)COO^-$ | Di-hydrate | 16.6 | 20.0 | 19.5 | 6.7 | 0.3 | 17.8 | 20.1 | 0.3 | 17.1 | 8.1 | 0.3 |
| Working example C118 | C11 | | | | | $CH_3SO_3^-$ | Di-hydrate | 16.1 | 20.0 | 19.4 | 6.5 | 0.3 | 17.6 | 20.0 | 0.3 | 16.9 | 7.5 | 0.3 |
| Comparative example C13 | C62 | $CH_3(CH_2)_3$ | $CH_3(CH_2)_3$ | $CH_3(CH_2)_3$ | $CH_3(CH_2)_3$ | $Br^-$ | None | — | 20.0 | 15.9 | 49.1 | 2.0 | 0.7 | 145.8 | 2.0 | — | | |
| Comparative example C14 | C63 | $BMI-BF_4$ | | | | | None | — | 20.0 | 14.5 | 64.3 | 2.7 | 0.0 | 134.4 | 1.9 | — | | |
| Comparative example C15 | C64 | Glycerin | | | | | None | — | 20.0 | 18.0 | 24.6 | 1.0 | 11.1 | 74.8 | 1.0 | 8.6 | 25.0 | 1.0 |

[Table 28B]

| | Compound | 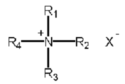 | | | | | Water retention property test 3 80 wt% aqueous solution | | | | | | | | | | | |
|---|---|---|---|---|---|---|---|---|---|---|---|---|---|---|---|---|---|---|
| | | | | | | | Presence of hydration water | Moisture rate of hydrate (%) | Pre-test moisture rate (%) | 120～144h | | | 144～168h | | | 168～192h | | |
| | | R1 | R2 | R3 | R4 | X⁻ | | | | Moisture rate after 144h (%) | Amount of moisture reduction in 120 to 144h (mg) | Amount of moisture reduction per hour (mg/h) | Moisture rate after 168h (%) | Amount of moisture reduction in144 to 168h (mg) | Amount of moisture reduction per hour (mg/h) | Moisture rate after 192h (%) | Amount of moisture reduction in 168 to 192h (mg) | Amount of moisture reduction per hour (mg/h) |
| Working example C117 | C8 | 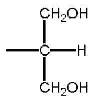 | H | H | H | $CH_3CH(OH)COO^-$ | Di-hydrate | 16.6 | 20.0 | 16.5 | 7.7 | 0.3 | 16.1 | 4.0 | 0.2 | 15.8 | 4.0 | 0.2 |
| Working example C118 | C11 | | | | | $CH_3SO_3^-$ | Di-hydrate | 16.1 | 20.0 | 16.2 | 7.2 | 0.3 | 15.8 | 4.7 | 0.2 | 15.4 | 4.1 | 0.2 |
| Comparative example C13 | C62 | $CH_3(CH_2)_3$ | $CH_3(CH_2)_3$ | $CH_3(CH_2)_3$ | $CH_3(CH_2)_3$ | $Br^-$ | None | — | 20.0 | — | | | — | | | — | | |
| Comparative example C14 | C63 | $BMI\text{-}BF_4$ | | | | | None | — | 20.0 | — | | | — | | | — | | |
| Comparative example C15 | C64 | Glycerin | | | | | None | — | 20.0 | 5.4 | 29.4 | 1.2 | 1.9 | 30.0 | 1.3 | 0.3 | 13.1 | 0.5 |

As a result, while a moisture rate of smaller than 1% was observed in the cases of the compounds C62 and C63 of the comparative examples after 96 hours and in the case of the compound C64 of a comparative example after 192 hours, a moisture rate of not smaller than 15% was observed in the cases of the compounds C8 and C11 of the working examples even after 192 hours; a long-term water retention effect was confirmed with regard to the hydrogen-bonding functional group-containing organic salt compound of the present application.

The moisture rates after 0 to 144 hours in the cases of the compounds C8 and C11 (compound C8: 16.5%, compound C11: 16.2%) were substantially equal to or larger than the moisture rates of the hydrates thereof (compound C8: 16.6%, compound C11: 16.1%); free water had volatilized, whereas for the time period of 144 to 192 hours, the moisture rates of these compounds were smaller than the moisture rates of the hydrates thereof as the hydration water had volatilized. While an amount of moisture reduction per hour for the time period of 0 to 144 hours in the cases of the compounds C8 and C11 where the free water had volatilized was 0.3 mg/h, the amount of moisture reduction per hour decreased to 0.2 mg/h for the time period of 144 to 192 hours where the hydration water had volatilized. That is, it was indicated that the hydration water had a low volatility, and that compounds of hydrates were superior in long-term water retention effect.

(4) Water confinement property test (barrier property)

Here, 0.01 $mL/cm^2$ of an 80 wt% aqueous solution of each of the compounds C8, C11, C20, C29, C43, C46, C47, C49 to C55 and C62 to C83 was applied to a 5C quantitative filter paper (by Toyo Roshi Kaisha, Ltd.) cut into a size of 3 cm × 3 cm. Next, 20.0 g of an ion-exchange water was put into a 40 mL pierce vial bottle (by AS ONE Corporation.), followed by removing an inner lid (rubber plug) of the pierce vial bottle, placing on the pierce vial bottle the filter paper to which each sample had been applied, and then plugging the bottle with an outer lid. This bottle was then put into a thermo-hygrostat set to 40°C and 25%RH and left to stand still for 24 hours before measuring the weight of the ion-exchange water in the vial.

A water evaporation amount with regard to a filter paper to which only the ion-exchange water had been applied was defined as a water evaporation amount W (g), a water evaporation amount with regard to the filter paper to which the sample had been applied was defined as a water evaporation amount S (g), a weight of the ion-exchange water in the vial before the storage of each of these filter papers was $Wm_1$=20.0 g, and a weight of the ion-exchange water in the vial after 24 hours was defined as $Wm_2$; a water evaporation inhibition rate (%) was calculated with the following formula to evaluate a water confinement property. As for the water confinement property, it is shown that the higher the numerical value was, the more superior the water confinement property was. Further, $Wm_1$, $Wm_2$, $Sm_1$ and $Sm_2$ in the formula below respectively represent the following numerical values.

$Wm_1$: Weight of ion-exchange water in vial before storage (using filter paper to which only ion-exchange water had been applied)
$Wm_2$: Weight of ion-exchange water in vial after 24 hours (using filter paper to which only ion-exchange water had been applied)
$Sm_1$: Weight of ion-exchange water in vial before storage (using filter paper to which sample had been applied)
$Sm_2$: Weight of ion-exchange water in vial after 24 hours (using filter paper to which sample had been applied)
Water evaporation amount

$$W\ (g)=Wm_1-Wm_2$$

Water evaporation amount

$$S\ (g)=Sm_1-Sm_2$$

Water evaporation inhibition rate

$$(\%)=[(W\ (g)-S\ (g))/W\ (g)]\times 100$$

The results of the water confinement property test are shown in Table 8.

[Table 29]

| | Compound | 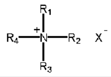 | | | | | Compounding molar ratio (acid:base) | Water evaporation inhibition rate |
|---|---|---|---|---|---|---|---|---|
| | | $R_1$ | $R_2$ | $R_3$ | $R_4$ | $X^-$ | | |
| Working example C119 | C8 | 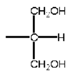 | H | H | H | $CH_3CH(OH)COO^-$ | 1:1 | 37.4% |
| Working example C120 | C11 | | | | | $CH_3SO_3$ | 1:1 | 40.0% |
| Working example C121 | C43 | 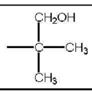 | H | H | H | $CH_3CH(OH)COO^-$ | 1:1 | 39.7% |
| Working example C122 | C65 | 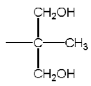 | H | H | H | $CH_3COO-$ | 1:1 | 39.4% |
| Working example C123 | C46 | | | | | $CH_3CH(OH)COO^-$ | 1:1 | 41.4% |
| Working example C124 | C66 | | | | | $HOCH_2COO^-$ | 1:1 | 38.1% |
| Working example C125 | C67 | | | | | $HOOC(CH_2)_2COO^-$ | 1:1 | 42.3% |
| Working example C126 | C68 | | | | | $HOOCCH_2CH(OH)COO^-$ | 1:1 | 38.8% |
| Working example C127 | C69 | | | | | $^-OOCCH_2CH(OH)COO^-$ | 1:2 | 36.7% |
| Working example C128 | C70 | | | | | $HOOCCH(OH)CH(OH)COO^-$ | 1:1 | 39.5% |
| Working example C129 | C71 | | | | | $^-OOCCH(OH)CH(OH)COO^-$ | 1:2 | 35.8% |
| Working example C130 | C72 | | | | | $CH_3COCH_2CH_2COO^-$ | 1:1 | 36.1% |
| Working example C131 | C73 | | | | | $Cl^-$ | 1:1 | 38.6% |
| Working example C132 | C74 | | | | | $OOC(CH_2)_2CH(COO\ )NHCOCH_3$ | 1:1 | 35.5% |
| Working example C133 | C75 | | | | | $H_2N(CH_2)_3COO^-$ | 1:1 | 36.6% |
| Working example C134 | C76 | | | | | $H_2N(CH_2)_5COO$ | 1:1 | 35.7% |
| Working example C135 | C47 | | | | | Ascorbic acid anion | 1:1 | 34.8% |
| Working example C136 | C77 | 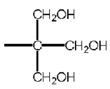 | H | H | H | $CH_3COO-$ | 1:1 | 35.4% |
| Working example C137 | C20 | | | | | $CH_3CH(OH)COO^-$ | 1:1 | 38.0% |
| Working example C138 | C50 | | | | | $HOCH_2COO^-$ | 1:1 | 39.1% |
| Working example C139 | C78 | | | | | $HOOC(CH_2)_2COO^-$ | 1:1 | 34.5% |
| Working example C140 | C51 | | | | | $HOOCCH_2CH(OH)COO^-$ | 1:1 | 35.6% |
| Working example C141 | C52 | | | | | $^-OOCCH_2CH(OH)COO^-$ | 1:2 | 36.8% |
| Working example C142 | C79 | | | | | $HOOCCH(OH)CH(OH)COO^-$ | 1:1 | 33.5% |
| Working example C143 | C80 | | | | | $^-OOCCH(OH)CH(OH)COO^-$ | 1:2 | 38.6% |
| Working example C144 | C53 | | | | | $CH_3COCH_2CH_2COO$ | 1:1 | 34.6% |
| Working example C145 | C81 | | | | | $Cl^-$ | 1:1 | 33.5% |
| Working example C146 | C54 | | | | | $HOOCCH_2CH(COO^-)NH_2$ | 1:1 | 30.3% |
| Working example C147 | C55 | | | | | $^-OOC(CH_2)_2CH(COO^-)NHCOCH_3$ | 1:2 | 34.1% |
| Working example C148 | C82 | | | | | $H_2N(CH_2)_3COO^-$ | 1:1 | 35.8% |
| Working example C149 | C83 | | | | | $H_2N(CH_2)_5COO^-$ | 1:1 | 31.3% |
| Working example C150 | C49 | | | | | Ascorbic acid anion | 1:1 | 32.1% |
| Working example C151 | C29 | $CH_3$ | $CH_3$ | $CH_3$ | $(CH_2)_2OH$ | $CH_3CH(OH)COO^-$ | 1:1 | 39.1% |
| Comparative example C16 | C62 | $CH_3(CH_2)_3$ | $CH_3(CH_2)_3$ | $CH_3(CH_2)_3$ | $CH_3(CH_2)_3$ | $Br^-$ | — | 29.5% |
| Comparative example C17 | C63 | BMI-$BF_4$ | | | | | — | 25.7% |
| Comparative example C18 | C64 | Glycerin | | | | | — | 16.2% |

While the water evaporation inhibition rates in the comparative examples C16 to C18 were 16.2 to 29.5%, the compounds of the working examples exhibited water evaporation inhibition rates of 30.3 to 42.3%; it was confirmed that the compounds of the working examples were superior in water confinement property.

Further, while crystal precipitation was observed after the test in the case of a filter paper to which the compound C62 of the comparative example C16 being a solid at room temperature had been applied, precipitation of crystals or the like was not observed in the cases of filter papers to which the compounds of the working examples being liquids at room temperature had been applied; the results indicated that a liquid compound was superior to a solid compound in application property, permeability and appearance after drying. In addition, it was indicated that if dissolving an active ingredient(s) therein, the effects of such active ingredient(s) could be maintained for a long period of time.

Further, after comparing the compounds C8, C11, C20, C29, C43, C46, C47, C49 to C55 and C65 to C83 of the working examples with the compounds C62, C63 and C64 of the comparative examples, a lower water evaporation inhibition rate was observed with the compound C64 of the comparative example; it was confirmed that a non-volatile compound having a salt structure was desired in terms of water evaporation inhibition effect. In addition, after comparing the compounds C8, C11, C20, C29, C43, C46, C47, C49 to C55 and C65 to C83 of the working examples having the salt structures with the compounds C62 and C63 of the comparative examples, higher water evaporation inhibition rates were observed with the compounds of the working examples, and it was even confirmed that the water evaporation inhibition effects were able to be further improved when a hydrogen-bonding functional group(s) were present in the salt structure(s).

(5) Sensory evaluation

Each compound listed in the working examples C152 to C191 and comparative examples C19 to C21 shown in Tables 30 and 31 was diluted to a given concentration and then put into a spray bottle. A given amount of the diluted compound was then sprayed onto the skin to evaluate a moisture retention feeling, non-stickiness and skin compatibility at the time of application. As a panel, five individuals were randomly selected regardless of age and sex; an average value(s) were calculated and were then regarded as evaluation values.

As for a moisture retention feeling after application, the aqueous solution of each compound was applied to the skin, and a feeling of the skin after spreading the aqueous solution applied was then evaluated on a scale of 1 to 5, in which 5 was given to examples where a moisture retention feeling was felt significantly, 3 was given to examples where a moisture retention feeling was felt, and 1 was given to examples where no moisture retention feeling was felt.

As for a stickiness after application, the aqueous solution of each compound was applied to the skin, and a feeling of the skin after spreading the aqueous solution applied was then evaluated on a scale of 1 to 5, in which 5 was given to examples where no stickiness was felt, 3 was given to examples where stickiness was felt slightly, and 1 was given to examples where stickiness was felt.

As for a skin compatibility after application, the aqueous solution of each compound was applied to the skin, and a feeling of the skin after spreading the aqueous solution applied was then evaluated on a scale of 1 to 5, in which 5 was given to examples where a favorable compatibility was observed, 3 was given to examples where a compatibility was observed, and 1 was given to examples where a poor compatibility was observed.

| | Compound | 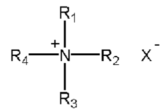 | | | | | Compound concentration (wt%) | Feeling of use | | |
|---|---|---|---|---|---|---|---|---|---|---|
| | | $R_1$ | $R_2$ | $R_3$ | $R_4$ | $X^-$ | | Moisture retention feeling | Non-stickiness | Skin compatibility |
| Working example 152 | 20 | 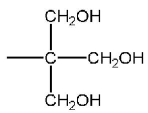 | H | H | H | $CH_3CH(OH)COO^-$ | 1 | 4.2 | 4.6 | 4.2 |
| Working example 153 | 20 | | | | | $CH_3CH(OH)COO^-$ | 10 | 4.6 | 4.1 | 4.3 |
| Working example 154 | 77 | | | | | $CH_3COO^-$ | 20 | 3.7 | 4.5 | 3.5 |
| Working example 155 | 20 | | | | | $CH_3CH(OH)COO^-$ | 20 | 4.6 | 3.7 | 3.7 |
| Working example 156 | 50 | | | | | $HOCH_2COO^-$ | 20 | 4.6 | 3.7 | 3.5 |
| Working example 157 | 78 | | | | | $HOOC(CH_2)_2COO^-$ | 20 | 3.7 | 4.6 | 3.7 |
| Working example 158 | 84 | | | | | $^-OOC(CH_2)_2COO^-$ | 20 | 3.7 | 4.5 | 3.8 |
| Working example 159 | 51 | | | | | $HOOCCH_2CH(OH)COO^-$ | 20 | 4.3 | 3.1 | 3.7 |
| Working example 160 | 52 | | | | | $^-OOCCH2CH(OH)COO^-$ | 20 | 3.7 | 3.8 | 3.7 |
| Working example 161 | 79 | | | | | $HOOCCH(OH)CH(OH)COO^-$ | 20 | 4.5 | 3.1 | 3.6 |
| Working example 152 | 80 | | | | | $^-OOCCH(OH)CH(OH)COO^-$ | 20 | 3.7 | 3.8 | 3.7 |
| Working example 163 | 85 | | | | | Fumaric acid anion | 20 | 3.7 | 4.4 | 3.7 |
| Working example 164 | 53 | | | | | $CH_3COCH_2CH_2COO^-$ | 20 | 4.6 | 3.8 | 3.8 |
| Working example 165 | 54 | | | | | $HOOCCH_2CH(COO^-)NH_2$ | 20 | 4.4 | 3.1 | 3.7 |
| Working example 166 | 86 | | | | | $HOOC(CH_2)_2CH(COO^-)NHCOCH_3$ | 20 | 4.4 | 3.1 | 3.7 |
| Working example 167 | 55 | | | | | $^-OOC(CH_2)_2CH(COO^-)NHCOCH_3$ | 20 | 4.3 | 3.1 | 3.5 |
| Working example 168 | 49 | | | | | Ascorbic acid anion | 20 | 4.5 | 3.8 | 3.8 |
| Comparative example 65 | 64 | Glycerin | | | | | 1 | 3.3 | 3.1 | 3.8 |
| Comparative example 66 | 64 | Glycerin | | | | | 10 | 3.9 | 2.6 | 3.9 |
| Comparative example 67 | 64 | Glycerin | | | | | 20 | 3.5 | 2.6 | 3.4 |

[Table 30B]

| | Compound | 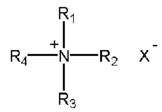 | | | | | Compound concentration (wt%) | Feeling of use | | |
|---|---|---|---|---|---|---|---|---|---|---|
| | | $R_1$ | $R_2$ | $R_3$ | $R_4$ | $X^-$ | | Moisture retention feeling | Non-stickiness | Skin compatibility |
| Working example C169 | C65 | 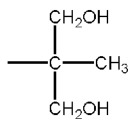 | H | H | H | $CH_3COO^-$ | 20 | 3.7 | 3.8 | 3.7 |
| Working example C170 | C46 | | | | | $CH_3CH(OH)COO^-$ | 20 | 4.5 | 3.1 | 3.5 |
| Working example C171 | C66 | | | | | $HOCH_2COO^-$ | 20 | 3.7 | 3.6 | 3.7 |
| Working example C172 | C67 | | | | | $HOOC(CH_2)_2COO^-$ | 20 | 4.3 | 3.7 | 3.8 |
| Working example C173 | C87 | | | | | $^-OOC(CH_2)_2COO^-$ | 20 | 4.2 | 3.1 | 3.7 |
| Working example C174 | C68 | | | | | $HOOCCH_2CH(OH)COO^-$ | 20 | 4.6 | 3.1 | 3.7 |
| Working example C175 | C69 | | | | | $^-OOCCH2CH(OH)COO^-$ | 20 | 3.7 | 3.8 | 3.6 |
| Working example C176 | C70 | | | | | $HOOCCH(OH)CH(OH)COO^-$ | 20 | 4.3 | 3.1 | 3.7 |
| Working example C177 | C71 | | | | | $^-OOCCH(OH)CH(OH)COO^-$ | 20 | 4.3 | 3.1 | 3.7 |
| Working example C178 | C88 | | | | | Fumaric acid anion | 20 | 3.7 | 4.5 | 3.8 |
| Working example C179 | C72 | | | | | $CH_3COCH_2CH_2COO^-$ | 20 | 4.5 | 3.8 | 3.7 |
| Working example C180 | C89 | | | | | $HOOC(CH_2)_2CH(COO^-)NHCOCH_3$ | 20 | 4.5 | 3.7 | 3.7 |
| Working example C181 | C74 | | | | | $^-OOC(CH_2)_2CH(COO^-)NHCOCH_3$ | 20 | 4.4 | 3.1 | 3.5 |
| Working example C182 | C47 | | | | | Ascorbic acid anion | 20 | 3.7 | 4.5 | 3.8 |
| Working example C183 | C44 | 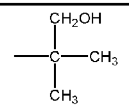 | H | H | H | $CH_3CH(OH)COO^-$ | 20 | 3.6 | 3.7 | 3.6 |
| Working example C184 | C60 | $(CH_2)_3COOH$ | H | H | H | $CH_3CH(OH)COO^-$ | 20 | 3.8 | 3.5 | 3.7 |
| Working example C185 | C61 | $(CH_2)_5COOH$ | H | H | H | $CH_3CH(OH)COO^-$ | 20 | 4.3 | 3.8 | 3.7 |

[Table 31]

| | Compound | 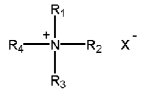 | | | | | Compound concentration (wt%) | Feeling of use | | |
|---|---|---|---|---|---|---|---|---|---|---|
| | | $R_1$ | $R_2$ | $R_3$ | $R_4$ | $X^-$ | | Non-stickiness | Spreadability | Skin compatibility |
| Working example C186 | C90 | 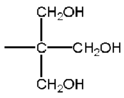 | H | H | H | Isostearic acid anion | 100 | 3.1 | 3.2 | 4.0 |
| Working example C187 | C91 | | | | | Oleic acid anion | 100 | 4.8 | 4.7 | 4.3 |
| Working example C188 | C92 | | | | | Linoleic acid anion | 100 | 4.5 | 4.6 | 4.1 |
| Working example C189 | C93 | 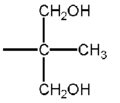 | H | H | H | Isostearic acid anion | 100 | 3.2 | 3.3 | 4.1 |
| Working example C190 | C94 | | | | | Oleic acid anion | 100 | 4.7 | 4.7 | 4.4 |
| Working example C191 | C95 | | | | | Linoleic acid anion | 100 | 4.5 | 4.5 | 4.1 |

As can be seen from the results shown in Table 30, by making comparisons under a same concentration, it was confirmed that the working examples C152 to C185 using the organic ammonium salt(s) were superior to the comparative examples C19 to C21 in moisture retention feeling, non-stickiness and skin compatibility.

Further, as can be seen from the results shown in Table 31, it was confirmed that the organic ammonium salt using, as an anion(s), a fatty acid-based anion(s) having 18 carbon atoms was likewise superior in any of the feelings of use including the moisture retention feeling, non-stickiness and skin compatibility.

In this way, it was indicated that the skin care agent of the present invention is suitable as a skin care agent as it brings about excellent feelings of use when applied to the skin. Particularly, as are the cases with the working examples 152 to 191, it can be said that a high safety can be realized when the raw materials of the cations and anions of the organic ammonium salt (acids, bases) are those listed in JSQI.

(6) Surface resistance measurement of organic ammonium salt

Here, 0.7 g (content in terms of anhydride) of each of the compounds C20 and C64 was applied to an artificial leather made of polyurethane, followed by using a high resistance meter (Stack TR-2 by Tokyo Electronics Co., Ltd.) to measure a surface resistance value. While the surface resistance value was greater than $4\times10^{11}\Omega$ when nothing had yet been applied thereto, a surface resistance value of lower than $1\times10^{8}\Omega$ was observed in the case of the compound C20, and a surface resistance value of $5\times10^{8}\Omega$ was observed in the case of the compound C64, provided that the compounds were both in a liquid state at the time of measurement; it was indicated that as compared to the compound C64, the compound C20 was able to bring about a more favorable electrical conductivity on the coating surface, suppress static electricity and impart an antistatic effect, thus showing a superiority of the structural characteristics of the skin care agent of the present invention.

Further, 0.7 g (content in terms of anhydride) of the compound C96 (sodium lactate) was applied to an artificial leather made of polyurethane. After drying the same to volatilize water, the coating surface turned into a solid state, and a surface resistance value measured in a similar manner as above was $2\times10^{8}\Omega$; confirmed were a non-volatility of the compound C20 (lower than $1\times10^{8}\Omega$) and an effect of liquidity at 25°C (capability of evenly coating an object to be coated).

In this way, it was indicated that the skin care agent of the present invention was superior in antistatic property and contributed to making it less likely for dust to adhere to the skin or cling to clothes due to static electricity.

(7) Evaluation on solubility of active ingredient

Solubilities of active ingredients in aqueous solutions of the compounds C20 and C64 listed in Table 32 were evaluated. As the active ingredients, there were used a poorly-soluble gallic acid having an antioxidant effect, and a glutamic acid having a moisture retention effect. While the solubility of the glutamic acid with respect to the comparative example C22 (glycerin 23 wt% aqueous solution) and the comparative example C23 (ion-exchange water) was lower than 1 wt%, the solubility of the glutamic acid with respect to the working example C192 (23 wt% aqueous solution of compound C20) was as high as 3 wt%. Further, similar tendencies were observed with regard to the gallic acid; larger amounts of the active ingredients were able to be dissolved. In this sense, the skin care agent of the present invention can be used as a base agent, solvent, carrier or the like of a skin care composition.

[Table 32]

| | | Working example C192 | Comparative example C22 | Comparative example C23 | Working example C193 | Comparative example C24 | Comparative example C25 |
|---|---|---|---|---|---|---|---|
| Compound C20 (g) | | 1.8 | | | 1.8 | | |
| Compound C64 (g) | | | 1.8 | | | 1.8 | |
| Ion-exchange water (g) | | 6.2 | 6.2 | 8 | 6.2 | 6.2 | 8 |
| Amount of dissolution | Glutamic acid (g) | 0.24 | <0.08 | <0.08 | | | |
| | Gallic acid (g) | | | | 0.32 | 0.08 | 0.08 |
| Solubility | Glutamic acid (wt%) | 3 | <1 | <1 | | | |
| | Gallic acid (wt%) | | | | 4 | 1 | 1 |

(8) Skin irritancy test

A skin irritancy test was performed with regard to the compound C20, using a human 3-dimensional cultured epidermal model "LabCyte EPI-MODEL" (by J-TEC). The test was performed by adding an aqueous solution of the compound 20 (concentration: 0.1, 1, 10, 25, 50 wt/v% aqueous solution, added amount: 500 μL) to the human epidermal tissue, and then leaving the same to stand still under conditions of exposure time: 24 hours, test temperature: 37°C, test condition: $CO_2$ incubator ($CO_2$ concentration 5 to 10%). After being left to stand still, the aqueous solution of the compound 20 was removed, followed by performing washing three times with 500 μL of a phosphate buffer solution, and then dispensing the washed product into 500 μL of a MTT (3-(4,5-dimethylthiazol-2-yl)-2,5-Diphenyltetrazoliumbromide) medium. The medium was then put into a $CO_2$ incubator and left to stand still at room temperature for three hours, followed by removing the human epidermal tissue that had been stained in a violet-blue color expressed by a product(s) upon a reaction between the reducing enzymes in viable cells and MTT. The stained human epidermal tissue was then put into a microtube together with 300 μL of an isopropylalcohol (IPA) to perform pigment extraction at room temperature for two hours, followed by using a microplate reader to measure an absorbance (570 nm) of each IPA extraction liquid obtained. Here, as a negative control, the absorbance of an IPA extraction liquid of a human epidermal tissue treated with a purified water in a similar manner as above was regarded as that of a condition of viable cell ratio 100%, and the viable cell ratio of each substance was then calculated based on a relative value(s) of the absorbance. Examples exhibiting a viable cell ratio of larger than 50% were evaluated as non-irritating, whereas examples exhibiting a viable cell ratio of not larger than 50% were evaluated as irritating. The test was performed at n=2. The results thereof are shown in Table 33.

[Table 33]

| | Compound | Compound concentration (wt%) | Viable cell ratio (%) | | | Skin irritancy (Viable cell ratio (%): >50%: None, ≤50%: irritating) |
|---|---|---|---|---|---|---|
| | | | n=1 | n=2 | Average value | |
| Working example C194 | C20 | 0.1 | 94.3 | 95.7 | 94.9 | None |
| Working example C195 | | 1 | 80.7 | 83.3 | 81.9 | None |
| Working example C196 | | 10 | 94.8 | 94.1 | 94.5 | None |
| Working example C197 | | 25 | 75.2 | 77.3 | 76.2 | None |
| Working example C198 | | 50 | 65.4 | 69.1 | 67.2 | None |

As can be seen from the results shown in Table 33, it became clear that the compound C20 exhibited a high viable cell ratio(s) at each concentration and was thus low-irritating. From these results, it was indicated that the cosmetics of the present invention such as the skin care agent and hair treatment agent had a low irritancy to the skin.

(9) Human patch test

With regard to the compound C20, a 24 hour-closed human patch test was performed by the following test method in accordance with a closed patch test: "Skin Irritation/Sensitization Test Method, and Skin Property Measurement and Evaluation", Chapter 1: Skin Irritation Test, Section 3: Human Patch Test (p29). The evaluation criteria are shown in Tables 34 and 35.

[Table 34]

| Japanese standard | Rating | Reaction |
|---|---|---|
| - | 0 | No reaction |
| ± | 0.5 | Slight erythema |
| + | 1 | Obvious erythema |
| ++ | 2 | Erythema+Edema, Papule |
| +++ | 3 | Erythema+Edema+Papule+Small blister |
| ++++ | 4 | Large blister |

[Table 35]

| Skin stimulation index | Categorization of 1995 |
|---|---|
| Not larger than 5.0 | Safe product |
| Larger than 5.0, but not larger than 15.0 | Acceptable product |
| Larger than 15.0, but not larger than 30.0 | Product requiring improvement |
| Larger than 30.0 | Hazardous product |

(Test method)

(1) As a patch test unit(s), 15 μL of the compound C20 (85.4 wt% aqueous solution) or a control substance; and a white vaseline of an appropriately evaluatable amount were applied to a filter paper on each chamber.

(2) Before attaching, a planned attachment site of a subject was observed so as to confirm the suitability thereof. The patch test unit prepared was then attached to the attachment site in a closed manner.

(3) The patch test unit was removed after 24 hours from the attachment, followed by lightly performing wiping with an unwoven cloth dampened with a purified water, and then marking the attachment site(s) of each sample by four-point assay. Evaluation was performed after 60 min and 24 hours from the removal of the patch test unit.

Testing agency: Maruishilabo Corporation

(Test results)

As a result of performing the 24 hour-closed attachment test on the human skin of 20 subjects, none of the subjects exhibited an erythema reaction. A skin stimulation index was thus 0.0; and according to a categorization of cosmetics by skin stimulation index, the compound was categorized as a "safe product." The results indicated that the cosmetics of the present invention such as the skin care agent and hair treatment agent had a high safety level.

(10) Feeling of use of emulsification composition containing organic ammonium salt

Emulsification compositions of the working examples C199 to C203 and comparative examples C26 to C35 were prepared in accordance with the composition ratios shown in Table 36, and the feelings of use thereof were then evaluated. Specifically, as a preparation method, an oil agent(s) and a surfactant were heated to 80°C and thus uniformly melted. Next, a product with the organic ammonium salt and an ion-exchange water being uniformly dissolved therein was gradually added while maintaining the temperature, and stirring was performed at 80°C for 3 min before cooling a composition thus obtained to 25°C. Here, after measuring an electrical conductivity of the emulsification composition thus obtained, a low electrical conductivity was observed, and the continuous phase of the composition was confirmed to be an oil phase; it was determined that the emulsification composition obtained was a W/O type emulsion.

A method for evaluating the feelings of use was such that a given amount of the emulsification composition

was put on the skin, followed by evaluating a spreadability, stickiness, moisture retention feeling, persistent moisture retention feeling, skin compatibility and skin elasticity when applying the emulsification composition to the skin. As a panel, five individuals were randomly selected regardless of age and sex; an average value(s) were calculated and were then regarded as evaluation values.

As for a spreadability at the time of applying the composition, evaluation was performed on a scale of 1 to 5, in which 5 was given to examples where the emulsification composition had spread significantly, 3 was given to examples where the emulsification composition had spread, and 1 was given to examples where the emulsification composition had spread unsatisfactorily. Average values of the panel were regarded as evaluation values.

As for a stickiness after application, the emulsification composition was applied to the skin, and a feeling of the skin after spreading the emulsification composition applied was then evaluated on a scale of 1 to 5, in which 5 was given to examples where no stickiness was felt, 3 was given to examples where stickiness was felt slightly, and 1 was given to examples where stickiness was felt. Average values of the panel were regarded as evaluation values.

As for a moisture retention feeling after application, the emulsification composition was applied to the skin, and a feeling of the skin after spreading the emulsification composition applied was then evaluated on a scale of 1 to 5, in which 5 was given to examples where a moisture retention feeling was felt significantly, 3 was given to examples where a moisture retention feeling was felt, and 1 was given to examples where no moisture retention feeling was felt. Average values of the panel were regarded as evaluation values.

As for a persistent moisture retention feeling after application, the emulsification composition was applied to the skin, and a feeling of the skin after two hours from spreading the emulsification composition applied was then evaluated on a scale of 1 to 5, in which 5 was given to examples where a persistent moisture retention feeling was felt significantly, 3 was given to examples where a persistent moisture retention feeling was felt, and 1 was given to examples where no persistent moisture retention feeling was felt. Average values of the panel were regarded as evaluation values.

A skin compatibility was evaluated on a scale of 1 to 5, in which 5 was given to examples where a favorable compatibility was observed, 3 was given to examples where a compatibility was observed, and 1 was given to examples where a poor compatibility was observed. Average values of the panel were regarded as evaluation values.

A skin elasticity was evaluated on a scale of 1 to 5, in which 5 was given to examples where a plump elasticity of the skin was felt, 3 was given to examples where an elasticity was felt to a certain degree, and 1 was given to examples where no elasticity was felt. Average values of the panel were regarded as evaluation values.

[Table 36]

| | | | Working example C199 | Com-parative example C26 | Com-parative example C27 | Working example C200 | Com-parative example C28 | Com-parative example C29 | Working example C201 | Com-parative example C30 | Com-parative example C31 | Working example C202 | Com-parative example C32 | Com-parative example C33 | Working example C203 | Com-parative example C34 | Com-parative example C35 |
|---|---|---|---|---|---|---|---|---|---|---|---|---|---|---|---|---|---|
| Composition ratio (wt%) | Organic ammonium salt : Compound C20 | | 18 | | | 11.3 | | | 18 | | | 7 | | | 15 | | |
| | Compound C64 | | | 18 | | | 11.3 | | | 18 | | | 7 | | | 15 | |
| | Ion-exchange water | | 62 | 62 | 80 | 38.8 | 38.8 | 50 | 67.5 | 67.5 | 85.5 | 23 | 23 | 30 | 50 | 50 | 65 |
| | Oil agent | Liquid paraffin | 10 | | | 25 | | | 2.5 | | | 28 | | | 18.6 | | |
| | | Squalane | 2 | | | 5 | | | 0.5 | | | 5.5 | | | 3.7 | | |
| | | Ceresin | 4 | | | 10 | | | 1 | | | 11 | | | 7.5 | | |
| | | White vaseline | 2 | | | 5 | | | 0.5 | | | 5.5 | | | 3.7 | | |
| | Surfactant | Diglyceryl monooleate | 2 | | | 5 | | | 10 | | | 20 | | | 1.5 | | |
| Appearance | | | Emulsification composition (w/o) | Emulsification composition (w/o) | Emulsification composition (w/o) | Emulsification composition (w/o) | Emulsification composition (w/o) | Emulsification composition (w/o) | Emulsification composition (w/o) | Emulsification composition (w/o) | Emulsification composition (w/o) | Emulsification composition (w/o) | Emulsification composition (w/o) | Emulsification composition (w/o) | Emulsification composition (w/o) | Emulsification composition (w/o) | Emulsification composition (w/o) |
| Feeling of use | Spreadability | | 3.8 | 2.8 | 3.0 | 4.0 | 3.6 | 2.6 | 3.0 | 2.6 | 2.8 | 4.4 | 3.4 | 3.2 | 3.6 | 3.4 | 3.2 |
| | Stickiness | | 3.8 | 3.4 | 3.4 | 3.7 | 3.1 | 3.0 | 3.2 | 2.8 | 2.8 | 3.8 | 2.8 | 3.0 | 3.8 | 2.8 | 3.1 |
| | Moisture retention feeling | | 3.8 | 3.4 | 3.4 | 4.2 | 3.8 | 3.8 | 3.6 | 3.2 | 3.2 | 4.2 | 3.8 | 3.6 | 3.8 | 3.4 | 3.4 |
| | Persistent moisture retention feeling | | 3.7 | 3.0 | 2.4 | 4.0 | 3.4 | 3.0 | 3.5 | 3.0 | 2.7 | 4.0 | 3.4 | 3.0 | 3.7 | 3.0 | 2.7 |
| | Skin compatibility | | 4.0 | 3.4 | 3.6 | 3.9 | 3.8 | 3.2 | 3.4 | 2.8 | 2.8 | 4.6 | 3.4 | 3.4 | 3.7 | 3.2 | 3.6 |
| | Skin elasticity | | 3.8 | 3.5 | 3.0 | 4.0 | 3.6 | 3.0 | 3.3 | 2.9 | 2.5 | 4.6 | 3.7 | 3.3 | 3.7 | 3.3 | 2.9 |

As can be seen from the results shown in Table 36, it was confirmed that the working examples using the

organic ammonium salt(s) were superior to the comparative examples using glycerin, in any of the feelings of use including the spreadability, stickiness, moisture retention feeling, skin compatibility and skin elasticity. Particularly, the working examples each exhibited an excellent persistent moisture retention feeling; it was indicated that the effects of the non-volatile organic ammonium salt(s) were expressed. Especially, in the cases of the working examples C200 and C202 containing, as oil agents, large amounts of a liquid paraffin and ceresin, significant improvements were observed in feelings of use including spreadability, moisture retention feeling and skin compatibility.

Next, as shown in Table 37, evaluations were also performed on a gel and an emulsification composition (O/W) whose forms are different from that of the emulsification composition (W/O). Specifically, as a preparation method of the gels (working examples C204 and C205), a surfactant and an oil agent(s) were heated to 80°C and thus uniformly melted. Next, an organic ammonium salt aqueous solution was gradually added while maintaining the temperature, and stirring was performed at 80°C for 3 min so as to mix the ingredients. Specifically, as a preparation method of the emulsification composition (O/W) (working example C206), the organic ammonium salt or a concentrated glycerin was uniformly dissolved in an ion-exchange water at room temperature. Next, an oil agent was gradually added, followed by performing stirring for 3 min. Here, after measuring an electrical conductivity of an emulsion thus obtained, an electrical conductivity at the same level as that of an ion-exchange water was observed, and the continuous phase of the emulsion was confirmed to be an aqueous phase; it was determined that the emulsion obtained was an O/W type emulsion.

With regard to the feelings of use of these working examples C204 to C206, as was the case with the organic ammonium salt-containing emulsification composition (W/O), a more favorable spreadability, stickiness, moisture retention feeling, skin compatibility and skin elasticity were achieved as compared to an emulsification composition(s) containing glycerin instead of the organic ammonium salt.

[Table 37]

| | | | Working example C204 | Working example C205 | Working example C206 |
|---|---|---|---|---|---|
| Composition ratio (wt%) | Organic ammonium salt: Compound C20 | | 76.9 | 76.9 | 10 |
| | Ion-exchange water | | 13.1 | 13.1 | 77 |
| | Oil agent | Liquid paraffin | | 4 | 10 |
| | | Squalane | | 0.7 | |
| | | Ceresin | | 1.6 | |
| | | White vaseline | | 0.7 | |
| | Surfactant | Diglyceryl monooleate | 10 | 3 | |
| | | Sodium deoxycholate | | | 3 |
| Appearance | | | Gel | Gel | Emulsification composition (O/W) |

(11) Affinity to skin

With regard to a 50 wt% aqueous solution of each of the compounds C20 and C64 as well as an ion-exchange water, an affinity to the skin was evaluated via contact angle measurement. Contact angle measurement was performed using Drop Shape Analyzer DSA30 (by KRUSS GmbH), and a droplet volume was 20 μL. Here, 20 μL of each sample having a distinct concentration was dropped onto the arms of a panel, and contact angles were measured every 1 minute. As the panel, five individuals were randomly selected regardless of age and sex; an average value(s) were calculated and were then regarded as evaluation values. The results are shown in Table 38.

[Table 38]

| | | Working example C207 | Comparative example C36 | Comparative example C37 |
|---|---|---|---|---|
| Compound | | C20 | C64 | Ion-exchange water |
| Compound concentration (wt%) | | 50 | 50 | 100 |
| Contact (° ) angle | 1min | 73 | 79 | 86 |
| | 2min | 73 | 79 | 80 |
| | 3min | 66 | 75 | 75 |
| | 4min | 58 | 76 | 73 |
| | 5min | 56 | 72 | 73 |

As can be seen from the results associated with the working example C207 and comparative examples C36 and C37, smaller contact angles were observed in the case of the compound C20 as compared to the compound C64 and the ion-exchange water; it was confirmed that the compound C20 was superior in affinity to the skin and skin compatibility. Further, it was also confirmed that the affinity had improved as the contact angle significantly decreased with time. In this way, it was indicated that the skin care agent of the present invention was superior to glycerin and an ion-exchange water in skin compatibility.

(12) Long-term retention property to skin

As for the compounds C20 and C64, a long-term adherability thereof to the skin was evaluated via contact angle measurement. An evaluation method was such that a 30 wt% aqueous solution of each of the compounds C20 and C64 as well as an ion-exchange water were applied to the arms of a panel; 20 μL of an ion-exchange water was then dropped onto the arms to which the sample(s) had been applied to measure a contact angle(s), immediately after the application, after 5 min from the application, and after 10 min from the application. Contact angle measurement was performed using Drop Shape Analyzer DSA30 (by KRUSS GmbH). As the panel, five individuals were randomly selected regardless of age and sex; an average value(s) were calculated and were then regarded as evaluation values. The results are shown in Table 39.

[Table 39]

| | | Working example C208 | Comparative example C38 | Comparative example C39 |
|---|---|---|---|---|
| Application sample | Compound | C20 | C64 | Ion-exchange water |
| | Compound concentration (wt%) | 30 | 30 | 100 |
| Contact (° ) angle | 0 min | 53 | 60 | 86 |
| | After 5 min | 53 | 60 | 86 |
| | After 10 min | 53 | 68 | 86 |

The contact angles of the ion-exchange water in the case of the working example C208 (compound C20) were smaller than those in the cases of the comparative examples; it was indicated that the working example C208 (compound C20) was able to improve the affinity between the skin and an ion-exchange water.

As for the working example C208 (compound C20), no change was observed in the contact angle of the ion-exchange water to the skin from immediately after the application to 10 min after the application; it was indicated that an affinity to the skin was maintained for a long period of time. Further, as a result of measuring a contact angle after 30 min from the application, a numerical value observed after 10 min from the application was confirmed to have been maintained; the compound did not volatilize, and a high long-term retention property to the skin was thus observed. As for the comparative example C38 (compound C64), it was confirmed that the contact angle of the ion-exchange water had increased with time after the application, and approached the contact angle(s) in the case of the comparative example C39 (ion-exchange water). In this sense, it is speculated that the level of adhesion to the skin decreased as glycerin volatilized.

Thus, the skin care agent containing the organic ammonium salt of the present invention can be retained on the skin for a long period of time due to the non-volatility of the organic ammonium salt and the affinity to the skin, thereby allowing the effects of the present invention such as the moisture retention property as well as the effects of the active ingredients to be retained in a persistent manner. Further, it is speculated that the reason that these effects are improved is also because the skin care agent is a liquid and can thus evenly coat the surface of the skin for a long period of time.

(13) Permeability to skin

Skin permeability was evaluated by a horny layer tape stripping method. There were used the compound C20 of the working example and the compounds C64 and C97 of the comparative examples that are all shown in Table 40. A sample was applied to the arm and then left thereon for an hour before performing tape stripping on the horn by eight layers. An adhered substance on this tape was then extracted with an ion-exchange water over the course of 10 min, followed by filtrating an extraction liquid thus obtained with a 0.2 μm filter, and then quantifying, via HPLC, the amount of each of the compounds C20, C64 and C97 in each extraction liquid filtrated.

The quantification results were such that in the cases of the first to eighth tape stripping layers, the concentration of the compound C20 of the working example in the extraction liquid was higher than those of the compounds C64 and C97 of the comparative examples in the extraction liquids; it was confirmed that the compound of the working example had a high permeability to the surface of the horny layer. That is, the skin care agent of the present invention has a high permeability to the skin, and can thus be used as, for example, a base agent, solvent and carrier of a skin care composition.

[Table 40]

| | Compound | 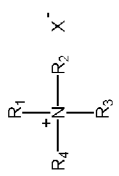 | | | | | Compound concentration (ml/L) | | | | | | | |
|---|---|---|---|---|---|---|---|---|---|---|---|---|---|---|
| | | | | | | | Tape stripping layer | | | | | | | |
| | | $R_1$ | $R_2$ | $R_3$ | $R_4$ | $X^-$ | 1 | 2 | 3 | 4 | 5 | 6 | 7 | 8 |
| Working example C209 | C20 | 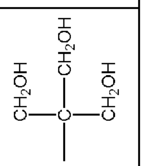 | H | H | H | $CH_3CH(OH)COO^-$ | 0.060 | 0.029 | 0.017 | 0.021 | 0.010 | 0.012 | 0.017 | 0.007 |
| Comparative example C40 | C64 | Glycerin | | | | | 0.030 | 0.014 | 0.009 | 0.009 | 0.008 | 0.006 | 0.006 | 0.006 |
| Comparative example C41 | C97 | Potassium lactate | | | | | 0.020 | 0.017 | 0.016 | 0.009 | 0.008 | 0.006 | 0.006 | 0.006 |

(14) Stability of ascorbic acid-based compound

With regard to a 33 wt% aqueous solution of each of the compounds C46, C49 and C98 to C100 and water, a

stability in an aqueous solution condition was visually evaluated. An evaluation method was such that there was prepared a 33 wt% aqueous solution of each of the compounds C46, C49 and C98 to C100, the aqueous solution was then left to stand still at room temperature or in a thermostatic bath of 50°C for seven days; coloration before and after the still standing was visually confirmed. The evaluation criteria were as follows.

◎: Colorless and transparent
○: Light yellow
Δ: Yellow
×: Brown to black

Coloration occurs as ascorbic acid decomposes. The evaluation results were such that significant improvements in coloration were observed in the cases of the compounds C46 and C49 of the working examples whose ascorbic acids were those of salt structures as compared to the compound C98 of the comparative example C42; there was confirmed a stabilization effect of an ascorbic acid-based compound in an aqueous solution condition due to an ascorbic acid having a salt structure. Further, even when compared with C100 as a case of a sodium salt, C46 and C49 exhibited a high stability; it was indicated that the cations of the present invention were effective. In addition, the compounds C46 and C49 of the working examples brought about results more favorable than that of C100. That is, by introducing the cations of the present invention, a holistically superior stabilization effect can be expressed under the condition of room temperature or 50°C as compared to the ascorbic acid-based compounds C99 and C100 that are generally used in cosmetics; a high effectiveness in cosmetics such as skin care and hair care cosmetics was indicated.

[Table 41]

| | Compound | 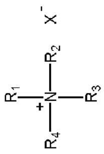 | | | | | Compound concentration (wt%) | Appearance (Visual evaluation) | | |
|---|---|---|---|---|---|---|---|---|---|---|
| | | | | | | | | Immediately after preparation | After 7 days | |
| | | $R_1$ | $R_2$ | $R_3$ | $R_4$ | $X^-$ | | | Room temperature | 50°C |
| Working example C210 | C49 | 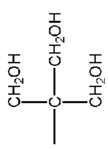 | H | H | H | Ascorbic acid anion | 33 | ◎ | ○ | △ |
| Working example C211 | C46 | 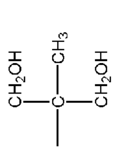 | H | H | H | Ascorbic acid anion | 33 | ◎ | ○ | △ |
| Comparative example C42 | C98 | Ascorbic acid | | | | | 33 | ◎ | × | × |
| Comparative example C43 | C99 | Ascorbic acid glucoside | | | | | 33 | ◎ | ○ | △~× |
| Comparative example C44 | C100 | Sodium ascorbyl phosphate | | | | | 33 | ◎ | ○~△ | △~× |

(15) Evaluation on whitening effect

With regard to a 50 wt% aqueous solution of each of the compounds C46, C49 and C101, a whitening effect was confirmed via a tyrosinase activity inhibition test. Tyrosinase (by SIGMA-ALDRICH) was dissolved with a 67 mM

phosphate buffer solution (pH 6.8) so that the concentration of the tyrosinase would be 0.2 mg/mL, thereby obtaining an enzyme solution. Further, a substrate solution was prepared by dissolving 3 mg of D, L-DOPA (by Tokyo Chemical Industry Co., Ltd.) into 10 mL of the 67 mM phosphate buffer solution. In the beginning, 80 μL of each 50 wt% aqueous solution of each of the compounds C46, C49 and C101 was added to 64 μL of the 67 mM phosphate buffer solution, followed by adding thereto 16 μL of the enzyme solution, and then carrying out preincubation at room temperature for 10 min. Next, 80 μL of the substrate solution was quickly added to the preincubated solution, and an absorbance (A1) at 475 nm immediately after the addition of the substrate solution was then measured. Further, after leaving this solution to stand still at room temperature for a week, an absorbance (A2) at 475 nm was again measured.

In addition, as a control, there were prepared samples containing an ultrapure water instead of the compounds of the working examples; and in a similar manner as above, there were measured an absorbance (A3) at 475 nm immediately after the addition of the substrate, and an absorbance (A4) at 475 nm after leaving the solution at room temperature for a week.

An inhibition rate was calculated with the following formula.

$$\text{Inhibition rate (\%)} = 100-(A2-A1)/(A4-A3) \times 100$$

As can be seen from the evaluation results, it was confirmed that the organic ammonium salt of the present invention was able to inhibit tyrosinase activity and thus had a skin whitening effect. That is, it was indicated that by introducing the skeleton of an active ingredient into the organic ammonium salt of the present invention, the effect(s) of such active ingredient could be exerted.

[Table 42]

| | Compound | 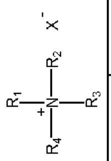 | | | | | Compound concentration (μg/mL) | Compounding molar ratio (acid:base) | Tyrosinase activity inhibition rate (%) |
|---|---|---|---|---|---|---|---|---|---|
| | | $R_1$ | $R_2$ | $R_3$ | $R_4$ | $X^-$ | | | |
| Working example C212 | C49 | 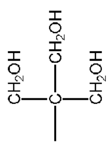 | H | H | H | Ascorbic acid anion | 500 | 1:1 | 95.8 |
| Working example C213 | C46 | 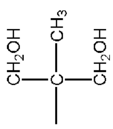 | H | H | H | Ascorbic acid anion | 500 | 1:1 | 88.1 |
| Working example C214 | C101 | 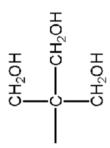 | H | H | H | Ascorbyl phosphate anion | 500 | 1:3 | 94.6 |

## Claims

1. A cosmetic compounding agent comprising an organic ammonium salt having a hydrogen-bonding functional group(s) in a cation and/or an anion.

2. The cosmetic compounding agent according to claim 1, wherein the cation is an ammonium cation.

3. The cosmetic compounding agent according to claim 2, wherein the ammonium cation is represented by the following formula (I):

[Chemical formula 1] $N^+[R^1]_n[R^2]_{4-n}$ (I)

wherein each $R^1$ independently represents a hydroxyalkyl group in which there is at least one hydroxy group, an alkyl moiety is a linear or branched moiety having 1 to 10 carbon atoms, and the alkyl moiety may contain an oxygen atom(s); a carboxyalkyl group in which there is at least one carboxy group, an alkyl moiety is a linear or branched moiety having 1 to 10 carbon atoms, and the alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and the alkyl moiety may contain an oxygen atom(s), and
wherein each $R^2$ independently represents a hydrogen atom or a linear or branched alkyl group having 1 to 18 carbon atoms, and n represents an integer of 0 to 4.

4. The cosmetic compounding agent according to claim 3, wherein n represents an integer of 1 to 4.

5. The cosmetic compounding agent according to claim 3 or 4, wherein $R^2$ represents a hydrogen atom.

6. The cosmetic compounding agent according to claim 4, wherein $R^1$ represents a monohydroxyalkyl group or monocarboxyalkyl group whose alkyl moiety is a linear or branched moiety having 1 to 10 carbon atoms.

7. The cosmetic compounding agent according to claim 6, wherein $R^2$ represents a hydrogen atom.

8. The cosmetic compounding agent according to claim 4, wherein $R^2$ represents a linear or branched alkyl group having 1 to 18 carbon atoms.

9. The cosmetic compounding agent according to claim 4, wherein at least one $R^1$ represents a polyhydroxyalkyl group in which there are at least two hydroxy groups, and the alkyl moiety is a linear or branched moiety having 1 to 10 carbon atoms.

10. The cosmetic compounding agent according to claim 9, wherein $R^2$ represents a hydrogen atom.

11. The cosmetic compounding agent according to any one of claims 1 to 10, wherein the anion is carboxylic acid-based anions.

12. The cosmetic compounding agent according to claim 11, wherein a hydrogen-bonding functional group(s) is present in the anion.

13. The cosmetic compounding agent according to claim 12, wherein the hydrogen-bonding functional group(s) is any one of a hydroxy group, a carboxy group and a carboxylate group.

14. The cosmetic compounding agent according to any one of claims 1 to 13, wherein an anhydride and hydrate of the organic ammonium salt are liquids at 25°C.

15. The cosmetic compounding agent according to any one of claims 1 to 14, wherein the cosmetic compounding agent is obtained by separately adding a base and an acid that form the organic ammonium salt.

16. The cosmetic compounding agent according to claim 1, wherein the cosmetic compounding agent is a hair treatment agent.

17. The cosmetic compounding agent according to claim 16, wherein the cation is an ammonium cation.

18. The cosmetic compounding agent according to claim 17, wherein the ammonium cation is represented by the following formula (I):

[Chemical formula 1] $N^+[R^1]_n[R^2]_{4-n}$ (I)

wherein each $R^1$ independently represents a hydroxyalkyl group in which there is at least one hydroxy group, an alkyl moiety is a linear or branched moiety having 1 to 10 carbon atoms, and the alkyl moiety may contain an oxygen atom(s); a carboxyalkyl group in which there is at least one carboxy group, an alkyl moiety is a linear or branched moiety having 1 to 10 carbon atoms, and the alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and the alkyl moiety may contain an oxygen atom(s), and
wherein each $R^2$ independently represents a hydrogen atom or a linear or branched alkyl group having 1 to 18 carbon atoms, and n represents an integer of 0 to 4.

**19.** The cosmetic compounding agent according to claim 18, wherein n represents an integer of 1 to 4.

**20.** The cosmetic compounding agent according to claim 18 or 19, wherein $R^2$ represents a hydrogen atom.

**21.** The cosmetic compounding agent according to claim 19, wherein $R^1$ represents a monohydroxyalkyl group or monocarboxyalkyl group whose alkyl moiety is a linear or branched moiety having 1 to 10 carbon atoms.

**22.** The cosmetic compounding agent according to claim 21, wherein $R^2$ represents a hydrogen atom.

**23.** The cosmetic compounding agent according to claim 19, wherein $R^2$ represents a linear or branched alkyl group having 1 to 18 carbon atoms.

**24.** The cosmetic compounding agent according to claim 19, wherein at least one $R^1$ represents a polyhydroxyalkyl group in which there are at least two hydroxy groups, and the alkyl moiety is a linear or branched moiety having 1 to 10 carbon atoms.

**25.** The cosmetic compounding agent according to claim 24, wherein $R^2$ represents a hydrogen atom.

**26.** The cosmetic compounding agent according to any one of claims 16 to 25, wherein the anion is a carboxylic acid-based anion.

**27.** The cosmetic compounding agent according to claim 26, wherein a hydrogen-bonding functional group(s) is present in the anion.

**28.** The cosmetic compounding agent according to claim 27, wherein the hydrogen-bonding functional group(s) is any one of a hydroxy group, a carboxy group and a carboxylate group.

**29.** The cosmetic compounding agent according to any one of claims 16 to 28, wherein an anhydride and hydrate of the organic ammonium salt are liquids at 25°C.

**30.** The cosmetic compounding agent according to any one of claims 16 to 29, wherein the cosmetic compounding agent is obtained by separately adding a base and an acid that form the organic ammonium salt.

**31.** The cosmetic compounding agent according to claim 1, wherein the cosmetic compounding agent is a skin care agent.

**32.** The cosmetic compounding agent according to claim 31, wherein the cation is an ammonium cation.

**33.** The cosmetic compounding agent according to claim 32, wherein the ammonium cation is represented by the following formula (I):

[Chemical formula 1] $N^+[R^1]_n[R^2]_{4-n}$ (I)

wherein each $R^1$ independently represents a hydroxyalkyl group in which there is at least one hydroxy group, an alkyl moiety is a linear or branched moiety having 1 to 10 carbon atoms, and the alkyl moiety may contain an oxygen atom(s); a carboxyalkyl group in which there is at least one carboxy group, an alkyl moiety is a linear or branched moiety having 1 to 10 carbon atoms, and the alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and the alkyl moiety may contain an

oxygen atom(s), and
wherein each $R^2$ independently represents a hydrogen atom or a linear or branched alkyl group having 1 to 18 carbon atoms, and n represents an integer of 0 to 4.

**34.** The cosmetic compounding agent according to claim 33, wherein n represents an integer of 1 to 4.

**35.** The cosmetic compounding agent according to claim 33 or 34, wherein $R^2$ represents a hydrogen atom.

**36.** The cosmetic compounding agent according to claim 34, wherein $R^1$ represents a monohydroxyalkyl group or monocarboxyalkyl group whose alkyl moiety is a linear or branched moiety having 1 to 10 carbon atoms.

**37.** The cosmetic compounding agent according to claim 36, wherein $R^2$ represents a hydrogen atom.

**38.** The cosmetic compounding agent according to claim 34, wherein $R^2$ represents a linear or branched alkyl group having 1 to 18 carbon atoms.

**39.** The cosmetic compounding agent according to claim 34, wherein at least one $R^1$ represents a polyhydroxyalkyl group in which there are at least two hydroxy groups, and the alkyl moiety is a linear or branched moiety having 1 to 10 carbon atoms.

**40.** The cosmetic compounding agent according to claim 39, wherein $R^2$ represents a hydrogen atom.

**41.** The cosmetic compounding agent according to any one of claims 31 to 40, wherein the anion is a carboxylic acid-based anion.

**42.** The cosmetic compounding agent according to claim 41, wherein a hydrogen-bonding functional group(s) is present in the anion.

**43.** The cosmetic compounding agent according to claim 42, wherein the hydrogen-bonding functional group(s) is any one of a hydroxy group, a carboxy group and a carboxylate group.

**44.** The cosmetic compounding agent according to any one of claims 31 to 43, wherein an anhydride and hydrate of the organic ammonium salt are liquids at 25°C.

**45.** The cosmetic compounding agent according to any one of claims 31 to 44, wherein the cosmetic compounding agent is obtained by separately adding a base and an acid that form the organic ammonium salt.

**46.** A cosmetic containing the cosmetic compounding agent according to any one of claims 1 to 45.

**47.** The cosmetic according to claim 46, wherein the cosmetic is a hair treatment composition.

**48.** The cosmetic according to claim 46, wherein the cosmetic is a skin care composition.

**49.** A cosmetic produced by separately adding, as raw materials of the cosmetic, an ammonium cation-forming base and an anion-forming acid that form an organic ammonium salt composed of an anion and an ammonium cation represented by the following formula (I):

[Chemical formula 1] $N^+[R^1]_n[R^2]_{4-n}$ (I)

wherein each $R^1$ independently represents a hydroxyalkyl group in which there is at least one hydroxy group, an alkyl moiety is a linear or branched moiety having 1 to 10 carbon atoms, and the alkyl moiety may contain an oxygen atom(s); a carboxyalkyl group in which there is at least one carboxy group, an alkyl moiety is a linear or branched moiety having 1 to 10 carbon atoms, and the alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and the alkyl moiety may contain an oxygen atom(s), and
wherein each $R^2$ independently represents a hydrogen atom or a linear or branched alkyl group having 1 to 18 carbon atoms, and n represents an integer of 0 to 4.

**50.** The cosmetic according to claim 49, wherein n represents an integer of 1 to 4.

**51.** The cosmetic according to claim 50, wherein $R^2$ represents a hydrogen atom.

**52.** The cosmetic according to claim 50, wherein $R^1$ represents a monohydroxyalkyl group or monocarboxyalkyl group whose alkyl moiety is a linear or branched moiety having 1 to 10 carbon atoms.

**53.** The cosmetic according to claim 52, wherein $R^2$ represents a hydrogen atom.

**54.** The cosmetic according to claim 50, wherein $R^2$ represents a linear or branched alkyl group having 1 to 18 carbon atoms.

**55.** The cosmetic according to claim 50, wherein at least one $R^1$ represents a polyhydroxyalkyl group in which there are at least two hydroxy groups, and the alkyl moiety is a linear or branched moiety having 1 to 10 carbon atoms.

**56.** The cosmetic according to claim 55, wherein $R^2$ represents a hydrogen atom.

**57.** The cosmetic according to any one of claims 49 to 56, wherein the anions is a carboxylic acid-based anion.

**58.** The cosmetic according to claim 57, wherein a hydrogen-bonding functional group(s) is present in the anions.

**59.** The cosmetic according to claim 58, wherein the hydrogen-bonding functional group(s) is any one of a hydroxy group, a carboxy group and a carboxylate group.

**60.** The cosmetic according to any one of claims 49 to 59, wherein an anhydride and hydrate of the organic ammonium salt are liquids at 25°C.

**61.** The cosmetic according to any one of claims 49 to 60, wherein the cosmetic is a hair treatment composition.

**62.** The cosmetic according to any one of claims 49 to 60, wherein the cosmetic is a skin care composition.

**63.** A method for producing a cosmetic, comprising a step of adding the cosmetic compounding agent according to any one of claims 1 to 45.

**64.** A method for producing a cosmetic, comprising a step of adding an ammonium cation-forming base and an anion-forming acid that form an organic ammonium salt composed of an anion and an ammonium cation represented by the following formula (I):

[Chemical formula 1] $N^+[R^1]_n[R^2]_{4-n}$ (I)

wherein each $R^1$ independently represents a hydroxyalkyl group in which there is at least one hydroxy group, an alkyl moiety is a linear or branched moiety having 1 to 10 carbon atoms, and the alkyl moiety may contain an oxygen atom(s); a carboxyalkyl group in which there is at least one carboxy group, an alkyl moiety is a linear or branched moiety having 1 to 10 carbon atoms, and the alkyl moiety may contain an oxygen atom(s); or a hydroxycarboxyalkyl group in which there are at least one hydroxy group and at least one carboxy group, an alkyl moiety is a linear or branched moiety having 1 to 10 carbon atoms, and the alkyl moiety may contain an oxygen atom(s), and
wherein each $R^2$ independently represents a hydrogen atom or a linear or branched alkyl group having 1 to 18 carbon atoms, and n represents an integer of 0 to 4.

**65.** The method for producing the cosmetic according to claim 64, wherein after producing a cosmetic compounding agent containing the organic ammonium salt by mixing the base and the acid, the cosmetic compounding agent is then added as a raw material of the cosmetic.

**66.** The method for producing the cosmetic according to claim 64, wherein the base and the acid are separately added as raw materials of the cosmetic.

**67.** The method for producing the cosmetic according to any one of claims 64 to 66, wherein n represents an integer

of 1 to 4.

**68.** The method for producing the cosmetic according to any one of claims 64 to 67, wherein $R^2$ represents a hydrogen atom.

**69.** The method for producing the cosmetic according to claim 67, wherein $R^1$ represents a monohydroxyalkyl group or monocarboxyalkyl group whose alkyl moiety is a linear or branched moiety having 1 to 10 carbon atoms.

**70.** The method for producing the cosmetic according to claim 69, wherein $R^2$ represents a hydrogen atom.

**71.** The method for producing the cosmetic according to claim 67, wherein $R^2$ represents a linear or branched alkyl group having 1 to 18 carbon atoms.

**72.** The method for producing the cosmetic according to claim 67, wherein at least one $R^1$ represents a polyhydroxyalkyl group in which there are at least two hydroxy groups, and the alkyl moiety is a linear or branched moiety having 1 to 10 carbon atoms.

**73.** The method for producing the cosmetic according to claim 72, wherein $R^2$ represents a hydrogen atom.

**74.** The method for producing the cosmetic according to any one of claims 64 to 73, wherein the anion is a carboxylic acid-based anion.

**75.** The method for producing the cosmetic according to claim 74, wherein a hydrogen-bonding functional group(s) is present in the anion.

**76.** The method for producing the cosmetic according to claim 75, wherein the hydrogen-bonding functional group(s) is any one of a hydroxy group, a carboxy group and a carboxylate group.

**77.** The method for producing the cosmetic according to any one of claims 64 to 76, wherein an anhydride and hydrate of the organic ammonium salt are liquids at 25°C.

**78.** The method for producing the cosmetic according to any one of claims 63 to 77, wherein the cosmetic is a hair treatment composition.

**79.** The method for producing the cosmetic according to any one of claims 63 to 77, wherein the cosmetic is a skin care composition.

| | Working example B50 | Working example B51 | Working example B52 | Comparative example B15 | Comparative example B16 | Comparative example B17 |
|---|---|---|---|---|---|---|
| | Compound B11 added (3.0% by mass) | Compound B23 added (3.0% by mass) | Compound B7 added (3.0% by mass) | Compound B26 added (3.0% by mass) | Compound B27 added (3.0% by mass) | Compound B17 added (3.0% by mass) |
| Appearance after air drying | 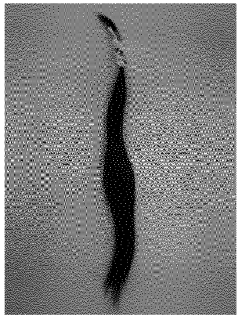 | 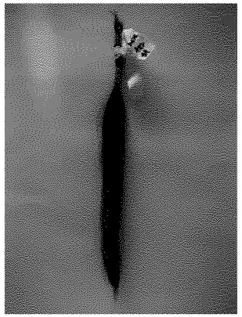 | 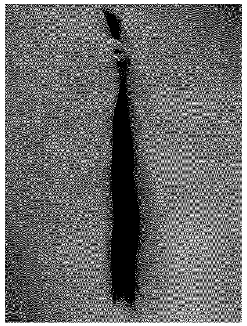 | 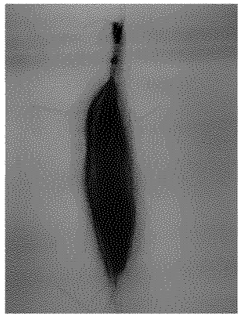 | 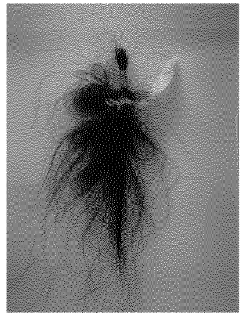 | 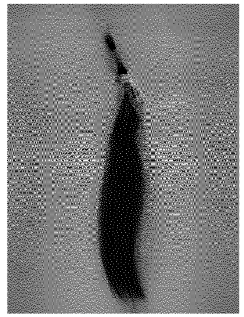 |

FIG.1A

| | Working example B50 | Working example B51 | Working example B52 |
|---|---|---|---|
| | Compound B11 added (3.0% by mass) | Compound B23 added (3.0% by mass) | Compound B7 added (3.0% by mass) |
| SEM image after air drying | 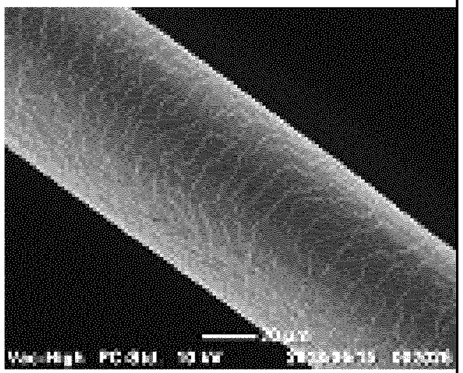 | 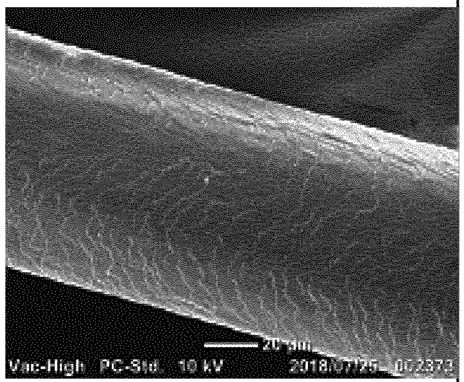  | 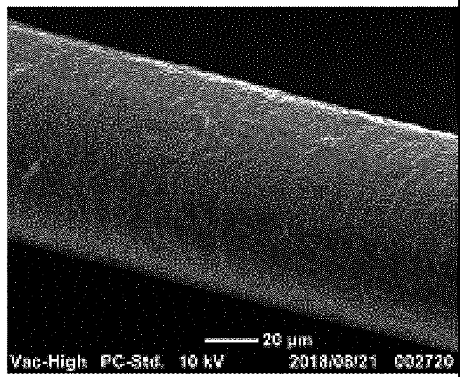  |

FIG.1B

| | Working example B57 | Comparative example B21 | Working example B58 | Comparative example B22 | Working example B59 | Comparative example B23 |
|---|---|---|---|---|---|---|
| | Lauroylmethyl-β-alanine sodium | | Triethanolamine cocoyl glutamate | | Sodium tetradecene sulfonate | |
| | Compound B11 added (3.0% by mass) | Compound B27 added (3.0% by mass) | Compound B11 added (3.0% by mass) | Compound B27 added (3.0% by mass) | Compound B11 added (3.0% by mass) | Compound B27 added (3.0% by mass) |
| Appearance after air drying | 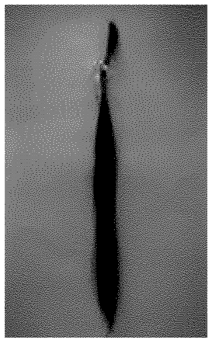 | 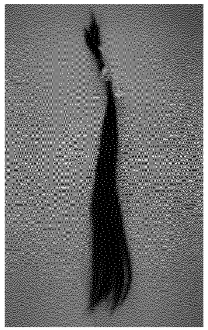 | 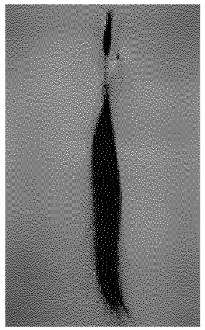 | 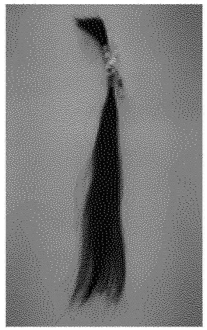 | 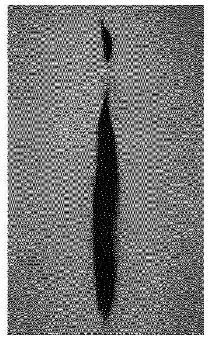 | 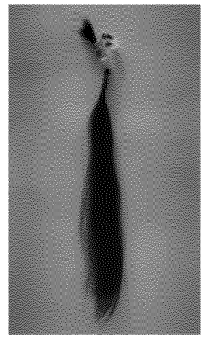 |

FIG.2

| | Working example B60 | Working example B61 | Comparative example B24 | Comparative example B25 | Comparative example B26 |
|---|---|---|---|---|---|
| | Compound B11 added (3.0% by mass) | Compound B23 added (3.0% by mass) | Compound B26 added (3.0% by mass) | Compound B27 added (3.0% by mass) | Compound B28 added (3.0% by mass) |
| Appearance after air drying | 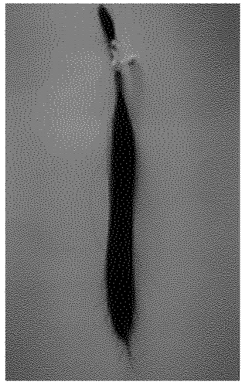 | 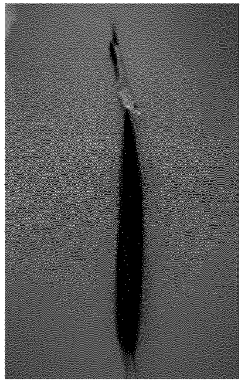 | 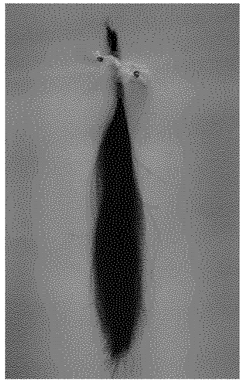 | 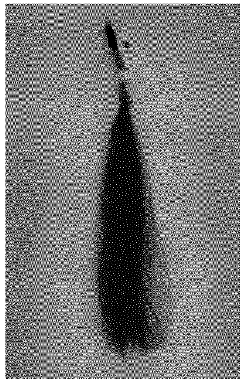 | 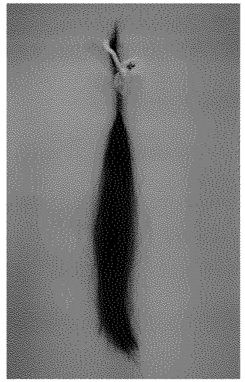 |

FIG.3

**INTERNATIONAL SEARCH REPORT**

International application No.
PCT/JP2020/005669

A. CLASSIFICATION OF SUBJECT MATTER
A61Q 19/00(2006.01)i; A61Q 5/00(2006.01)i; A61Q 5/02(2006.01)i; A61Q 5/12(2006.01)i; A61K 8/20(2006.01)i; A61K 8/24(2006.01)i; A61K 8/36(2006.01)i; A61K 8/40(2006.01)i; A61K 8/41(2006.01)i; A61K 8/46(2006.01)i; A61K 8/67(2006.01)i
FI: A61K8/40; A61Q5/00; A61Q5/02; A61Q5/12; A61Q19/00; A61K8/36; A61K8/46; A61K8/20; A61K8/24; A61K8/67; A61K8/41
According to International Patent Classification (IPC) or to both national classification and IPC

B. FIELDS SEARCHED
Minimum documentation searched (classification system followed by classification symbols)
A61Q19/00; A61Q5/00; A61Q5/02; A61Q5/12; A61K8/20; A61K8/24; A61K8/36; A61K8/40; A61K8/41; A61K8/46; A61K8/67

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 08-508021 A (HENKEL KOMMANDITGESELLSCHAFT AUF AKTIEN.) 27.08.1996 (1996-08-27) claims 1-9, examples, table 1, formulation J | 1-4, 6, 8-9, 11-13, 15-19, 21, 23-28, 30, 46-47, 49-50, 52, |
| X | | 54-55, 57-59, 61-63, 67-69, 71-72, 74-76, 78 |
| A | | 5, 7, 10, 14, 20, 22, 29, 31-45, 48, 51, 53, 56, 60, 62, 68, 70, |
| A | | 73, 77 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 April 2020 (10.04.2020) | 28 April 2020 (28.04.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/005669

C (Continuation). DOCUMENTS CONSIDERED TO BE RELEVANT

| Category* | Citation of document, with indication, where appropriate, of the relevant passages | Relevant to claim No. |
|---|---|---|
| X<br><br><br><br><br>X<br><br><br><br>A<br><br><br><br>A | JP 08-508055 A (HENKEL KOMMANDITGESELLSCHAFT AUF AKTIEN.) 27.08.1996 (1996-08-27) claims 1-11, examples | 1-4, 6, 8-9, 11-13, 15-19, 21, 23-28, 30, 46-47, 49-50, 52,<br>54-55, 57-59, 61, 63-67, 69, 71-72, 74-76, 78<br>5, 7, 10, 14, 20, 22, 29, 31-45, 48, 51, 53, 56, 60, 62, 68, 70, 73, 77 |
| X | JP 2014-131974 A (MIYOSHI OIL & FAT CO., LTD.) 17.07.2014 (2014-07-17) claims, paragraph [0068], examples | 1-79 |
| X | JP 2014-131975 A (MIYOSHI OIL & FAT CO., LTD.) 17.07.2014 (2014-07-17) claims, paragraph [0071], examples | 1-79 |
| A | JP 2011-521951 A (AVEDA CORPORATION) 28.07.2011 (2011-07-28) | 1-79 |
| P, X | JP 2019-023185 A (MIYOSHI OIL & FAT CO., LTD.) 14.02.2019 (2019-02-14) claims, paragraphs [0091], [0094]-[0095], examples | 1-79 |

Form PCT/ISA/210 (continuation of second sheet) (January 2015)

**INTERNATIONAL SEARCH REPORT**
Information on patent family members

International application No.
PCT/JP2020/005669

| Patent Documents referred in the Report | Publication Date | Patent Family | Publication Date |
|---|---|---|---|
| JP 08-508021 A | 27 Aug. 1996 | WO 1994/021226 A1<br>EP 689420 A1 | |
| JP 08-508055 A | 27 Aug. 1996 | WO 1994/021771 A1<br>EP 690907 A1 | |
| JP 2014-131974 A | 17 Jul. 2014 | (Family: none) | |
| JP 2014-131975 A | 17 Jul. 2014 | (Family: none) | |
| JP 2011-521951 A | 28 Jul. 2011 | US 2009/0297466 A1<br>WO 2009/148825 A2<br>EP 2309973 A2<br>KR 10-2011-0014237 A | |
| JP 2019-023185 A | 14 Feb. 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014136678 A
- JP 2013040153 A
- JP 2014151015 A
- JP 2014131974 A
- JP 2014131975 A
- JP 2019023185 A
- JP 2012031137 A
- JP 2018136893 A